(19) **Europäisches Patentamt / European Patent Office / Office européen des brevets**

(11) **EP 4 725 409 A2**

(12) # EUROPEAN PATENT APPLICATION

(43) Date of publication:
 **15.04.2026 Bulletin 2026/16**

(21) Application number: **25198665.9**

(22) Date of filing: **29.12.2021**

(51) International Patent Classification (IPC):
 ***A61B 5/03*** (2006.01)

(52) Cooperative Patent Classification (CPC):
 **A61F 6/08; A61B 5/0205; A61B 5/0261;
 A61B 5/036; A61B 5/1076; A61B 5/224;
 A61B 5/4337; A61B 8/08; A61B 8/4227;
 A61B 8/4416; A61B 8/483; A61B 8/5223;
 A61B 8/523; A61B 8/565; G06T 7/11;** (Cont.)

(84) Designated Contracting States:
 **AL AT BE BG CH CY CZ DE DK EE ES FI FR GB
 GR HR HU IE IS IT LI LT LU LV MC MK MT NL NO
 PL PT RO RS SE SI SK SM TR**

(30) Priority: **05.01.2021 US 202163133913 P**

(62) Document number(s) of the earlier application(s) in
 accordance with Art. 76 EPC:
 **21916687.3 / 4 274 482**

(71) Applicant: **COSM Medical Corp.
 Ontario, M6B 4M4 (CA)**

(72) Inventors:
 • **SHAM, Derek
 Ontario, M6B 4M4 (CA)**
 • **BORAZJANI, Ali
 Ontario, M6B 4M4 (CA)**
 • **SAN, Aye
 Ontario, M6B 4M4 (CA)**
 • **BLOKKER, Alexandra
 Ontario, M6B 4M4 (CA)**
 • **LANCEFIELD, Robert
 Ontario, M6B 4M4 (CA)**
 • **AMERI, Golafsoun
 Ontario, M6B 4M4 (CA)**
 • **XIA, Wenyao
 Ontario, M6B 4M4 (CA)**

(74) Representative: **Grünecker Patent- und
 Rechtsanwälte
 PartG mbB
 Leopoldstraße 4
 80802 München (DE)**

Remarks:
 This application was filed on 28.08.2025 as a
 divisional application to the application mentioned
 under INID code 62.

(54) **METHODS AND SYSTEMS FOR VAGINAL THERAPEUTIC DEVICE FITTING**

(57) Pelvic organ prolapse (POP) and urinary incontinence (UI) are common, often distressing conditions, where, at present, physical non-surgical devices are fitted by best guess or trial-and-error with devices offered in a range of standard designs and sizes. However, each user is unique and accordingly methods, systems and devices providing a personal pelvic health characterization and provisioning approach are outlined that factor user specific anatomy and physiology, user lifestyle, user experiences and automated assessments into provisioning custom vaginal therapeutic devices. Further, user specific anatomy and physiology should be obtained in a reproducible manner with devices and systems that remove measurement artifacts, errors, bias etc. whilst providing the patient with an improved experience and the medical personnel with ergonomic, efficient, and easy to use systems exploiting combinations of dedicated multi-patient measurement equipment with user specific consumable items for cleanliness etc.

Figure 1

EP 4 725 409 A2

(52) Cooperative Patent Classification (CPC): (Cont.)
**G06T 7/143; G16H 10/20; G16H 20/30;**
**G16H 20/40; G16H 30/40; G16H 40/63;**
**G16H 40/67; G16H 50/20; G16H 50/50;**
**G16H 50/70; G16H 80/00;** A61B 5/1121;
A61B 5/205; A61B 5/6847; A61B 8/0841;
A61B 8/485; G06T 2207/10132

**Description**

**CROSS-REFERENCE TO RELATED APPLICATIONS**

This application claims the benefit of priority to U.S. Patent Application 63/133,913 filed January 5, 2021.

**FIELD OF THE INVENTION**

The present invention relates to vaginal devices and more specifically to devices as well as methods and systems for the retention of such devices, the devices being for measurement, diagnostics, therapy, or treatment.

**BACKGROUND OF THE INVENTION**

For women pelvic organ prolapse (POP) and urinary incontinence (UI) are common and often distressing conditions. Research indicates that in the United States alone there are 3.3 million women with pelvic organ prolapse and approximately 300,000 surgeries are performed annually in the United States. Additionally, approximately 25% of all women, 33% of older women, have some degree of urinary incontinence. Further, male urinary incontinence whilst it exists has only recently become perhaps evident to the general population with the advent of advertisements for male and female incontinence underwear. An aging population at this point would not indicate any reduction in such figures in the near term whilst a massive expenditure and ease of availability of ultra-thin liners for women's underwear, male and female incontinence underwear and emerging products such as liners for male underwear within supermarkets and pharmacies indicate that the demand and market are high enough for multinational household product and pharmaceutical enterprises to have product lines and brands in this area.

Today, a range of surgical treatment options for POP as well as non-surgical treatments exist. Non-surgical treatment options include Kegel exercises, Kegel assist devices, pessaries, core/floor strengthening exercises, biofeedback, electrical stimulation, hormone replacement therapy, tibial nerve stimulation and support garments. At present, physical non-surgical devices such as Kegel assist devices and pessaries are fitted by best guess, trial-and-error. Pessaries being prosthetic devices that can be inserted into the vagina to support its internal structure as a remedy for urinary incontinence, fecal incontinence, cervical incompetence and/or pelvic organ prolapse. Whilst the literature is replete with information explaining to medical personnel how to fit a pessary or users how to tell if their pessary fits correctly, this potentially exacerbates the situation as there are at least 20 designs of pessary alone each available in multiple sizes, in some instances 10 or more. So, type of pessary and size both need to be factored into addressing the correct device for a user.

As discussed by the inventors previously, see for example World Patent Application 2019/051,579 entitled "Methods and Systems for Vaginal Therapeutic Device Fitting", it would be beneficial to replace the current manual processes of sizing, fitting etc. as well as pessary design with a personal pelvic health characterization and provisioning approach that factors user specific anatomy and physiology, user life style, user experiences, automated assessments etc. into provisioning custom vaginal therapeutic devices.

Accordingly, the user specific anatomy and physiology should be obtained in a reproducible manner with devices and systems that remove measurement artifacts, errors, bias etc. whilst providing the patient with an improved experience and the medical personnel with ergonomic, efficient, and easy to use systems exploiting combinations of dedicated multi-patient measurement equipment with user specific consumable items for cleanliness etc.

Other aspects and features of the present invention will become apparent to those ordinarily skilled in the art upon review of the following description of specific embodiments of the invention in conjunction with the accompanying figures.

**SUMMARY OF THE INVENTION**

It is an object of the present invention to mitigate limitations within the prior art relating to vaginal devices and more specifically to devices as well as methods and systems for the retention of such devices, the devices being for measurement, diagnostics, therapy, or treatment.

In accordance with an embodiment of the invention there is provided a balloon comprising a first balloon disposed at a first position on a catheter; a second balloon disposed adjacent the first balloon on the catheter; wherein each of the first balloon and second balloon is filled with a predetermined fluid independent from the other of the first balloon and second balloon; the first balloon is either attached at both ends to the catheter or attached at a first end of the catheter and extends beyond the catheter at a second distal end to the first end; and the second balloon is attached at both ends to the catheter.

In accordance with an embodiment of the invention there is provided a method comprising providing an outer cushion formed from a low elastic modulus material for conforming to a predetermined portion of a patient's body; providing a structural shield formed from a high elastic modulus material disposed with the outer cushion between it and the patient; providing a manometry balloon for establishing first data relating to the patient; and providing a probe for establishing second data relating the patient having one or more transducers generating and/or receiving signals of a predetermined type; wherein the outer cushion and structural shield maintain the manometry balloon in position within a cavity of the patient; the manometry balloon is filled to a predetermined pressure and/or volume with a predetermined fluid; the body of the manometry balloon and the predetermined fluid have high contrast against the patient's body to the signals of the predetermined type; and the probe can be position against or in close proximity to the outer cushion.

In accordance with an embodiment of the invention there is provided a method comprising providing a structural shield formed from a high elastic modulus material; providing a manometry balloon for establishing first data relating to the patient; and providing a probe for establishing second data relating the patient; wherein the structural shield maintains the manometry balloon in position within a cavity of the patient; the manometry balloon is filled to a predetermined pressure and/or volume with a predetermined fluid; the body of the manometry balloon and predetermined fluid present a high contrast against the patient's body to the probe; and the probe can be positioned against or in close proximity to a window within the structural shield.

In accordance with an embodiment of the invention there is provided a method comprising providing a cushion formed from a low elastic modulus material for conforming to a predetermined portion of a patient's body; wherein providing a manometry balloon for establishing first data relating to the patient; providing a probe for establishing second data relating the patient; and providing a seat within an opening; wherein the cushion is disposed between patient's body and the seat; the cushion maintains the manometry balloon in position within a cavity of the patient; the manometry balloon is filled to a predetermined pressure and/or volume with a predetermined fluid; the body of the manometry balloon and predetermined fluid present a high contrast against the patient's body to the probe; and the probe is positioned in or in close proximity to the opening within the seat to acquire the second data.

In accordance with an embodiment of the invention there is provided a method of performing measurements with respect to a patient comprising: providing a balloon for insertion within a cavity of the patient; providing a probe exploiting signals of a predetermined type; providing a manometry system coupled to the balloon; providing a support for the patient.

In accordance with an embodiment of the invention there is provided a device comprising:

an outer cushion formed from a low elastic modulus material for conforming to a predetermined portion of a patient's body;

a structural shield formed from a high elastic modulus material disposed with the outer cushion between it and the patient; wherein

the outer cushion and structural shield maintains a manometry balloon in position within a cavity of the patient when the manometry balloon is inserted into the cavity of the patient.

In accordance with an embodiment of the invention there is provided a method comprising automatically identifying a pubic symphysis (PS) of a patient and an anorectal angle (ARA) of the patient from a plurality of transperineal ultrasound images.

In accordance with an embodiment of the invention there is provided a device comprising:

a body;
a plurality of pads; and
a plurality of arms each connected to the body at a first end and to a pad of the plurality of pads at a second distal end; wherein
each arm of the plurality of arms can be extended from or retracted into the body; and
a subset of the plurality of pads comprise sensors, each pad of the predetermined subset of the plurality pads comprising a predetermined sensor.

In accordance with an embodiment of the invention there is provided a device comprising:

a body;
a plurality of sensors;
a distributed element around a periphery of the device comprising a plurality of elements; and
an electronic circuit comprising a microprocessor coupled to each sensor of the plurality of sensors and each element of the plurality of elements of the distributed element.

In accordance with an embodiment of the invention there is provided a method of establishing a user specific therapeutic device (USTD) comprising:

establishing user data by performing measurement and characterisation with respect to the user
where:

a first portion of the user data comprises structural measurements of a region of an anatomy of the user to which the USTD relates;
a second portion of the user data comprises at least one of physiological measurements and biomechanical measurements of the region of the anatomy of the user to which the USTD relates;
a third portion of the user data comprises establishing at least one of currently quality of life (QoL) data for the user, one or more QoL goals for the user, one or more symptoms experienced by the user, and user lifestyle data;

performing an automatic analysis and modelling of the USTD upon a computer system in dependence upon at least the user data;
establishing a design of the USTD in dependence upon the automatic analysis and modelling of the USTD;
establishing clinician approval of the design of the USTD via a clinical decision confirmation system (CDCS); and
manufacturing the USTD to the design of the USTD approved by the clinician.

Other aspects and features of the present invention will become apparent to those ordinarily skilled in the art upon review of the following description of specific embodiments of the invention in conjunction with the accompanying figures.

## BRIEF DESCRIPTION OF THE DRAWINGS

Embodiments of the present invention will now be described, by way of example only, with reference to the attached Figures, wherein:

Figure 1 depicts an exemplary process flow for providing a user with a user specific therapeutic device (USTD) according to an embodiment of the invention;
Figure 2 depicts schematically the measurements, modelling, and fitting of a USTD according to embodiments of the invention for two different patients;
Figure 3 depicts an exemplary USTD platform and system supporting embodiments of the invention;
Figure 4 depicts three measurement and image analysis process flows employed within a USTD platform according to an embodiment of the invention and the resulting parameters established with respect to a patient's physiology;
Figure 5A depicts an exemplary process flow within a USTD platform according to an embodiment of the invention using the parameters established with respect to a patient's physiology in Figure 4 to establish the design of a USTD for the patient according to an embodiment of the invention;
Figure 5B depicts an exemplary flow for generating a pessary registry allowing 3 methods of creating USTD according to an embodiment of the invention as employed in Figure 5A;
Figure 6 depicts an exemplary USTD ring pessary together with parameters established within the exemplary process flow of Figure 5A to establish the USTD for the patient;
Figure 7 depicts a simplified schematic of a vaginal manometry system (VAMA system) employed in establishing patient physiology;
Figure 8 depicts an exemplary configuration according to an embodiment of the invention for patient physiology characterization combining a VAMA system with ultrasound imaging and robotic manipulation within a seat;
Figure 9 depicts an exemplary configuration according to an embodiment of the invention for patient physiology characterization combining a VAMA system with ultrasound imaging and robotic manipulation;
Figure 10 depicts exemplary manometry bag (or manometry balloon) configurations for a VAMA system according to an embodiment of the invention for patient physiology characterisation;
Figure 11A depicts an exemplary manometry balloon and catheter configuration according to an embodiment of the invention for patient physiology characterisation;
Figure 11B depicts exemplary manometry balloon and catheter configuration employing dual balloons according to an embodiment of the invention for patient physiology characterisation;
Figure 12 depicts schematically a structural shield approach for use in conjunction with a VAMA system according to embodiments of the invention for automatically retaining the manometry balloon during patient physiology characterisation;
Figure 13 depicts a three dimensional rendering of a structural shield and Outer Cushion such as that employed in Figure 12 according to an embodiment of the invention;
Figure 14 depicts different exemplary structural shield designs for ultrasound imaging integration with a VAMA system according to embodiments of the invention for automatically retaining the manometry balloon and define placement of the ultrasound probe head during patient physiology characterisation;

Figure 15 depicts exemplary structural shield designs according to embodiments of the invention;

Figure 16 depicts an exemplary design for "membrane clothing" integrating a structural shield and/or ultrasound probe holder according to embodiments of the invention;

Figure 17 depicts an inflatable support according to an embodiment of the invention supporting use with a VAMA system in conjunction with a seat for supporting the anterior portion of the patient with integral ultrasound probe window or holder according to an embodiment of the invention;

Figure 18 depicts an inclined cushion for the anterior support of the patient according to an embodiment of the invention use with a VAMA system in conjunction with a seat for supporting the anterior portion of the patient with integral ultrasound probe window or holder according to an embodiment of the invention;

Figure 19 depicts a vaginal manometry plug according to an embodiment of the invention for retaining the manometry balloon within the vaginal canal during VAMA system characterization of a patient's physiology in conjunction with a seat for supporting the anterior portion of the patient with integral ultrasound probe window or holder according to an embodiment of the invention;

Figure 20 depicts a vaginal manometry plug according to an embodiment of the invention for retaining the manometry balloon within the vaginal canal during VAMA system characterization of a patient's physiology;

Figure 21 depicts an exemplary geometry of a cup according to an embodiment of the invention for retaining the manometry balloon within the vaginal canal during VAMA system characterization of a patient's physiology in conjunction with an access port for an ultrasound probe;

Figure 22 depicts the measurements of a demographic patient set employed in the design of the cup depicted in Figure 21;

Figure 23 depicts computer aided design (CAD) schematics of cups according to embodiments of the invention;

Figure 24 depicts membrane underwear according to an embodiment of the invention allowing coupling of a probe to the user's body for obtaining anatomical images and measurements to support other embodiments of the invention;

Figures 25 to 27B depict exemplary prototype membrane underwear according to embodiments of the invention;

Figure 28 depicts cloud components according to an embodiment of the invention supporting an exemplary USTD platform and system such as depicted in Figure 3;

Figure 29 depicts an exemplary system diagram for fitting USTD devices according to embodiments of the invention that includes sensors to measure strain, force, and/or pressure;

Figure 30A depicts a USTD ring pessary device according to embodiments of the invention;

Figure 30B depicts USTD ring pessary devices with pull-tabs according to embodiments of the invention;

Figure 31A depicts a USTD Gellhorn style pessary device according to embodiments of the invention;

Figure 31B depicts a USTD Gellhorn style with pull-tab according to embodiments of the invention;

Figure 32 depicts a USTD traditional Marland style ring pessary devices according to embodiments of the invention;

Figure 33 depicts a USTD double ring Marland style ring pessary device according to embodiments of the invention;

Figure 34 depicts an air-pocket design integrated into prior art Gellhorn style pessary device to facilitate ease of removal;

Figure 35 depicts a USTD Gellhorn style pessary device according to embodiments of the invention with pull tab and stem for ease of removal;

Figure 36 depicts an exemplary configuration for a pelvic floor scanner probe placement system according to an embodiment of the invention with automated detection of anatomical features and positioning of the sensor probe;

Figure 37 depicts an exemplary process flow for the automated analysis of hiatus dimensions based upon three-dimensional transperineal ultrasound measurements;

Figure 38 depicts an automated adjustment for a manometry system according to embodiments of the invention;

Figure 39 depicts an exemplary pelvic floor ultrasound midsagittal plane illustrating key anatomies as employed within embodiments of the invention;

Figure 40 depicts an exemplary probability mask created using 35 hand labeled pubic synthesis (PS) datasets;

Figure 41 depicts positive and negative edge maps, detected urethra edges together with extracted PS landmark and anorectal angle (ARA) landmark using a processing pipeline according to an embodiment of the invention;

Figure 42 depicts an exemplary workflow for automatic plane of minimal hiatal dimensions (PMHD) extraction method from 3D ultrasound (US) pelvic floor images according to an embodiment of the invention;

Figure 43 depicts landmark detection and PMHD extraction images relating to an exemplary workflow for automatic PMHD extraction method from 3D ultrasound (US) pelvic floor images according to an embodiment of the invention through midsagittal plane images, detected landmarks of PS and ARA, extracted PMHD and PMHD groundtruth;

Figure 44 depicts exemplary error metrics from an exemplary workflow for automatic PMHD extraction method from 3D ultrasound (US) pelvic floor images according to an embodiment of the invention for absolute landmark distance, relative landmark distance, and plane angle difference;

Figure 45 depicts extracted PMHD and hiatus segmentations for 15 patients using an exemplary workflow for automatic PMHD extraction method from 3D ultrasound (US) pelvic floor images according to an embodiment of the invention;

Figure 46 depicts scatter plots showing the distribution of the resulting Sørensen-Dice Coefficient (DICE) and mean boundary distance (MBD) from an exemplary workflow for automatic PMHD extraction method from 3D ultrasound (US) pelvic floor images according to an embodiment of the invention;

Figure 47 depicts exemplary illustrations of the segmentation outliers for groundtruth PMHD, extracted PMHD, and their overlay arising from an exemplary workflow for automatic PMHD extraction method from 3D ultrasound (US) pelvic floor images according to an embodiment of the invention;

Figure 48 depicts an exemplary device for fitting USTD devices according to embodiments of the invention that includes sensors to measure strain, force, and/or pressure; and

Figure 49 depicts an exemplary device for fitting USTD devices according to embodiments of the invention that includes sensors to measure strain, force, and/or pressure.

## DETAILED DESCRIPTION

The present invention is directed to vaginal devices and more specifically to devices as well as methods and systems for the retention of such devices, the devices being for measurement, diagnostics, therapy, or treatment.

The ensuing description provides representative embodiment(s) only, and is not intended to limit the scope, applicability, or configuration of the disclosure. Rather, the ensuing description of the embodiment(s) will provide those skilled in the art with an enabling description for implementing an embodiment or embodiments of the invention. It being understood that various changes can be made in the function and arrangement of elements without departing from the spirit and scope as set forth in the appended claims. Accordingly, an embodiment is an example or implementation of the inventions and not the sole implementation. Various appearances of "one embodiment," "an embodiment" or "some embodiments" do not necessarily all refer to the same embodiments. Although various features of the invention may be described in the context of a single embodiment, the features may also be provided separately or in any suitable combination. Conversely, although the invention may be described herein in the context of separate embodiments for clarity, the invention can also be implemented in a single embodiment or any combination of embodiments.

Reference in the specification to "one embodiment", "an embodiment", "some embodiments" or "other embodiments" means that a particular feature, structure, or characteristic described in connection with the embodiments is included in at least one embodiment, but not necessarily all embodiments, of the inventions. The phraseology and terminology employed herein is not to be construed as limiting but is for descriptive purpose only. It is to be understood that where the claims or specification refer to "a" or "an" element, such reference is not to be construed as there being only one of that element. It is to be understood that where the specification states that a component feature, structure, or characteristic "may", "might", "can" or "could" be included, that particular component, feature, structure, or characteristic is not required to be included.

Reference to terms such as "left", "right", "top", "bottom", "front" and "back" are intended for use in respect to the orientation of the particular feature, structure, or element within the figures depicting embodiments of the invention. It would be evident that such directional terminology with respect to the actual use of a device has no specific meaning as the device can be employed in a multiplicity of orientations by the user or users. Reference to terms "including", "comprising", "consisting" and grammatical variants thereof do not preclude the addition of one or more components, features, steps, integers, or groups thereof and that the terms are not to be construed as specifying components, features, steps, or integers. Likewise, the phrase "consisting essentially of", and grammatical variants thereof, when used herein is not to be construed as excluding additional components, steps, features integers or groups thereof but rather that the additional features, integers, steps, components, or groups thereof do not materially alter the basic and novel characteristics of the claimed composition, device, or method. If the specification or claims refer to "an additional" element, that does not preclude there being more than one of the additional element.

"Artificial intelligence" (AI, also machine intelligence, MI) as used herein may refer to, but is not limited to, intelligence exhibited by machines rather than humans or other animals which exhibit so-called natural intelligence (NI). Colloquially, the term AI is employed when a machine mimics "cognitive" functions which humans associate with other human minds, such as "learning" and "problem solving". AI may employ one or more tools, including, but not limited to search and optimization, logic, probabilistic methods for uncertain reasoning, classifiers and statistical learning methods, neural networks, deep feedforward neural networks, deep recurrent neural networks, and control theory.

A "portable electronic device" (PED) as used herein and throughout this disclosure, refers to a wireless device used for communications and other applications that requires a battery or other independent form of energy for power. This includes devices, but is not limited to, such as a cellular telephone, smartphone, personal digital assistant (PDA), portable computer, pager, portable multimedia player, portable gaming console, laptop computer, tablet computer, a wearable device, an electronic reader, a vaginal therapy device (VTD), and a user specific therapeutic device (USTD).

A "fixed electronic device" (FED) as used herein and throughout this disclosure, refers to a wireless and /or wired device used for communications and other applications that requires connection to a fixed interface to obtain power. This includes, but is not limited to, a laptop computer, a personal computer, a computer server, a kiosk, a gaming console, a digital set-top box, an analog set-top box, an Internet enabled appliance, an Internet enabled television, and a multimedia player.

An "application" (commonly referred to as an "app") as used herein may refer to, but is not limited to, a "software application", an element of a "software suite", a computer program designed to allow an individual to perform an activity, a computer program designed to allow an electronic device to perform an activity, and a computer program designed to communicate with local and / or remote electronic devices. An application thus differs from an operating system (which runs a computer), a utility (which performs maintenance or general-purpose chores), and a programming tools (with which computer programs are created). Generally, within the following description with respect to embodiments of the invention an application is generally presented in respect of software permanently and / or temporarily installed upon a PED and / or FED.

A "user" as used herein may refer to, but is not limited to, an individual exploiting a vaginal therapeutic device according to an embodiment or embodiments of the invention. As such an individual may be employing a vaginal therapeutic device with respect to one or more conditions, requirements, and/or preventions. As such an individual may include, but not be limited to, a person with a vagina, an animal with a vagina, a recipient of gender affirming surgery (also known as sex reassignment surgery, gender confirmation surgery, gender specific reconstruction surgery, and sex realignment surgery). In its broadest sense the user may further include, but not be limited to, mechanical systems, robotic systems, android systems, etc. that may be characterised by a requirement to exploit one or more embodiments of the invention. A user may be associated with biometric data which may be, but not limited to, monitored, acquired, stored, transmitted, processed and analysed either locally or remotely to the user. A user may also be associated through one or more accounts and / or profiles with one or more of a service provider, third party provider, enterprise, social network, social media etc. via a dashboard, web service, website, software plug-in, software application, and graphical user interface.

The terms "woman" or "female" as used herein, and throughout this disclosure, refers to a human having a vagina or surgically formed vaginal structure and optionally a clitoris or clitoral region, uterus, bladder, a urethra, rectum, and/or an anus. The terms "woman" and "female" are used interchangeably herein.

"User information" as used herein may refer to, but is not limited to, user behavior information and / or user profile information. It may also include a user's biometric information, an estimation of the user's biometric information, or a projection / prediction of a user's biometric information derived from current and / or historical biometric information.

A "vaginal therapeutic device" (VTD, commonly referred to as a pessary) refers to a medical device and is a specific form of a user specific therapeutic device (USTD). A USTD may be used to support the uterus, vagina, bladder, or rectum. A USTD may be employed to treat a pelvic organ prolapse (POP), such as prolapse of the uterus for example, treat an intestinal issue, an enterocele (essentially a vaginal hernia), reduce the impact of an evolving POP, treat and/or reduce the impact of urinary incontinence (UI), treat and/or reduce the impact of stress UI, and treat and/or reduce the impact of urge UI. Alternatively, a USTD may be employed during pregnancy to treat an incompetent (or insufficient) cervix (cervix starts to shorten and open too early) as an alternative to cervical cerclage since there are fewer potential complications. A USTD may also be used to address constipation, fecal incontinence, retroverted uterus, address cystocele, address rectocele, manage menstruation, induce an abortion, or provide and/or support contraception. A USTD may be placed temporarily or permanently. A pharmaceutical USTD may provide an effective means for the delivery of one or more pharmaceutical substances which are easily absorbed through the mucosa of the vagina, or intended to have action in the locality, for example to delivery hormones or act against inflammation or infection, or on the uterus. An occlusive USTD may perform similarly to a cervical cap and may be used in combination with spermicide as a contraception. A stem USTD, a type of occlusive USTD, is an early form of the cervical cap shaped like a dome to cover the cervix but with a central rod or "stem" entering the os to hold it in place. USTD's within the prior art are offered in a variety of forms including, but not limited, ring USTDs, lever USTDs, Gehrung USTDs, inflatable USTDs, doughnut USTDs, cube USTDs, Gellhorn USTDs, and incontinence USTDs. USTDs according to embodiments of the invention are designed in dependence upon the user for custom fitting and/or applications including, but not limited to, prolapse, urinal incontinence, and fecal incontinence.

"Gender affirming surgery" (also known as gender reassignment surgery, gender confirmation surgery, genital reconstruction surgery, gender-affirming surgery, or gender realignment surgery) as used herein may refer to, but is not limited to, one or more surgical procedures that adjust a user's physical appearance and function with respect to their genitalia which may require the user to use a vaginal therapeutic device according to an embodiment of the invention.

A "wearable device" or "wearable sensor" relates to miniature electronic devices that are worn by the user including those under, within, with or on top of clothing and are part of a broader general class of wearable technology which includes "wearable computers" which in contrast are directed to general or special purpose information technologies and media development. Such wearable devices and / or wearable sensors may include, but not be limited to, smartphones, smart watches, e-textiles, smart shirts, activity trackers, smart glasses, environmental sensors, medical sensors, biological sensors, physiological sensors, chemical sensors, ambient environment sensors, position sensors, neurological sensors, drug delivery systems, medical testing and diagnosis devices, and motion sensors. The wearable devices and / or wearable sensors may include, but not be limited to, devices that can stimulate and/or measure parameters related to the function of the vagina, urethra, uterus, bladder, cervix, rectum, anal sphincter, urethral sphincter, and abdominal cavity. It may also be used to measure intra-abdominal pressure which can be correlated to the amount of force that the USTD will need to support.

"Biometric" information as used herein may refer to, but is not limited to, data relating to a user characterised by data relating to a subset of conditions including, but not limited to, their environment, medical condition, biological condition, physiological condition, chemical condition, ambient environment condition, position condition, neurological condition, drug condition, and one or more specific aspects of one or more of these said conditions. Accordingly, such biometric information may include, but not be limited, blood oxygenation, blood pressure, blood flow rate, heart rate, temperate, fluidic pH, viscosity, particulate content, solids content, altitude, vibration, motion, perspiration, EEG, ECG, energy level, etc. In addition, biometric information may include data relating to physiological characteristics related to the shape and / or condition of the body wherein examples may include, but are not limited to, fingerprint, facial geometry, baldness, DNA, hand geometry, odour, and scent. Biometric information may also include data relating to behavioral characteristics, including but not limited to, typing rhythm, gait, and voice.

A "profile" as used herein, and throughout this disclosure, refers to a computer and/or microprocessor readable data file comprising data relating to a USTD according to an embodiment of the invention and/or biometric data of a user.

A "scaffold" or "scaffolds" as used herein, and throughout this disclosure, refers to a structure that is used to hold up, interface with, or support another material or element(s). This includes, but is not limited to, such two-dimensional (2D) structures such as substrates and films, three-dimensional (3D) structures such as geometrical objects, non-geometrical objects, combinations of geometrical and non-geometrical objects, naturally occurring structural configurations, and manmade structural configurations. A scaffold may be solid, hollow, and porous or a combination thereof. A scaffold may contain recesses, pores, openings, holes, vias, and channels or a combination thereof. A scaffold may be smooth, textured, have predetermined surface profiles and / or features. A scaffold may be intended to support one or more other materials, one or more films, a multilayer film, one type of particle, multiple types of particles etc. A scaffold may include, but not be limited to, a spine of a device and / or a framework, for example, which also supports a shell and / or a casing.

A "shell" as used herein, and throughout this disclosure, refers to a structure that is used to contain and / or surround at least partially and / or fully a number of elements within devices according to embodiments of the invention. A shell may include, but not limited to, a part or parts that are mounted to, attached to, and/or surround all or part of a scaffold or scaffolds that support elements within a device according to an embodiment of the invention.

A "casing" or "skin" as used herein, and throughout this disclosure, refers to a structure surrounding a scaffold and / or shell. This includes structures typically formed from an elastomer and / or silicone to provide a desired combination of physical tactile surface properties to the device it forms part of and other properties including, but not limited to, hermeticity, liquid ingress barrier, solid particulate ingress barrier, surface sheen, and colour. A casing may include, but not limited to, a part or parts that are mounted to a scaffold or scaffolds and / or a casing or casings forming part of a device according to an embodiment of the invention.

A "resin" as used herein may refer to, but is not limited to, a solid or highly viscous substance which is typically convertible into polymers. Resins may be plant-derived or synthetic in origin.

A "polymer" as used herein may refer to, but is not limited to, is a large molecule, or macromolecule, composed of many repeated subunits. Such polymers may be natural and synthetic and typically created via polymerization of multiple monomers. Polymers through their large molecular mass may provide unique physical properties, including toughness, viscoelasticity, and a tendency to form glasses and semi-crystalline structures rather than crystals.

A "polyester" as used herein, and throughout this disclosure, refers to a category of polymers that contain the ester functional group in their main chain. This includes but is not limited to polyesters which are naturally occurring chemicals as well as synthetics through step-growth polymerization, for example. Polyesters may be biodegradable or not. Polyesters may be a thermoplastic or thermoset or resins cured by hardeners. Polyesters may be aliphatic, semi-aromatic or aromatic. Polyesters may include, but not be limited to, those exploiting polyglycolide, polylactic acid (PLA), polycaprolactone (PCL), polyhydroxyalkanoate (PHA), polyhydroxybutyrate (PHB), polyethylene adipate (PEA), polybutylene succinate (PBS), polyethylene terephthalate (PET), polybutylene terephthalate (PBT), polytrimethylene terephthalate (PTT), and polyethylene naphthalate (PEN).

A "thermoplastic" or "thermo softening plastic" as used herein and throughout this disclosure, refers to a category of polymers that become pliable or moldable above a specific temperature and solidify upon cooling. Thermoplastics may include, but not be limited, polycarbonate (PC), polyether sulfone (PES), polyether ether ketone (PEEK), polyethylene (PE), polypropylene (PP), poly vinyl chloride (PVC), polytetrafluoroethylene (PTFE), polyimide (PI), polyphenylsulfone (PPSU), polychlorotrifluoroethene (PCTFE or PTFCE), fluorinated ethylene propylene (FEP), and perfluoro alkoxy alkane (PFA).

An "aramid" as used herein, and throughout this disclosure, refers to an aromatic polyamide. Aramids are a class of materials fibers in which the chain molecules are highly oriented along the fiber axis, so the strength of the chemical bond can be exploited. Examples include but are not limited to fibers distributed under brand names such as Kevlar™, Technora™, Twaron™, Heracron™, Nomex™, Innegra S™ and Vectran™ as well as nylon and ultra-high molecular weight polyethylene.

A "silicone" as used herein, and throughout this disclosure, refers to a polymer that includes any inert, synthetic compound made up of repeating units of siloxane.

An "elastomeric" material or "elastomer" as used herein, and throughout this disclosure, refers to a material, generally a polymer, with viscoelasticity. Elastomers may include, but not be limited to, unsaturated rubbers such as polyisoprene, butyl rubber, ethylene propylene rubber, silicone rubber, fluorosilicone rubber, fluoroelastomers, perfluoroelastomers, and thermoplastic elastomers.

The term "flexible," as used herein, refers to the ability of a body that is capable of being bent or flexed and refers to the ability of a body that has been subjected to an external force to return to its original size and/or shape once the external force has been removed or reduced to below a particular level. Something that is flexible can be, for example, resilient or malleable. A "flexible" material, such as a rubber for example, may be characterised by a low Young's modulus.

The term "resilient," as used herein, refers to the ability of a body that has been subjected to an external force to recover, or substantially recover, its original size and/or shape, following deformation. The term "malleable," as used herein, refers to the ability of a body that has been subjected to an external force to deform and maintain, or substantially maintain, the deformed size and/or shape. Accordingly, a malleable material supports plastic deformation. A resilient material, such as polytetrafluoroethylene for example, may be characterised by a moderate Young's modulus. A rigid material, for example steel, may be characterised by a high Young's modulus but may under appropriate conditions undergo plastic deformation.

A "CAD model" as used herein may refer to, but is not limited to, an electronic file containing information relating to a component, piece-part, element, assembly to be manufactured. A CAD model may define an object within a two-dimensional (2D) space or a three-dimensional (3D) space and may in addition to defining the internal and / or external geometry and structure of the object include information relating to the material(s), process(es), dimensions, tolerances, etc. Within embodiments of the invention the CAD model may be generated and transmitted as electronic content to a system providing manufacturing according to one or more embodiments of the invention. Within other embodiments of the invention the CAD model may be derived based upon one or more items of electronic content directly, e.g. a 3D model may be created from a series of 2D images or extracted from electronic content.

A "fluid" as used herein may refer to, but is not limited to, a substance that continually deforms (flows) under an applied shear stress. Fluids may include, but are not limited to, liquids, gases, plasmas, and some plastic solids.

A "powder" as used herein may refer to, but is not limited to, a dry, bulk solid composed of a large number of very fine particles that may flow freely when shaken or tilted. Powders may be defined by both a combination of the material or materials they are formed from and the particle dimensions such as minimum, maximum, distribution etc. A powder may typically refer to those granular materials that have fine grain sizes but may also include larger grain sizes depending upon the dimensions of the part being manufactured, the characteristics of the additive manufacturing system etc.

"Additive manufacturing" (AM) as used herein may refer to, but is not limited to, a process or processes used to create a three-dimensional object in which layers of material are formed under computer control. Commonly referred to as "3D printing" the processes of AM are currently defined in ISO/ASTM52900-15, which defines several categories of AM processes although others may also be viewed as AM processes. These categories being binder jetting, directed energy deposition, material extrusion, material jetting, powder bed fusion, sheet lamination and vat photopolymerization. "3D printers" exploiting custom "inkjet" print heads are a special application of plastic extrusion known as fused deposition modelling. AM processes may be applied to plastics, ceramics, and metals. AM processes for AM sintering or melting include selective laser sintering, direct metal laser sintering, and selective laser melting whilst those for deposition may include microcasting and sprayed materials. In some instances, sacrificial and/or support materials may be employed in conjunction with AM processes to achieve the desired geometry and/or combination of materials.

"Non-additive manufacturing" (NAM) as used herein may refer to, but is not limited to, a process or processes used to create a three-dimensional object by subtractive or transformative manufacturing. NAM processes may include, but not be limited to, hydro-forming, stamping, injection molding, casting, machining, and welding.

As noted above a range of surgical treatment options for POP as well as non-surgical treatments exist. Non-surgical treatment options include Kegel exercises, Kegel assist devices, pessaries, core/floor strengthening exercises, biofeedback, and support garments. Electrical stimulation, hormone replacement therapy and tibial nerve stimulation provide non-surgical treatment options to other pelvic floor disorders (PFDs). At present, physical non-surgical devices such as pessaries are fitted today by best guess, trial-and-error. Whilst the literature is replete with information explaining to medical personnel how to fit a pessary or users how to tell if their pessary correctly fits this potentially exacerbates the situation as there are at least 20 designs of pessary alone each available in multiple sizes, in some instances 10 or more. So, type of pessary and size both need to be factored into addressing the correct device for a user.

As discussed by the inventors previously, see for example World Patent Application 2019/051,579 entitled "Methods and Systems for Vaginal Therapeutic Device Fitting", it would be beneficial to replace the current manual processes of sizing, fitting etc. as well as pessary design with a personal pelvic health characterization and provisioning approach that factors user specific anatomy and physiology, user life style, user experiences, automated assessments etc. into provisioning custom vaginal therapeutic devices.

Accordingly, the inventors have established custom USTD processes wherein core advantages include simplifying the fitting process as well as establishing a new paradigm between the two characteristics of support and comfort which runs counter to prior art USTDs the more support the less comfortable, less prone to expulsion during exercise, or tissue erosion over long term. Accordingly, the process established by the inventors resets this paradigm through a custom fitting and manufacturing process with a single material or multiple material USTD design allowing support to be established from a scaffold within the device whilst a shell and/or skin around the scaffold provide for increased comfort. Further, adoption of additive manufacturing processes allows the custom USTD manufacturing to be established in multiple locations with a city, state, province, country allowing improved delivery, responsiveness and supporting exploitation of custom USTDs with reduced usage duration as they exploit anti-microbial coatings, contraceptive coating, etc.

Accordingly, the user specific anatomy and physiology should be obtained in a reproducible manner with devices and systems that remove measurement artifacts, errors, bias etc. whilst providing the patient with an improved experience and the medical personnel with ergonomic, efficient, and easy to use systems exploiting combinations of dedicated multi-patient measurement equipment with user specific consumable items for cleanliness etc.

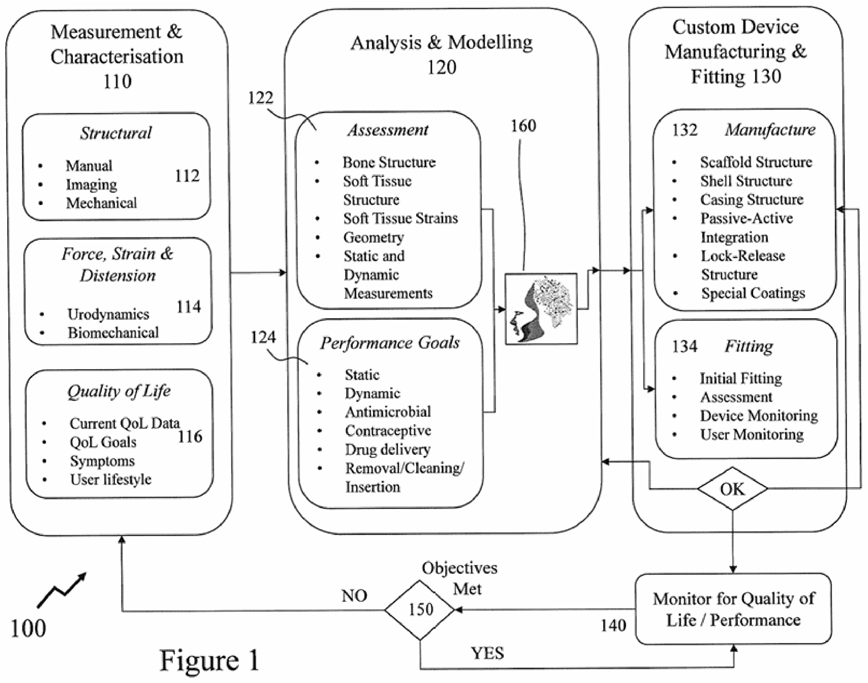

Accordingly, referring to Figure 1 there is depicted an exemplary process Flow 100 for providing a user with a custom USTD according to an embodiment of the invention such that the process is reduced from a bewildering array of USTD types and dimensions to a single USTD option without significant effort from either the patient or the clinician. Accordingly, at step 110 the process begins with the step of Measurement and Characterisation (M&C) 110 before progressing to Analysis and Modelling (A&M) 120 and Custom Device Manufacturing and Fitting (CUDEMAF) 130 wherein the patient (user) is now provided and fitted with a custom USTD. Next, the process proceeds to step 140 wherein ongoing monitoring of quality of life (QoL) and performance of the USTD wherein a decision process 150 may determine whether the objectives of the USTD are being met or still being met on an ongoing basis and hence determine whether monitoring should continue or whether the process should begin again with step 110.

An ongoing monitoring and cyclic process may be appropriate for a variety of USTD use cases including, but not limited to:

- changing physical characteristics of the user as they get older which may be more gradual in older users such as adults or the elderly and more rapid in younger users;
- changing physiology of the user wherein additional symptoms and/or conditions manifest themselves;
- changing physiology of the user in that muscles and tissue resilience, strength, compliance etc. may change; and
- degradation in the USTD itself.

Accordingly, as depicted M&C 110 comprises three sub-processes, these being:

- Structural 112;
- Force, Strain and Distension 114; and
- Quality of Life 116.

Within embodiments of the invention the custom USTD may be employed in combination with other therapies and/or pharmaceutical coatings etc. in order to combine a custom USTD with regenerative medicine. Accordingly, within other embodiments of the invention a USTD according to an embodiment of the invention may exploit an energy delivery system such as electrical stimulation, infrared irradiation or ultraviolet irradiation for example. A custom USTD may also be employed in conjunction with other medical procedures and/or treatment regimens including, for example, exploitation of biological therapies including recombinant proteins, recombinant peptides, and stem cells for example.

Structural 112 may comprise one or more measurements of the user's anatomy and/or measurements of the user's physical characteristics such that one or more characteristics such as the dimensions of the user's major anatomical structures, anatomical geometry, etc. are defined. For example, a Pelvic Organs Prolapse Quantification (POP-Q) may be performed, this being a standardized tool for documenting the examination findings recognized by International Continence Society (ICS) and International Urogynecological Association (IUGA). Within the POP-Q system six principal landmarks are defined to describe the degree (quantity) of Pelvic Organ Prolapse (POP). These points (Aa, Ba, C, D, Ap, Bp) are located on vaginal walls and cervix and are related to the hymen which is considered a fixed point of reference. Another three distances (GH, TVL, and PB) may also be defined for more detailed description. The "stage" of prolapse is typically defined according to the evaluation of these points. These nine points are defined by letters Aa, Ba, C, D, Ap, Bp, GH, TVL, and PB respectively, these being:

- Point Aa: This point is located in the midline anterior vaginal wall approximately 3cm proximal from the external urethral meatus. The range of its position relative to hymen is typically from -3cm to +3cm.

- Point Ba: The most distal position of any part in the anterior vaginal wall from the vaginal cuff or anterior vaginal fornix to Point Aa. In absence of prolapse, this point is at -3cm.
- Point C: The most distal edge of the cervix or vaginal cuff (hysterectomy scar) after total hysterectomy.
- Point D: Represents the pouch of Douglas or the location of posterior vaginal fornix. It is also a point of measurement for differentiation apical compartment prolapse from cervical elongation. Accordingly, in the absence of a cervix point D is omitted.
- Point Ap: Located in the middle of posterior vaginal wall 3cm proximal to the hymen. The range of its position relative to hymen is typically from -3cm to +3cm.
- Point Bp: Represents the most distal position of any part in the posterior vaginal wall from the vaginal cuff or posterior vaginal fornix to point Ap.
- Genital hiatus (GH): The distance between external urethral meatus and posterior margin of the hymen.
- Total Vaginal Length (TVL): The deepest length of the vagina (cm) measured when point D (or the vaginal cuff) are reduced to normal position.
- Perineal Body (PB): The distance measured from posterior margin of the hymen to the mid-anal opening.

All measurements are taken on Valsalva except TVL. A clinician may employ a manual procedure to measure the basic six or full nine points Aa, Ba, C, D, Ap, Bp, GH, TVL, and PB, respectively. This may be via the use of a ruler, swab, or other mechanical measuring device. The necessary user-specific structural/anatomical parameters may also be derived from one or more imaging techniques including, but not limited to, ultrasound imaging, magnetic resonance imaging (MRI), elastography, acoustic analysis, tactile imaging, photoacoustic (optoacoustic) imaging, tomography, echocardiography, functional near-infrared spectroscopy, and electrical impedance tomography. Alternatively, mechanical based devices may be employed to perform measurements and/or support one or more transducers for one or more imaging techniques, manual processes etc. Further these measurements may be at least one intravaginal, translabial, and transperineal.

For example, within an embodiment of the invention, ultrasound imaging may be used to determine specific anatomic parameters such as cross-sectional diameter of the vagina at various cross-sections along its length. Distances between various anatomical structures may also be used to determine specific anatomical parameters including but not limited to distances between any of the following anatomical structures: pubic symphysis, cervix (anterior lip, posterior lip, or os), urethra, bladder neck, bladder, rectum, anus, or levator ani and other pelvic floor musculature. Importantly, mobility of the various anatomical structures may also be measured by obtaining measurements at rest and on maximum Valsalva and contraction. These mobility measures help characterize the prolapsing compartment(s) and have been correlated with patient's symptoms. For example, bladder descent greater than 1cm below the pubic symphysis on Valsalva is correlated with symptoms of prolapse. In one embodiment, these data may be used to generate a prosthetic that optimally fits within the vagina and limits bladder descent and thereby minimizes patients' symptoms. In addition to distances, thicknesses, surface area, and shape of the vaginal wall may also be assessed using ultrasound. Thickness data may be used to customize the mechanical properties and shape of the prosthetic device such that it minimizes the risk for vaginal ulcerations and erosions.

Force, Strain and Distension 114 may comprise one or more measurements of characteristics of the user's anatomy and/or measurements of the user's physical characteristics such as compliance / resilience of the user's tissues, the movement(s) and strength of user's musculature within the appropriate anatomical regions. These may involve mechanical and/or imaging testing discretely or in combination with other tests. Such tests may include, but not be limited to:

- Vaginal manometry.
- Vaginal distension with imaging from any imaging modality such as, but not limited to, ultrasound, magnetic resonance imaging (MRI), endoscope, LIDAR and X-ray for example.
- Urodynamic measurements including, but not limited to:

    ○ Post-void residual volume via insertion of a urinary catheter or with the aid of a bladder scanner;
    ○ Uroflowmetry where "free" uroflowmetry measures the rate of bladder evacuation, "pressure" uroflowmetry combines rate of voiding measurements with simultaneous assessment of bladder and rectal pressures;
    ○ Multichannel cystometry which exploits a pair of pressure monitoring catheters to measure the pressure in the rectum and in the bladder to deduce the presence of contractions of the bladder wall, during bladder filling, or during other provocative maneuvers. The strength of the urethra can also be tested during the filling phase, using a cough or Valsalva maneuver, to confirm genuine stress incontinence; and
    ○ Urethral pressure profilometry which measures the strength of sphincter contraction.

- Tactile imaging for force and strain measurements.
- Elastography from ultrasound or MRI as well as other intravaginal measurements and perineal measurements.

- Electromyography (EMG) measurements of electrical activity of the pelvic floor muscles.
- Fluoroscopy, dynamic X-ray sequences, of the bladder and bladder neck during voiding.
- Intravaginal molding or casts.
- Finger palpation

Techniques may include those identified *supra* and others including, but not be limited, leak point pressure, vaginal manometry, ultrasound, elastography, strain sensor array, acoustic analysis, tactile imaging, and photoacoustic (optoacoustic) imaging. The measurements performed within Structural 112 and Force, Strain and Distension 114 may be statically acquired, i.e. with the user sitting / laying / standing within a clinic or another environment and/or dynamically acquired with the user performing one or more routine aspects of their life such as Valsalva effort, walking, exercising, running, lifting, bending, etc.

In contrast to the Structural 112 and Force, Strain and Distension 114 the Quality of Life 116 is user reported assessment. Accordingly, Quality of Life (QoL) 116 may include, but not limited to:

- Current QoL data for the user (patient) using validated questionnaires such as the Pelvic Floor Distress Inventory (PFDI) and Pelvic Floor Impact Questionnaire (PFIQ) for example;
- QoL goals for the user (patient);
- Symptoms experienced by the user; and
- User lifestyle.

Accordingly, QoL 116 establishes baseline QoL data which may be employed subsequently for the monitoring, QoL and performance of the USTD once manufactured and employed according to embodiments of the invention. Accordingly, for one user a QoL goal may be the elimination of a symptom that occurs only during sexual activity whilst for another it may be during a specific exercise, sporting activity, etc. or for another over specific periods of time and/or generally monitored etc. Additionally, the USTD in terms of being permanent, semi-permanent, or temporary is established wherein for temporary use at least the installation / removal means and/or mechanisms are established with the user. For permanent and semi-permanent the installation / removal means are geared primarily to the clinician rather than the user.

In establishing the QoL 116 a user may employ an application upon a PED and/or FED in order to track the user's (patient's) perceived QoL, to monitor and/or log occurrences such as incontinence, pain, prolapse symptoms, pessary fall out, etc.

From M&C 110 the process proceeds to A&M 120 wherein sub processes of Assessment 122 and Performance Goals 124 are undertaken. Within Assessment 122 the data obtained within the M&C 110 step are analysed, for example, through their entry into a human body (anatomical) model (HBM) to define a series of two-dimensional (2D) and/or three-dimensional (3D) perspectives of the user's anatomy. These 2D and 3D perspectives and modelling / analysis with the HBM may exploit one or more machine learning processes and/or algorithms and/or systems. Additionally, other parameters and/or aspects including, but not limited to, the following may be included:

- Bone structure definition;
- Soft tissue structure definition;
- Soft tissue strains;
- Relative positions of bones and/or soft tissues and/or surrounding organs;
- Static body position in one or more position such as supine, sitting, and standing, for example;
- Dynamic body position such as walking, bending, squatting, lifting, and jogging, for example;
- Dynamic forces and structural measurements; and
- Dynamic pressure from activities such as cough and Valsalva, for example.

Within Performance Goals 124 the QoL 116 data is established as specific static and dynamic performance goals for the USTD. These may include, but not be limited to, whether the USTD is to address urinary and/or fecal incontinence, number of episodes and volume, degree of comfort level required, will or can the user perform self-removal / cleaning / insertion etc., will this require periodic visits to a physician or clinic, and will any coatings require the user periodically dispose of the USTD and use a new USTD. Additionally, additional characteristics may be established with respect to providing an antimicrobial coating, providing controlled pharmaceutical product release(s) such as combinations of estrogen and progesterone for contraception, estrogen for treatment of genitourinary syndrome of menopause, spermicide, proteins, regenerative medicine(s) or other drugs for the user. These together with the data from Assessment 122 are employed in defining the custom USTD for the user in terms of physical geometry, e.g. dimensions of any ring structure, knob, support etc. Additionally, the mechanical properties of the custom USTD are defined in respect of the flexibility, dimensional stability, installation / removal means, physical characteristics of the USTD such as smooth / contoured surfaces and/or regions, etc. as well as other aspects such as any locking and/or release mechanisms.

The accumulated data from the Analysis & Modelling 120 as defined within Assessment 122 and Performance Goals 124 is coupled to an Artificial Intelligence (AI) Engine 160 which employs a plurality of algorithms which may exploit one or more approaches including, but not limited to, those based on symbol manipulation, cognitive simulation, logic-based programming, anti-logic programming, natural language processing, knowledge based, sub-symbolic, embodied intelligence, computational intelligence and soft computing, and statistical either individually or in combination such as within methodologies such as the intelligent agent, multiple interacting agents in a multi-agent system, and a hybrid intelligent system.

The AI Engine 160 may employ a hierarchal control system to bridge between sub-symbolic AI and symbolic AI. Tools exploited by the AI Engine 160 may include, but are not limited to, search and optimization, evolutionary computation, swarm intelligence algorithms, evolutionary algorithms, logic programming, fuzzy systems, subjective logic, default logics, non-monotonic logics, circumscription, probabilistic methods for uncertain reasoning, Bayesian networks, Hidden Markov models, utility theory, decision theory, Kalman filters, dynamic decision networks, classifiers and statistical learning methods, classifiers, neural networks, kernel methods, k-nearest neighbour algorithm, naive Bayes classifier, decision tree, neural networks, artificial neural networks, acyclic or feedforward neural networks, recurrent neural networks, perceptrons, multi-layer perceptrons, radial basis networks, backpropagation networks, deep feedforward neural networks, convolutional neural networks, reinforcement learning, deep recurrent neural networks, recurrent neural networks, and gradient descent training.

The output of the AI Engine 160 is coupled to Custom Device Manufacturing and Fitting (CUDEMAF) 130 which proceeds with a sequence comprising Manufacture 132 and Fitting 134. Within Manufacture 132 the custom USTD is defined in respect of the materials providing its physical geometry with the desired mechanical properties as well as external characteristics. Accordingly, the custom USTD may be defined by one or more aspects including, but not limited to:

- Scaffold structure by dimension(s), material(s) etc.
- Shell structure by dimension(s), material(s) etc.
- Casing structure by dimension(s), property or properties, material(s).
- Passive - active integration such as is USTD passive or does it embed sensor(s), control and/or data logging circuitry, wireless interface(s) etc.
- Lock-release structure.
- Coatings.

Accordingly, a CAD model is established from which the Manufacture 132 process is undertaken. Within an embodiment of the invention an initial CAD model may be established by combining three-dimensional (3D) modelling with computational fluid dynamics (CFD), finite element analysis (FEA), and/or multi-organ free-body diagram models. The CAD model may be simplified to reduce the required computational power and complexity of the processing applied prior to the AI Engine 160 executes. The AI Engine 160 may process based upon this initial pre-processing solely or may apply the pre-processing to a more complete human body (anatomical) model and USTD model in order to define the USTD design, CAD, and materials requirements. Optionally, the pre-processing may be bypassed where appropriate levels of computing resources are available. Within an embodiment of the invention the AI Engine 160 generates the specifications for design of the USTD in dependence upon the computational modelling, FEA analysis, 3D modelling either individually or in combination.

Accordingly, a USTD as designed and manufactured may range from a passive USTD through to an active USTD, with lock-release structure, anti-microbial coating, and wireless interface for transmitting and logging data relating to the user. An active USTD may incorporate one or more sensors or stimulation mechanisms, such as electrostimulation, for example.

Within Fitting 134 the custom USTD is provided to the user and either fitted by themselves, e.g. for temporary use USTD that the user will insert/remove as desired, or by a clinician, e.g. semi-permanent or permanent use. At this point one or more assessments may be carried out such as outlined previously in respect of Structural 112 and/or Force, Strain and Distension 114 whereby, for example, mechanical, imaging, static and/or dynamic assessment etc. are performed to assess the USTD fit against the target design / user physiology etc. Optionally, the Structural 112 and/or Force, Strain and Distension 114 may be device based assessments and/or non-device based (e.g. clinical) assessments. This stage may also include device-acquired user monitoring, e.g. via internal sensors to the USTD, as well as user-reported monitoring, e.g. by personally noting performance of the USTD etc. Based upon these results a determination is made as to whether the USTD meets the initial requirements wherein if yes, the process proceeds to step 140. If not, then the process proceeds to loop back to either A&M 120 or CUDEMAF 130 according to the nature and/or complexity of the modifications / amendments required.

In step 140 the user employs the USTD on an ongoing basis wherein device-acquired monitoring, e.g. via internal sensors to the USTD, as well as user-reported monitoring, e.g. by personally noting performance of the USTD etc. are performed wherein periodically this data is employed in determining whether the objectives for the USTD were met in step 150. If yes, then the process loops back to step 140 otherwise it proceeds back to step 110. For example, a young user may require multiple USTDs within the space of a few years / decade during their childhood, puberty, adolescence, etc. with evolving dimensions and requirements whereas an elderly user may require a single adjustment or no adjustment according to their circumstances.

Within the description *supra* monitoring of the user has been described and discussed with respect to the fitting, assessment, and performance monitoring of a USTD or USTD according to an embodiment of the invention. Whilst this may exploit one or more sensors embedded within the body of the USTD or upon its surface as discussed below it would be evident that the assessment may employ and exploit data acquired from a range of other wearable devices and biometric sensors in order to enhance, for example, the assessment, fitting, and monitoring of USTDs and/or USTDs according to embodiments of the invention wherein the additional data obtained, e.g. biometric data, environmental data, activity data, body position data, etc., provides correlation data and/or additional data For example, a patient suffering UI may experience incontinence when bending over and/or walking but not during sitting and/or being prone. Further, the ongoing acquisition of data from a range of other wearable devices and biometric sensors may also be employed in association with or without sensors within the USTD to provide ongoing quality of life (QoL) data to assess the effectiveness of the USTD.

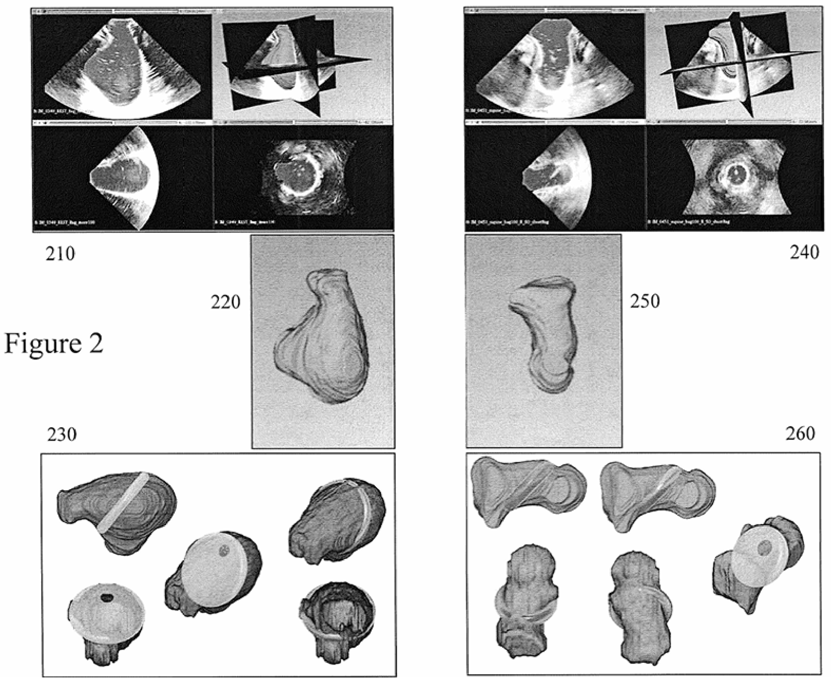

Figure 2 depicts schematically the measurements, modelling, and fitting of a USTD according to embodiments of the invention for two different patients. Referring to first to third images 210 to 230 respectively then for a first patient, Patient A, there are depicted:

- First image 210 comprising ultrasound images acquired on the first user's anatomy;
- Second image 220 depicted a 3D model based upon data extraction from the ultrasound image to generate the first user's vaginal anatomy; and
- Third image 230 wherein a 3D CAD model of a USTD design in dependence upon the user's physiology has been overlaid with the first user's vaginal anatomy to show the resulting fit of the USTD for the first patient.

Now referring to fourth to sixth images 240 to 260 respectively then, for a second patient, Patient B, there are similarly depicted:

- Fourth image 240 comprising ultrasound images acquired on the second user's anatomy;
- Fifth image 250 depicted a 3D model based upon data extraction from the ultrasound image to generate the second user's vaginal anatomy; and
- Sixth image 260 wherein a 3D CAD model of a USTD design in dependence upon the user's physiology has been overlaid with the second user's vaginal anatomy to show the resulting fit of the USTD for the second patient.

Table 1 presents some characterizations of the 3D CAD model where the ultrasound images in first and fourth images 210 and 240 respectively were obtained in the supine position at 100% vaginal capacity.

Table 1: 3D CAD Model Outputs for Patients A and B

| Patient | Volume (mL) | Surface Area (cm$^2$) |
|---------|-------------|------------------------|
| A | 436.7 | 338.8 |
| B | 139.2 | 167.3 |

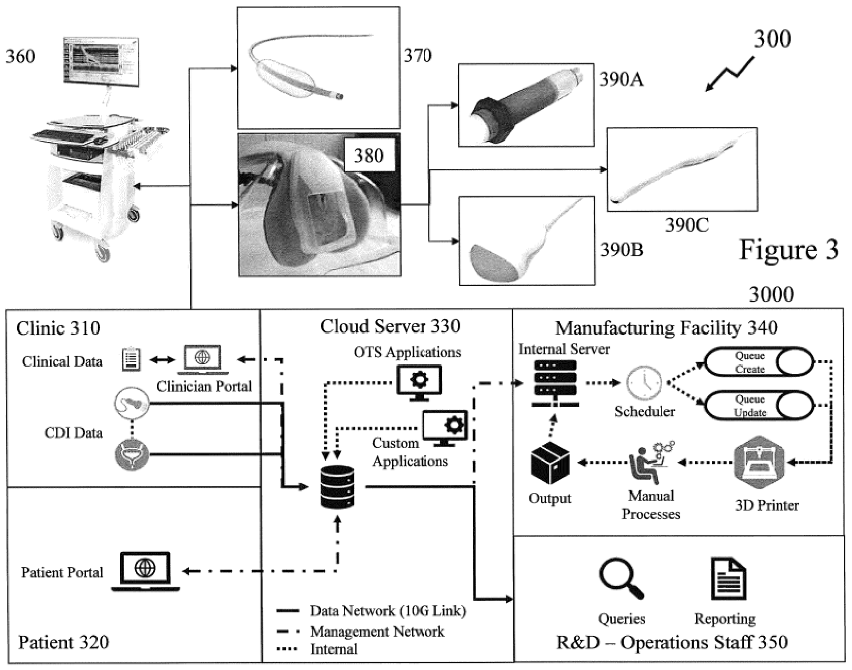

Now referring to Figure 3 there is depicted an exemplary USTD Platform and System 300 supporting embodiments of the invention comprising an initial measurement stage involving Clinic 310 and Patient 320. The data from the measurement stage is analysed using Cloud Server 330 resulting in outputs to Manufacturing Facility 340 and R&D - Operations Staff 350. Considering Clinic 310 then this is depicted as comprising clinical data which is obtained via a Clinician Portal and stored upon the remote cloud server and clinical data integration (CDI) data which is automatically transferred from the measurement system(s) to the remote Cloud Server 330. This CDI data may include, but not be limited to colpodynamic imaging data. The clinical data may comprise clinician measurements, observations, assessments etc. of the patient such as described and depicted above with Manual measurements in Structural 112 of Figure 1 for example together with data for Quality of Life 116 in Figure 1. The CDI data, in contrast, is automatically acquired data relating to the user through one or more measurement systems such as Manometry System 360 together with Manometer Balloon 370 and an embodiment of the invention, a Manometry Cap 380, which is employed with a two-dimensional (2D) ultrasound Probe 390A or a three-dimensional ultrasound Probe 390B within embodiments of the invention. This CDI data being such as described and depicted in Figure 2 with first and fourth images 210 and 240 respectively is automatically pushed to the remote Cloud Server 330.

Within Patient 320 the user through a Patient Portal enters data relating, for example, to Quality of Life 116 in Figure 1. Subsequent to the user being fitted with the USTD then the user through the Patient Portal and the clinician through the Clinician Portal may each enter additional comments, observations, notes etc. with respect to the monitoring of the quality of life /performance of the USTD such as within step 140 of Figure 1.

The data once stored upon the remote Cloud Server 330 from the Clinic 310 and Patient 320 is employed by a combination of Off-the-Shelf (OTS) applications and custom applications to generate the data required for the Manufacturing Facility 340 as well as any communications required to the R&D - Operations Staff 350. The remote Cloud Server 330 based upon the processing by the OTS applications and custom applications may also send communications to the Clinic 310 and the Patient 320. Data provided to R&D - Operations Staff 350 may include data for reporting and/or queries. The data provided to the Manufacturing Facility 340 from the remote Cloud Server 330 would be stored upon an internal server of the Manufacturing Facility 340 and would comprise USTD tracking data to associate the USTD to the Patient 320, manufacturing data for the USTD specific to the Patient 320, etc. This is then employed to schedule the manufacturing and control the manufacturing process using additive and/or non-additive manufacturing processes. The Cloud Server 330 may include an AI Engine, such as AI Engine 160 in Figure 1.

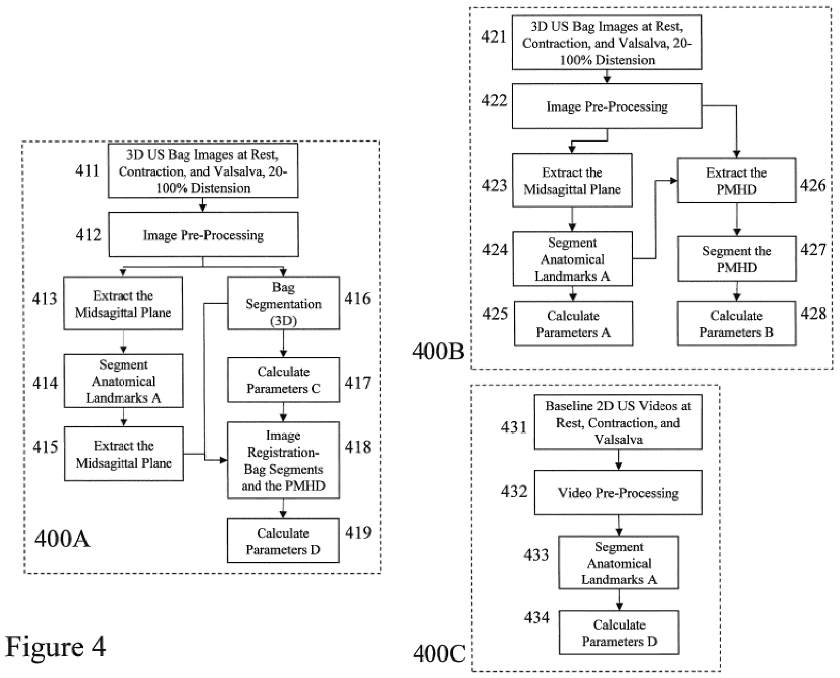

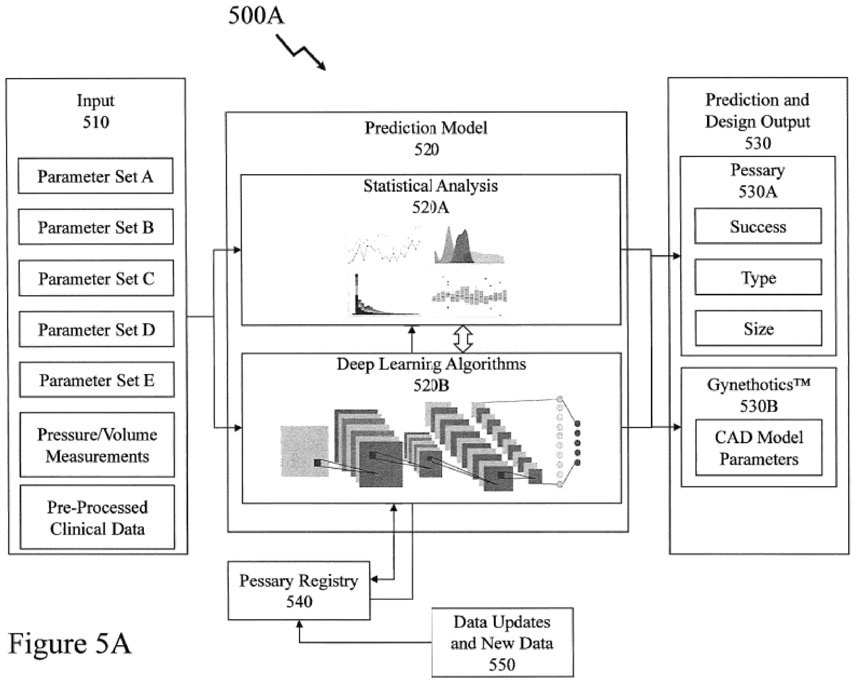

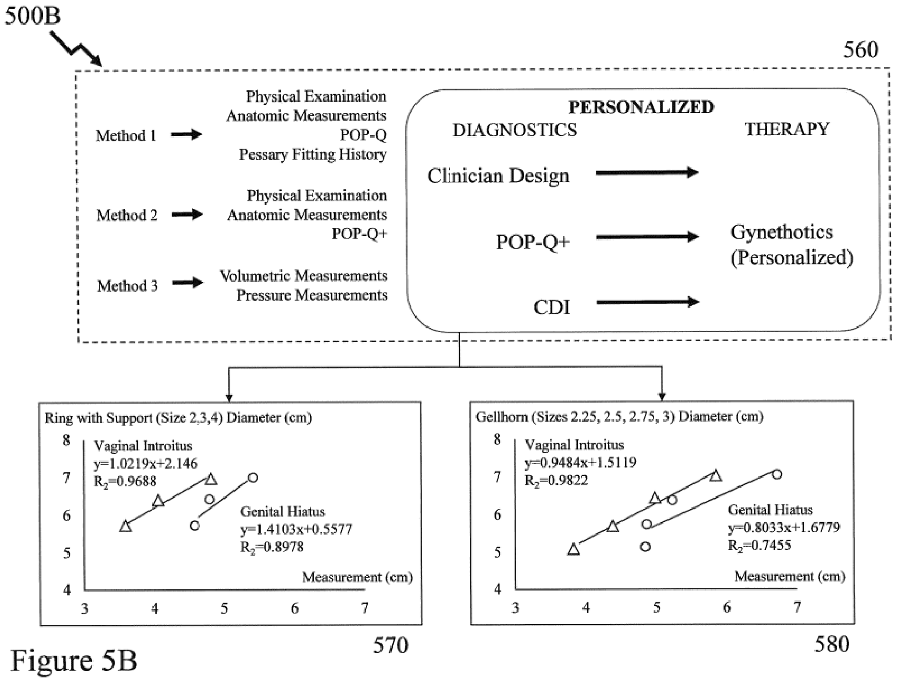

After QA processes etc. the Clinic 310 and Patient 320 are advised the USTD has been manufactured and the USTD shipped to the Clinic 310 or the Patient 320. Accordingly, measurements from the Manometry System 360 together with Manometer Balloon 370 and an embodiment of the invention, a Manometry Cap 380, which is employed with an instrument such as two-dimensional (2D) ultrasound Probe 390A,a three-dimensional ultrasound Probe 390B, or endoscopic Probe 390C for example, are employed by the Cloud Server 330 to generate the USTD specific manufacturing data including dimensions, design, material(s), etc. employed by the Manufacturing Facility 340. This conversion from patient centric data to USTD design data are described and depicted in more detail in Figures 4 and 5 according to embodiments of the invention.

Accordingly, the USTD Platform and System 300 provides for collection of data from several sources including, but not limited to:

- a clinician application or clinician portal (which may integrate with a clinic's Electronic Medical Records (EMR) software suite or cloud services, Electronic Health Records (EHR) software suite or cloud services, etc.;
- a patient application or portal; and
- a provider of USTDs (e.g. COSM Medical) clinical data integration (CDI) software application or cloud services.

Accordingly, the USTD Platform and System 300 stores data within one or more cloud services wherein this stored data is exploited to provide analysis and prediction software methods and applications including, but not limited to:

- Anatomy identification
- 3D segmentation and key metric extraction;
- 2D video processing for dynamic analysis and/or analysis of pressure, force, direction and/or magnitude; and
- USTD device geometry and dimensions using one or more prediction models.

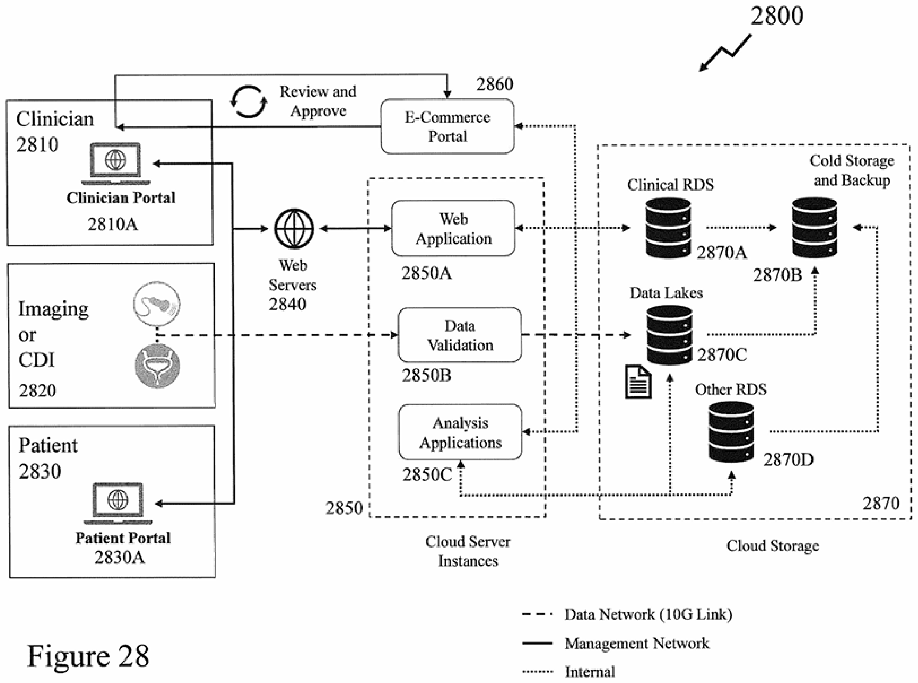

Accordingly, several software applications and/or portals as depicted within USTD Platform and System 300 transfer data to / from the Cloud Server 330. Accordingly, referring to Figure 28 depicts there is depicted Cloud Infrastructure 2800 according to an embodiment of the invention supporting an USTD Platform and System 300 other USTD platforms and systems according to embodiments of the invention. Accordingly, there is depicted a Clinician 2810 accessing a Clinician Portal 2810A which provides data to and/or receives data from Web Servers 2840. These Web Servers 2840 being in communication with Web Application 2850A which interfaces with Clinical Remote Desktop Services (RDS) Server 2870A. Similarly, a Patient 2830 accesses a Patient Portal 2830A which similarly communicates with the Web Servers 2840.

Also depicted is Imaging or Clinical Data Integration (CDI) 2820 wherein acquired imaging and/or clinical data is communicated to another Cloud Server Instance 2850, Data Validation 2850B, wherein the acquired data is processed to ensure it is valid before it is stored within one or more Data Lakes 2870C. As depicted the Imaging or CDI 2820 communicates directly with Data Validation 2850B via secure links, data tunnels, etc. as known in the art. Optionally, this data may also be pushed via one or more Web Servers 2840. The data stored within Clinical RDS 2870A and Data Lakes 2870C is archived / backed up within Cold Storage and Backup 2870B. The data from Clinical RDS 2870A and Data Lakes 2870B is accessed by Analysis Applications 2850C representing yet further Cloud Server Instances 2850.

The Analysis Applications 2850C may access Other RDS 2870D storing data including, but not limited to, analysis software, design databases, material databases, quality of life databases etc. The Analysis Applications 2850C or a specific application within the Analysis Applications 2850C also communicate with an E-Commerce Portal 2860 allowing a Clinician 2810 to initiate an analysis, design, and manufacturing process through an e-commerce process. During this e-commerce process the Clinician 2820 may be provided with data prior to their review and approval of the manufacturing process. This data may include, but not be limited to, the USTD device geometry and dimensions, patient data, patient quality of life data, CDI data and predictions of USTD quality of life. Similarly, the Other RDS 2870D may be archived / backed up within Cold Storage and Backup 2870B.

Within embodiments of the invention the E-Commerce Portal 2860 provides one or more functions (features) to a clinician / physician as well as to other third parties. For example, the E-Commerce Portal 2860 may provide for:

- A clinical decision confirmation system (CDCS) providing:

  ○ Recommended USTD designs;
  ○ Three-dimensional visualization of patient's physiology and USTD device;
  ○ Ability for clinician to edit and update key features and/or dimensions;
  ○ Clinician approval of the design for manufacturing;

- Transmittal of physician approved USTD device to manufacturing system and/or USTD device provider;
- Payment processing including, for example, billing clinic, patient, and insurance;
- Shipment tracking
- Notifications;
- USTD device fitting appointment scheduling; and
- A telemedicine platform for post USTD device fitting care pathway.

Referring to Figure 4 there are depicted first to third measurement and image analysis (MIA) process Flows 400A to 400C respectively employed within a USTD platform according to an embodiment of the invention and the resulting parameters established with respect to a patient's anatomy and physiology. First MIA process Flow 400A relates to measurements made using a Vaginal Manometry (VAMA) system and an ultrasound where the VAMA allows the distension of the vagina to be established as well as obtaining the measurements of volume / pressure etc. employed within Input 510 in Figure 5A together with the ultrasound derived Parameters A-E. As depicted first MIA process Flow 400A comprises first to ninth steps 411 to 419 respectively. Initially, the process executes first and second steps 411 and 412. Subsequently a first branch comprising third to fifth steps 413 to 415 is executed in parallel to a second branch comprising sixth and seventh steps 416 and 417. The outputs from the first and second branches are then employed in third branch comprising eighth and ninth steps 418 and 419 respectively. Accordingly, first and second steps 411 and 412 comprising:

- First step 411 wherein a VAMA system is employed to inflate a manometer bag (or manometer balloon) under predetermined conditions, e.g. patient at rest, patient contracting (e.g. their pelvic floor muscles), and performing a Valsalva maneuver (hereinafter simply referred to as Valsalva), and over a range of distensions (e.g. 20%

  - 100% at 20% steps) wherein three dimensional (3D) ultrasound images of the manometer bag are obtained under these different conditions; and

- Second step 412 wherein the images are subjected to pre-processing using one or more image processing algorithms.

Optionally, first step 411 may be replaced with translabial ultrasound. Optionally, first step 411 may comprise an initial determination of vaginal capacity to define distension(s). Within the first branch third to fifth steps 413 to 415 are executed, these comprising

- Third step 413 wherein the 3D ultrasound images of the lower distension volumens, e.g. those below a threshold distension, are employed to extract a preliminary mid-sagittal plane of the measurements of the patient;
- Fourth step 414 wherein the images are processed to extract and segment a first set of anatomical landmarks (Landmarks A); and
- Fifth step 415 wherein the Plane of Minimal Hiatal Dimensions (PMHD) Perceptually Modified Hausdorff Distance (PMHD) is established and this result provided to the third branch in eighth step 418.

Within the second branch sixth and seventh steps 416 and 417 are executed, these comprising

- Sixth step 416 wherein the 3D ultrasound images of either the higher distension volumes above the distension threshold or all volumes are employed to perform bag segmentation in 3D where these results are provided to the second branch in eighth step 418; and

- Seventh step 417 wherein a first set of parameters, Parameters C, are calculated from the 3D bag segmentation.

Within the second branch the outputs from the first and second branches are employed in eighth and ninth steps 418 and 419 respectively, these comprising:

- Eighth step 418 wherein the outputs of the first and second branches are employed to register images, the bag segments and extract the PMHD; and
- Ninth step 419 wherein another set of parameters, Parameters D, are calculated.

Referring to second MIA process Flow 400B this relates to measurements made using a Vaginal Manometry (VAMA) system. As depicted second MIA process Flow 400B comprises first to eighth steps 421 to 429 respectively. Initially, the process executes first and second steps 421 and 422. Subsequently a first branch comprising third to fifth steps 423 to 425 is executed in parallel to a second branch comprising sixth and eighth steps 426 and 428 respectively. Accordingly, first and second steps 421 and 422 comprising:

- First step 411 wherein a VAMA system is employed to inflate a manometer bag (or manometer balloon) under predetermined conditions, e.g. patient at rest, patient contracting (e.g. their pelvic floor muscles), and performing a Valsalva maneuver (hereinafter simply referred to as Valsalva), and over a range of distensions wherein three dimensional (3D) ultrasound images of the manometer bag are obtained under these different conditions; and
- Second step 412 wherein the images are subjected to pre-processing using one or more image processing algorithms.

Within the first branch third to fifth steps 423 to 425 are executed, these comprising:

- Third step 423 wherein the 3D ultrasound images are employed to extract a mid-sagittal plane of the measurements of the patient;
- Fourth step 424 wherein the images are processed to extract and segment first and second sets of anatomical landmarks (Landmarks A and Landmarks B); and
- Fifth step 425 wherein based upon the extracted Landmarks A and Landmarks B a further set of parameters, Parameters A, are calculated.

Within the second branch sixth and seventh steps 416 and 417 are executed, these comprising

- Sixth step 416 wherein the outputs of the first and second branches are employed to register images, the bag segments and extract the PMHD;
- Seventh step 417 wherein the extracted PMHD is segmented; and
- Eighth step 418 wherein based upon the extracted PMHD segments a further set of parameters, Parameters B, are calculated.

Referring to third MIA process Flow 400C this relates to measurements made using a Vaginal Manometry (VAMA) system but now rather than static 3D ultrasound images a 2D video ultrasound is acquired and processed. These 2D video ultrasound images may then be processed to generate 3D ultrasound images. As depicted third MIA process Flow 400C comprises first to fourth steps 431 to 434 respectively, these comprising.

- First step 431 wherein base line two dimensional (2D) ultrasound videos are obtained under predetermined conditions, e.g. patient at rest, patient contracting (e.g. their pelvic floor muscles), and performing a Valsalva maneuver (hereinafter simply referred to as Valsalva);
- Second step 432 wherein pre-processing is applied to the acquired video segments;
- Third step 433 wherein the images are processed to extract and segment first and second sets of anatomical landmarks (Landmarks A and Landmarks B); and
- Fourth step 434 wherein based upon the extracted Landmarks A and Landmarks B a set of parameters, Parameters E, are calculated.

First MIA process Flow 400A and/or second MIA process Flow 400B may exploit 3D transverse plane ultrasound (TPUS).

Now referring Figure 5A there is depicted an exemplary process Flow 500A within a USTD platform according to an embodiment of the invention using the parameters established with respect to a patient's anatomy and physiology in Figure 4 to establish the design of a USTD for the patient according to an embodiment of the invention. Accordingly, as depicted Flow 500A takes Input 510 and applies this with Prediction Model 520 to generate Prediction and Design Output 530. As depicted the Input 510 comprises, for example:

- Parameter Set A;
- Parameter Set B;
- Parameter Set C;
- Parameter Set D;
- Parameter Set E;
- Pressure / Volume measurements from VAMA system; and
- Pre-processed clinical data, such as provided by the clinician in Clinic 310 in Figure 3.

The Prediction Model 520 exploits parallel threads of Statistical Analysis 520A and Deep Learning Algorithms 520B using Input 510 and Pessary Registry 540. The resulting outputs from the parallel threads in Prediction Model 520 are coupled to Pessary 530A and USTD 530B in the Prediction and Design Output 530. Pessary 530A comprises data relating to the success of the modelling, type of pessary selected, and size of pessary selected. This data, may for example, be the data transmitted back from the Cloud Server 330 to the Patient 320 and/or Clinic 310. USTD 530B comprises the CAD modelling parameters which, may for example, be passed from the Cloud Server 330 to the Manufacturing Facility 340 directly or be processed further prior to being passed to the Manufacturing Facility 340 in Figure 3. The Prediction Model 520 may include an AI Engine, such as AI Engine 160 in Figure 1. Additional updates to the registry data and new data are fed from one or more External Processes 550, such as the ongoing monitoring of quality of life (QoL) and performance of the USTD in step 140 of Figure 1 for example. Further, as depicted, the Statistical Analysis 520A and Deep Learning Algorithms 520B may exchange data with one another.

The Pessary Registry 540 comprises data obtained through a process such as described with respect to exemplary Flow 500B in Figure 5B according to an embodiment of the invention. The Pessary Registry 540 is a list that includes a large number of patients. The list includes various clinical evaluations (including evaluations such as those in Clinic 310) of each patient along with the outcome of each pessary the patient has used. This Pessary Registry 540 is continuously maintained and used to fuel the Deep Learning Algorithms 520B such as the one or more External Processes 550 so that the Pessary Registry 540 may, within embodiments of the invention, store data relating to current and future patient data of one or more suppliers of devices, systems, applications that includes, but is not limited to, anatomical parameters paired with pessary geometry. As depicted in Flow 500B the Pessary Registry, may for example, be established in dependence upon continuous updating of patient data, including but not limited to, Pelvic Organ Prolapse Quantification (POPQ), extended POPQ (POPQ + measurements of vaginal caliber or introital dimensions), questionnaires, patient diaries, demographic data, clinical history, etc. for example together with the pessaries designed and manufactured for these patients. Accordingly, data relating to clinical assessments (with or without ultrasound data), the selected pessary types, and selected sizes is stored within a database together with patient acquired data relating to the experiences of the patients. Accordingly, the Pessary Register provides the Statistical Analysis 520A and Deep Learning Algorithms 520B in Figure 5A with training data sets, validation data sets, statistics, etc. Accordingly, clinician measurements may be correlated against ultrasound generated measurements, ultrasound measurements correlated to pessary size assigned by clinicians, etc. For example, first graph 570 in Figure 5B presents two correlations of measurements of vaginal introitus and genital hiatus against pessary diameter for a ring pessary with support at sizes 2-4 from the manufacturer. Second graph 580 in Figure 5B presents two correlations of measurements of vaginal introitus and genital hiatus against pessary diameter for a Gellhorn pessary at sizes 2.25, 2.5, 2.75 and 3 respectively from the manufacturer. Accordingly, the Pessary Register can be populated with data for the current standard of care as well as those under the new standard USTD platform according to embodiments of the invention.

Accordingly, Flow 500A in Figure 5A and Flow 500B in Figure 5B exploiting embodiments of the invention including, but not limited to, Cloud Infrastructure 2800 in Figure 28 and USTD Platform and System 300 in Figure 3 allow for the display of data and/or providing supportive information to a clinician. Within embodiments of the invention the analysis may define the size and shape of the USTD device either from a standard library or by establishing a patient-specific USTD device. The USTD Platform and System 300 and/or Cloud Infrastructure 2800 are depicted as being accessed through one of three different pathways although it would be evident that other pathways may be employed without departing from the scope of the invention.

*Pathway 1: Clinician Design.* In this pathway the USTD device may be designed based upon physical examination by the clinician, such as taking anatomical measurements, using a Pelvic Organs Prolapse Quantification (POP-Q) and the patient's pessary history. Accordingly, the USTD Platform and System 300 and/or Cloud Infrastructure 2800 result in the clinician being presenting with a list of available USTD devices including options for dimensional customization.

*Pathway 2: POP-Q+.* In this pathway a physician enters anatomical measurements which results in the USTD Platform and System 300 and/or Cloud Infrastructure 2800 recommending patient specific USTD device design(s) for the physician's review and approval using a clinical decision confirmation system (CDCS), for example. In this pathway, a clinician will review, edit, and approve the USTD device prior to providing the device to the patient.

*Pathway 3: Clinical Data Integration (CDI).* Within this pathway a physician / clinician employs, for example, colpodynamic imaging, to obtain ultrasound images of patient's pelvic floor, as well as manometry data of the vaginal cavity for example. The USTD Platform and System 300 and/or Cloud Infrastructure 2800 recommending patient specific USTD device design(s) for the physician's review and approval using clinical decision support software for example. In this pathway, a clinician will review, edit, and approve the USTD device prior to providing the device to the patient.

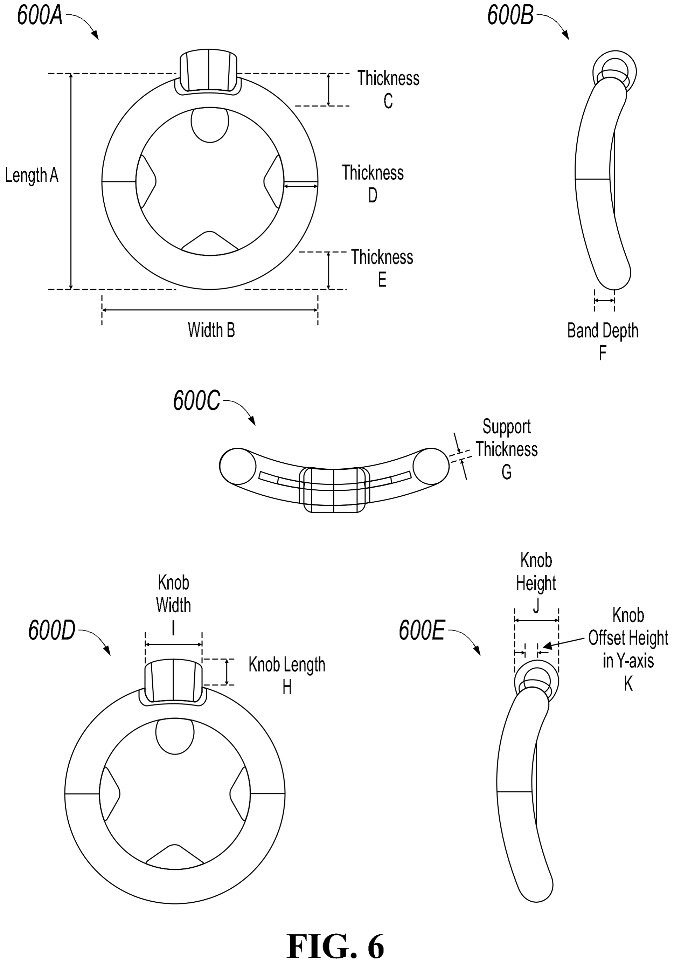

Now referring to Figure 6 there is depicted an exemplary USTD ring pessary in first to fifth images 600A to 600E respectively together with parameters established within the exemplary process flow of Figure 5A to establish the USTD for the patient. Accordingly, referring to first image 600A the dimensions identified are:

- Length A, being the linear dimension of the ring along the axis through the bead (also referred to as knob);
- Width B, being the linear dimension of the ring perpendicular to the axis through the bead (i.e. the pessary may be elliptical as well as circular);
- Thickness C, being the thickness of the pessary ring at the bead;
- Thickness D being the thickness of the pessary ring along the axis of width B; and
- Thickness E, being the thickness of the pessary ring distal to the bead.

Referring to second image 600B the dimension identified is the Bead Depth F which defines the distance from the centre of the bead to the centre of the ring at its maximum depth so that the ring may vary from flat, i.e. F=0, be concave, i.e. F>0 along the Y-axis, or convex, i.e. F<0 along the Y-axis. Third image 600C defines the thickness of the support, this being the portion of the pessary within the periphery of the ring which is a membrane of thickness G.

Fourth image 600D defines:

- Knob length H, being the radial distance the knob projects from the ring; and
- Knob width I, being the width of the knob along the axis of the ring having width B.

Fifth image 600E defines:

- Knob length J, being the thickness of the knob; and
- Knob offset K, being the difference between the central axis of knob and the central axis of the ring where it joins the knob such that as viewed with respect to fifth image 600E K>0 along the Y-axis moves the knob to the left relative to the ring and K<0 along the Y-axis moves the knob to the right relative to the ring.

As noted within Figure 3 a VAMA system 360 may be employed in conjunction with a Manometer Balloon 370 and an embodiment of the invention, a Manometry Cap 380, which is employed with a two-dimensional (2D) ultrasound Probe 390A or a three-dimensional ultrasound Probe 390B within embodiments of the invention. Within Figure 4 the first Process Flow 400A employs ultrasound measurements at different distensions of the vagina. The VAMA system 360 allows these distensions to be established as well as obtaining the measurements of volume / pressure etc. employed within Input 510 in Figure 5A together with the ultrasound derived Parameters A-E. The Manometer Balloon 360, Manometry Cap 380 and 2D probe 390A and/or 3D probe 390B allow the measurements required by first to third MIA process Flows 400A to 400C respectively with improvement ergonomics, usability, patient care etc.

The Manometer Balloon 360, Manometry Cap 380 and 2D probe 390A and/or 3D probe 390B allow the measurements required for diagnosis of Pelvic Organ Prolapse, dimensional determination of the vagina at various stages of distention, monitoring pelvic muscle strength and tissue elasticity, or for surgical planning to be obtained. Within the description of embodiments of the invention 2D ultrasound and 3D ultrasound are described with respect to imaging the vagina at different distensions, under different user conditions (e.g. seated, supine, dynamic, static etc.), etc. to be obtained. However, it would be evident that other imaging methods may be employed without departing from the scope of the inventions described and depicted. Further, the descriptions presented are with respect to vaginal measurements, but it would be evident that other embodiments of the invention may exploit rectal, urethral, or other measurements.

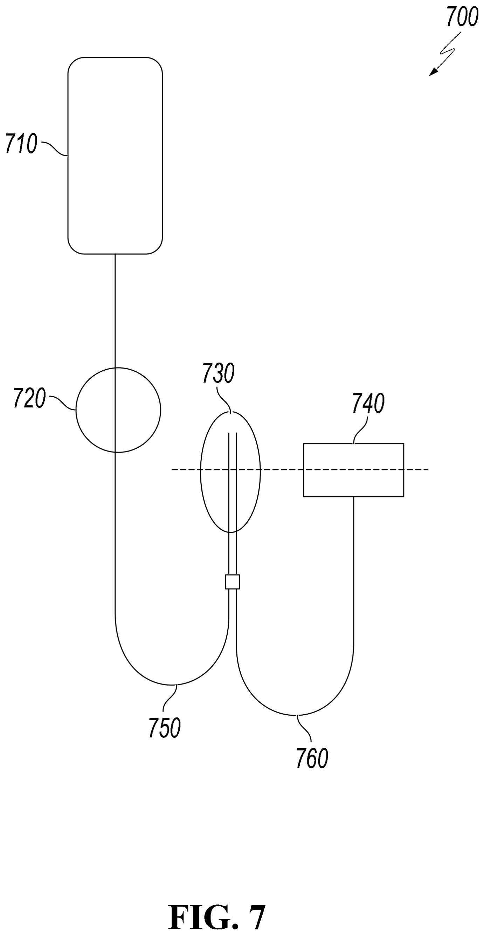

Referring to Figure 7 there is depicted a simplified schematic of a vaginal manometry (VAMA) System 700, such as VAMA system 360 in Figure 3 employed in establishing patient physiology, measurements etc. Accordingly, a Fluid Source 710 of a fluid, e.g. water, is coupled via a Pump 720 to the inside of a Manometer Balloon 730 via a first tube 750. A second tube 760 is coupled from the inside of the Manometer Balloon 730 to a Pressure Transducer 740. Accordingly, via operation of the Pump 720 with the Manometer Balloon 730 retained, through the use of a Manometry Cap, within the vagina the pressure within the Manometer Balloon 730 can be monitored as a function of volume of fluid within the Manometer Balloon 730. Accordingly, the pressure / volume relationship provided to the modelling algorithms such as described and depicted in respect to Figure 5A.

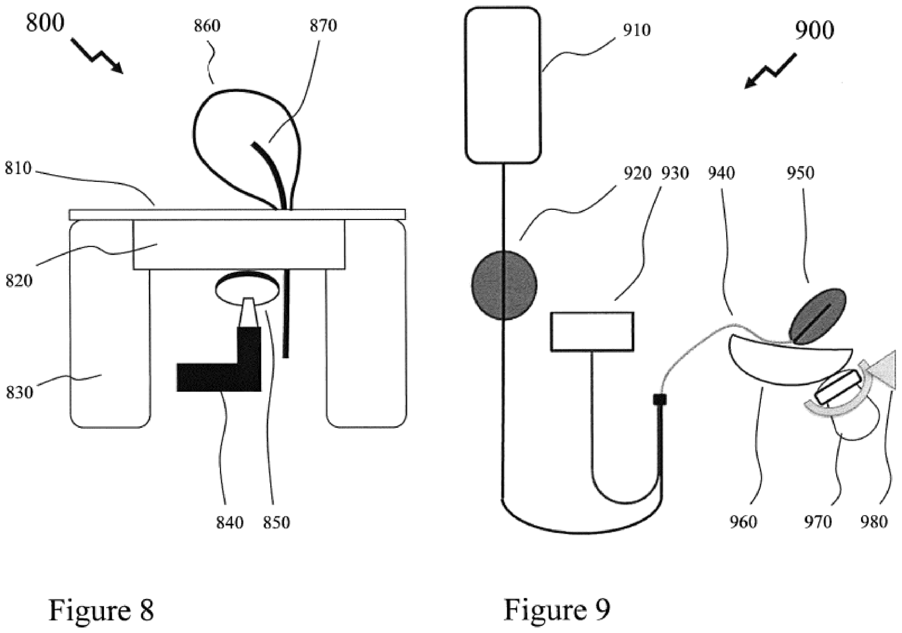

Now referring to Figure 8 there is depicted an exemplary Configuration 800 according to an embodiment of the invention for patient anatomical characterisation combining a VAMA System with ultrasound imaging and robotic manipulation within a seat. As depicted a Tube 870 extends from the inside of a Manometer Balloon (hereinafter Balloon) 860 to a manometry system, not depicted for clarity, through a Seat 820 and Seat Cover 810. Accordingly, with the user sitting on the Seat 820 and the Seat Cover 810 with the Balloon 860 inserted into the vagina, measurements can be performed with the VAMA system but also with the Ultrasound Probe 850 which is disposed beneath the Seat 820 and Seat Cover 810. The Seat 820 and Seat Cover 830 being formed from materials transparent to ultrasound signals. The Ultrasound Probe 850 being mounted to a Robotic Arm 840 allowing the Ultrasound Probe 850 to be moved relative to the seated user remotely. The Seat 820 and Seat Cover 810 being supported by Legs 830. Accordingly, the manometry pressure/volume measurements can be obtained in a single session together with the required ultrasound images for first to third MIA Flows 400A to 400C respectively as described and depicted in Figure 4. In this manner once the patient is seated a control system, not depicted for clarity, can interface to the manometry system, robotic arm, and ultrasound probe to automatically execute the processes and data acquisition rendering the process more reproducible and independent of the clinician.

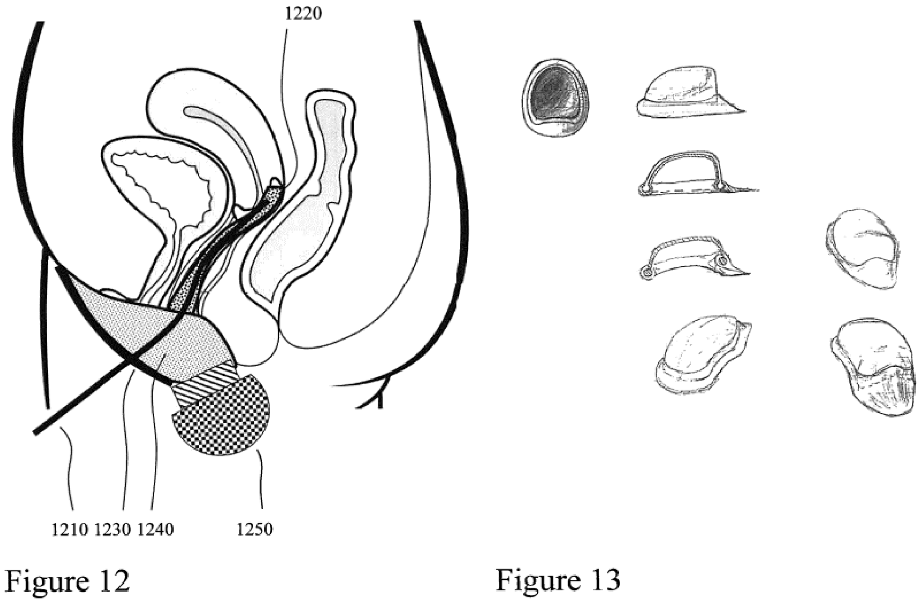

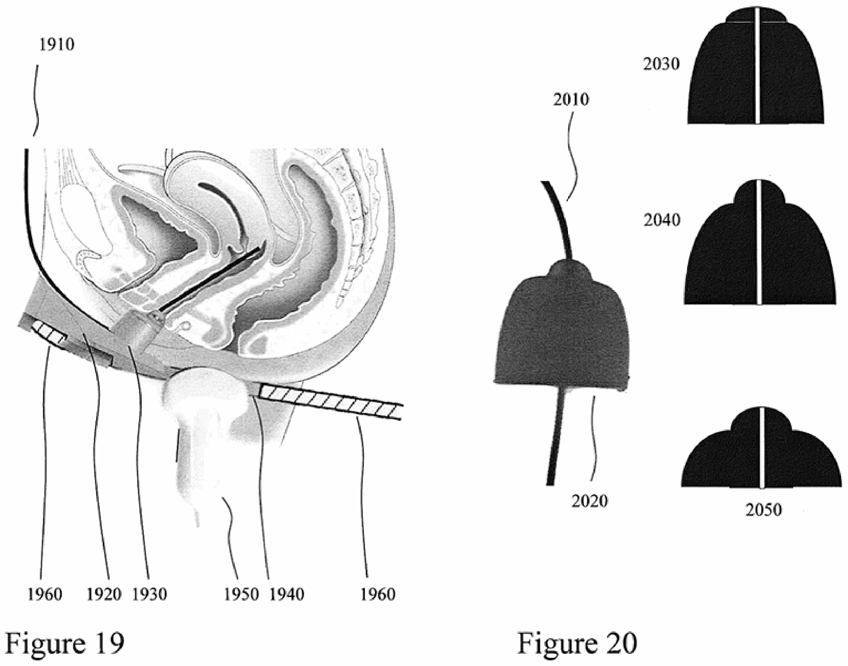

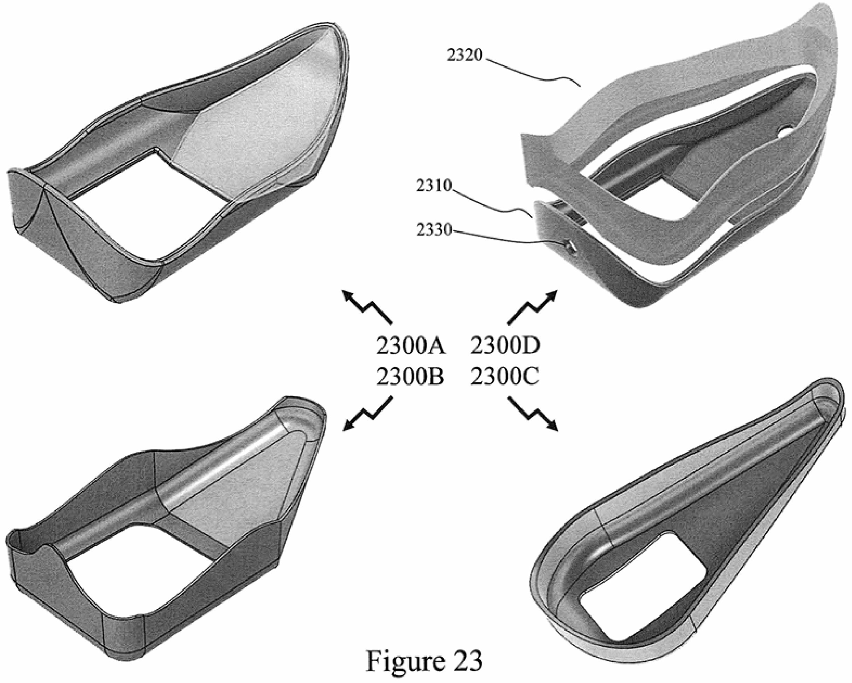

Referring to Figure 9 there is depicted an exemplary Configuration 900 according to an embodiment of the invention for patient physiology characterisation combining a VAMA system with ultrasound imaging and robotic manipulation. As depicted within Configuration 900 a Fluid Source 910 is coupled to a Balloon 950 via a Pump 920 and Tube 940. The Balloon 950 is also coupled to a Pressure Transducer 930 via the Tube 940. Accordingly, the pressure / volume of the Balloon 950 can be established through the volume of fluid pumped by Pump 920 and the pressure monitored by the Pressure Transducer 930. The Balloon 950 is disposed adjacent to a Balloon Retention Component 960 which abuts the lower end of the Balloon 950 against the user's body, not depicted for clarity, to retain the Balloon 950 within the user's body when it is inflated. Also coupled to the Balloon Retention Component 960 is Imaging Probe 970 attached to a Motorized Arm 980. The Imaging Probe 970 may, for example, be a 2D ultrasound probe or a 3D ultrasound probe whilst the Motorized Arm 980 may, for example, be a robotic manipulator. Accordingly, as will become evident from the embodiments of the invention described and depicted below the Balloon Retention Component 960 allows for improved ergonomics for the patient and/or clinician with respect to performing the measurements as well as improved reproducibility, as the Balloon 950 does not move out of the user's body, improved automation etc. The Motorized Arm 980 may be omitted. Optionally, within embodiments of the invention the Balloon Retention Component 960 may be an element such as Plug 2020 as described and depicted in respect of Figures 19 and 20, first to fourth CAD Schematics 2300A to 2300D of manometry cups in Figure 23 or Structural Shield 1230 and Outer Cushion 1240 as described and depicted in respect of Figures 12 and 13.

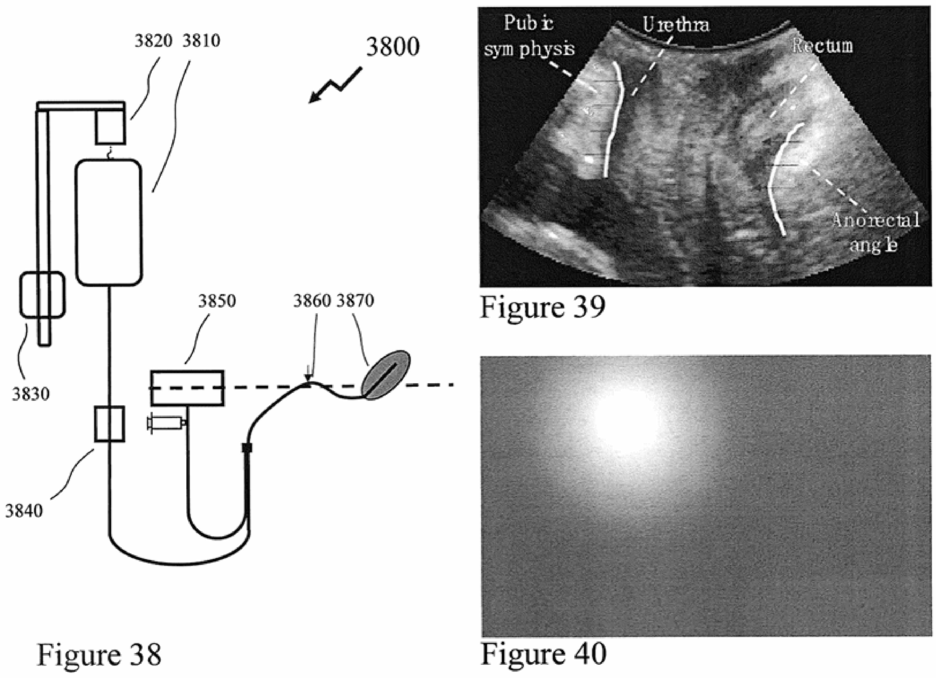

Referring to Figure 38 there is depicted an automated VAMA System 3800 for use with exemplary VAMA systems such as VAMA System 700 in Figure 7 and Configuration 900 in Figure 9. Accordingly, VAMA System 3800 comprises Pressure Transducer 3850, Catheter 3860, and Manometry Balloon 3870 together with Fluid Source 3810. However, the Fluid Source 3810 is now suspended from Sensor 3820 which provides for a weight measurement of the Fluid Source 3810 (e.g. IV bag) and the fluidic tubing between the Fluid Source 3810 and Pressure Transducer 3850 now includes a Control Valve 3840 which is connected to a controller (not depicted for clarity) as are the Pressure Transducer 3850 and Sensor 3820. The Fluid Source 3810 and Sensor 3820 are mounted onto a pole which is coupled to a Motor 3830 which is also connected to the controller. Accordingly, based upon the measurements provided by the Sensor 3820 and Pressure Transducer 3850 the controller can adjust the flow rate through Control Valve 3840 and/or Motor 3830 (by raising or lowering the Fluid Source 3810).

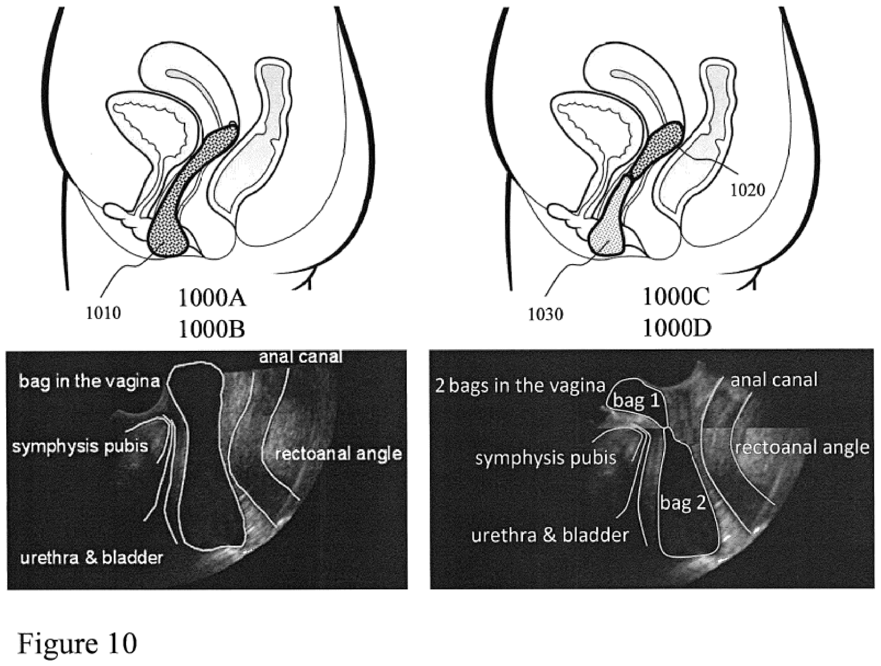

Now referring to Figure 10 there is depicted exemplary manometry bag (or manometry balloon or Balloon) configurations for a VAMA system according to an embodiment of the invention for patient physiology characterisation. Referring to first and second images 1000A and 1000B respectively there are depicted a cross-sectional schematic and ultrasound image respectively of an embodiment of the invention exploiting a single Balloon 1010. In contrast, referring to third and fourth images 1000C and 1000D respectively there are depicted a cross-sectional schematic and ultrasound image respectively of an embodiment of the invention exploiting a pair of manometry bags, Balloon A 1020 and Balloon B 1030. Accordingly, the single Balloon 1010 may employ in conjunction with a single lumen based catheter for coupling the fluid source and the pressure transducer to the Balloon 710. However, in third and fourth images 1000C and 1000D respectively a dual lumen based catheter would be required allowing fluid to be coupled to Balloon A 1020 from a first pump and to Balloon B 1030 from a second pump whilst Balloons A and B 1020 and 1030 are each coupled to different pressure transducers. It would be evident that within other embodiments of the invention the number of balloons, N, may be N=3, 4, etc. where N is a positive integer in conjunction with N pumps and N pressure transducers. Alternatively, a single pump and pressure transducer may be coupled to the N balloons via 1:N fluidic switches. Alternatively, M pumps and P pressure transducers may be coupled to the N balloons via appropriate fluidic switching matrices.

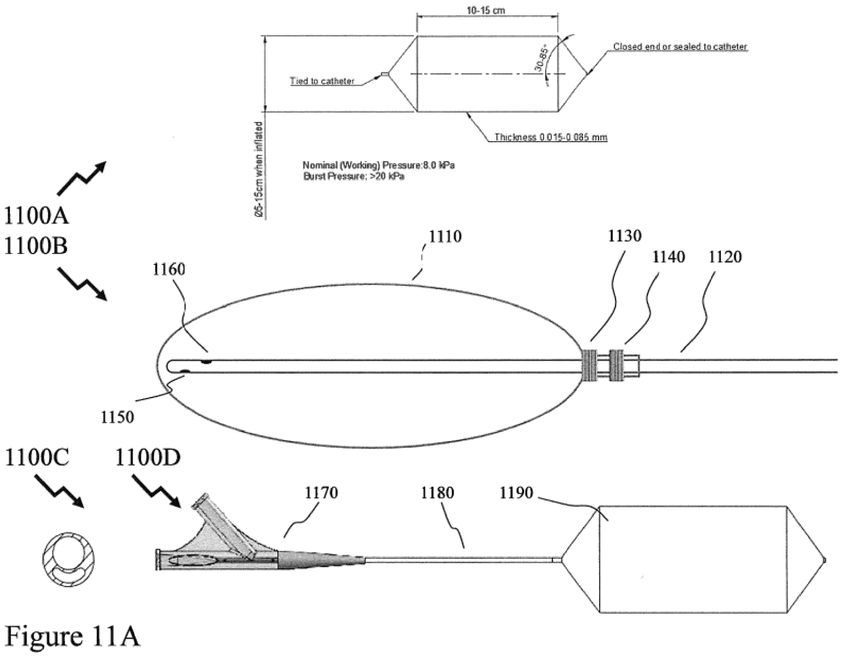

Referring to Figure 11A there are depicted first to fourth images 1100A to 1100D respectively an exemplary manometry balloon and catheter configuration according to an embodiment of the invention for patient physiology characterisation. Referring to first image 1100A the balloon is depicted as being attached to the catheter at a distal end from the tip and either attached at the end of the catheter or being a sealed balloon with a single opening. As depicted the balloon employed may having a length between 10-15cm (approximately 4-6"), a tip angle between 30° and 85°, having a thickness between 0.015mm and 0.085mm (approximately 0.0006" to 0.0033") and an inflated diameter (absent restrictions) of 5-10cm (approximately 2-4"). The nominal working pressure is 8kPa (approximately 1.2 lb/in$^2$) with a burst pressure >20kPa (approximately 3 lb/in$^2$). It would be evident that these dimensions and pressures are exemplary and that other dimensions, pressure ranges etc. may be employed without departing from the scope of the invention.

An alternate balloon is depicted in second image 1100B wherein the Balloon 1110 is attached to a pair of Fluid Seals 1130 and 1140 to the Catheter 1120 and is a single opening design rather than a dual opening balloon requiring attachment to the Catheter 1120 at each end by fluid seals, such as Fluid Seals 1130 and 1140 respectively. Also depicted are the Fluid Port 1150 for filling the Balloon 1110 through a first bore of the Catheter 1120 and the Pressure Port 1160 which is coupled to the external pressure transducer via a second bore of the Catheter 1120. Optionally, within other embodiments of the invention the Pressure Port 1160 and external pressure transducer are replaced by one or more pressure transducers integrated into the Catheter 1120 and coupled to external monitoring circuitry via one or more electrical connections. Third image 1100C is depicting an exemplary dual bore of the Catheter 1120 whilst fourth image 1100D depicts a Catheter 1180 and attached Balloon 1190 (of a configuration as depicted in first image 1100A) coupled to a Lumen 1170 which provides connections to the two bores from the pump and fluid source and to the external pressure transducer respectively. Within embodiments of the invention the catheter may be of an external diameter between 2.0-5.0mm (approximately 0.08-0.20") with an inner fill bore of 1.5mm (approximately 0.06"), for example. It would be evident that these dimensions are exemplary and that other dimensions may be employed without departing from the scope of the invention.

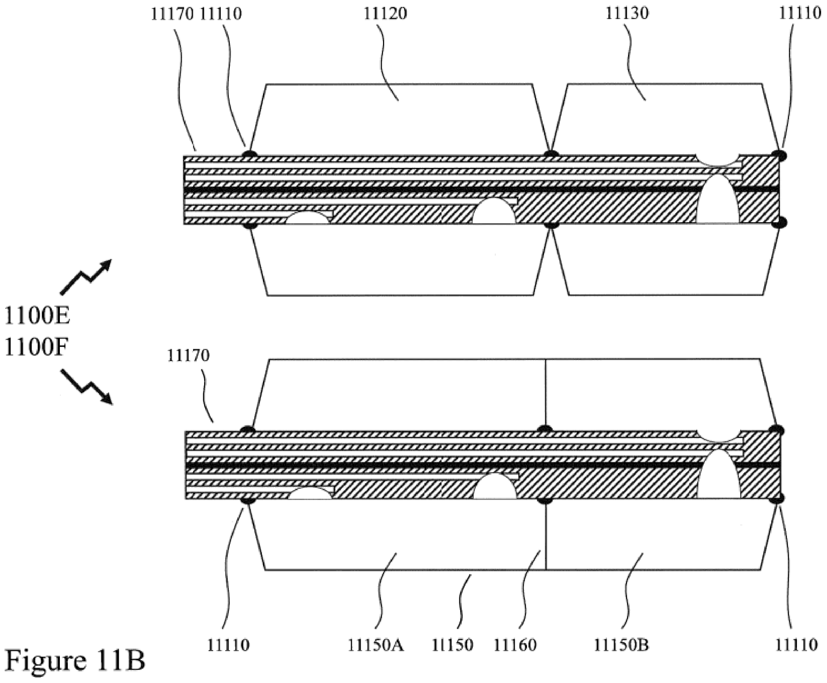

Now referring to Figure 11B there are depicted exemplary first and second Balloon Assemblies 1100E and 1100F with catheter configurations employing dual balloons according to an embodiment of the invention for patient physiology characterisation. Referring to first Balloon Assembly 1100E the catheter now comprises four bores, two for coupling to the fluid source via a pump or pumps and two for coupling to the external pressure transducer or pressure transducers. As depicted in first Balloon Assembly 1100E the two balloons, Balloon A 11130 and Balloon B 11120 are each attached at either end to the Catheter 11170 via Fittings 11110 which may be physical seals attached to the balloon and catheter or fixing means such as thermocompression, epoxy, resin, etc. according to the materials employed for the balloons and the catheter. Balloon A 11130 may be a single opening balloon. It would be evident that other configurations with N balloons, N being a positive integer, may be employed where increasing N may lead to the integration of microelectromechanical systems (MEMS) pressure monitors and electrical interfaces rather than additional bores within the catheter.

Considering second Balloon Assembly 1110F then a single Balloon 11150 is employed with an interior wall 11160 to form first and second Chambers 11150A and 11150B respectively. It would be evident that other configurations with N balloons, N being a positive integer, may be employed where increasing N may lead to the integration of microelectromechanical systems (MEMS) pressure monitors and electrical interfaces rather than additional bores within the catheter.

Now referring to Figure 12 there is depicted schematically a Manometry Cap approach for use in conjunction with a VAMA system according to embodiments of the invention for automatically retaining the manometry balloon during patient physiology characterisation. It would be evident that a balloon for use in manometry and ultrasound measurements inserted within the vaginal canal should be maintained not only within the vaginal canal but also in a consistent position as it is inflated, and measurements are taken. Ideally, a small portion of the balloon will be maintained outside of the introitus so that the area of the introitus can be imaged without artifact from mechanisms aimed at keeping the balloon inside. The area near the introitus, i.e. distal vagina, is of particular importance given that the pelvic floor muscles wrap around the vagina in this region. Therefore, the ability to obtain a clear characterization of this region is of significant importance. Accordingly, maintaining the position and insertion of the balloon requires physical pressure be applied. Whilst this could be the patient's hand(s) or clinician's hand(s) these are liable to movement, fatigue, etc. as well as being inconvenient or a personal issue for the patient with the clinician etc. In order to address this the inventors have established a structural shield encasing the area on, near, and/or around the vulva with an outer bag or balloon which provides sufficient pressure to hold internal vaginal devices or components (such as a manometry bag or balloon) inside the vagina without obscuring the critical region near the introitus. The manometry balloon whilst providing pressure / volume data also provides a high contrast with surrounding anatomy when used in medical imaging (e.g. ultrasound). The balloon material and/or fill fluid may be selected to enhance this contrast.

As depicted in Figure 12 the Balloon 1220 with inserted Tube 1210 (e.g. catheter) are inserted within the vaginal canal. Disposed external to the vagina is an Outer Cushion 1240 and Structural Shield 1230 together with an Imaging Probe 1250 (e.g. ultrasound probe). As depicted the Tube 1210 passes through the Outer Cushion 1240 and Structural Shield 1230 but within other embodiments of the invention it may not pass through either or it may pass through one or other of these elements. The Outer Cushion 1240 is formed from and/or has an outer skin and filler formed from a material or materials which are transparent to the imaging probe signals, e.g. an ultrasound transparent material or materials. The Outer Cushion 1240 may be formed from a material which is non-expandable to avoid the Outer Cushion 1240 going into introitus. For example, the Outer Cushion 1240 may be formed from a silicone or it may be a silicone filler encased within an outer shell. Alternatively, the Outer Cushion 1240 may be formed from gelatin with a thin high elastic modulus silicone shell. The Structural Shield 1230 is formed from a rigid material such that the Outer Cushion 1240 is pushed against the patient's body. For example, the Structural Shield 1230 may be formed from a rigid plastic, a fiber reinforced plastic (e.g. carbon fiber or Kevlar reinforced), for example), or a metal/alloy etc. Within embodiments of the invention the Structural Shield 1230 and Outer Cushion 1240 may be a single piece-part which may be a consumable element such that it is only used with one patient or they may be different parts such that the Outer Cushion 1240 is a consumable element, but the Structural Shield 1230 can be reused with appropriate sanitization / cleaning etc. For example, using a thin stainless steel Structural Shield 1230 would render it compatible with the same cleaning processes for autoclavable medical tools whilst a plastic Structural Shield 1230 may be UV cleaned, chemically sterilized as well as autoclavable, for example. Such plastics may include, but not be limited to, PEEK and polysulfone.

Referring to Figure 13 there are depicted hand drawn three dimensional rendering of a Structural Shield 1230 and Outer Cushion 1240 such as that employed in Figure 12 according to an embodiment of the invention from different viewpoints.

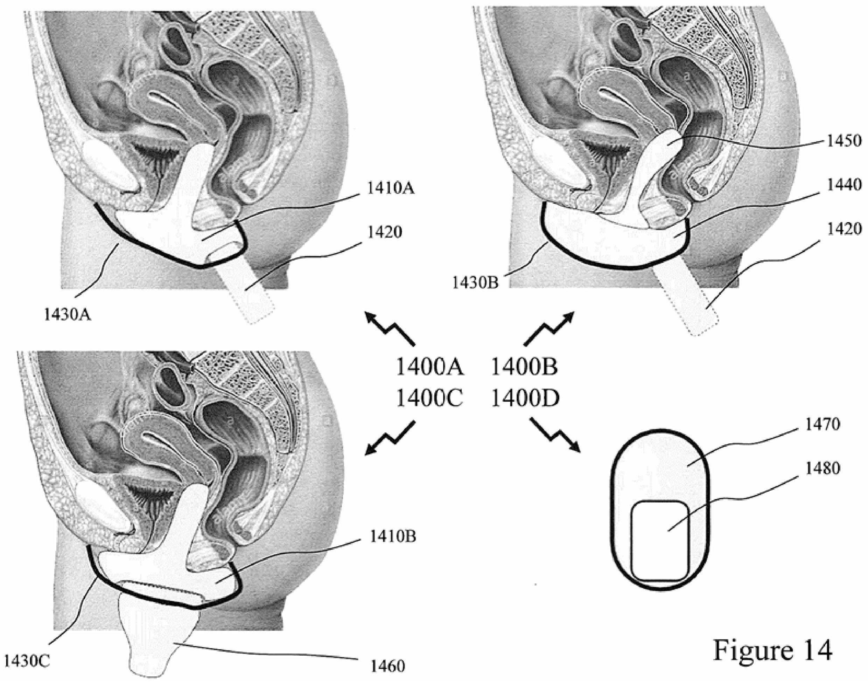

Now referring to Figure 14 there is depicted first to third exemplary structural shield /outer cushion designs (Cushioned Shield) 1400A to 1400C respectively for ultrasound imaging integration with a VAMA system according to embodiments of the invention for automatically retaining the manometry balloon and define placement of the ultrasound probe head during patient physiology characterisation. Referring to first Cushioned Shield design 1400A there is depicted Cushion Balloon 1410A, External Shield 1430A and Probe 1420. Connections for manometry being omitted for clarity. As depicted the Cushion Balloon 1410A integrates the outer cushion, such as Outer Cushion 1240 in Figure 12, with the manometry balloon such that a single piece part is employed. Optionally, a semi-rigid ring may be disposed at the periphery of the balloon as it joins the cushion to aid in the patient aligning the Cushion Balloon 1410A. The Probe 1420 as depicted being insertable through an opening within the External Shield 1430A.

Within second Cushioned Shield design 1400B the Balloon 1450 and Outer Cushion 1440 are separate elements. Also depicted are the Probe 1420 and Structural Shield 1430B. Referring to third Cushioned Shield design 1400C the Balloon and Outer Cushion are again depicted as a single Cushioned Balloon 1410B in conjunction with a Probe 1460 and Structural Shield 1430C. The opening, for example Opening 1480 in Image 1400D within the Structural Shield 1470 may be dimensioned to retain a specific probe in a predetermined position or it may be dimensioned to allow positioning of the probe to be varied either during an initial configuration step prior to measurements or during the measurements. Optionally, multiple openings may be provided such that, for example, Probe 1420 can be initially employed and then Probe 1460 employed or vice-versa.

Accordingly, within embodiments of the invention:

• the balloon, outer cushion and structural shield may be discrete separate elements;
• the balloon and outer cushion may be a combined element used in conjunction with a separate structural shield; or
• the outer cushion and structural shield may be a combined element used in conjunction with a separate balloon.

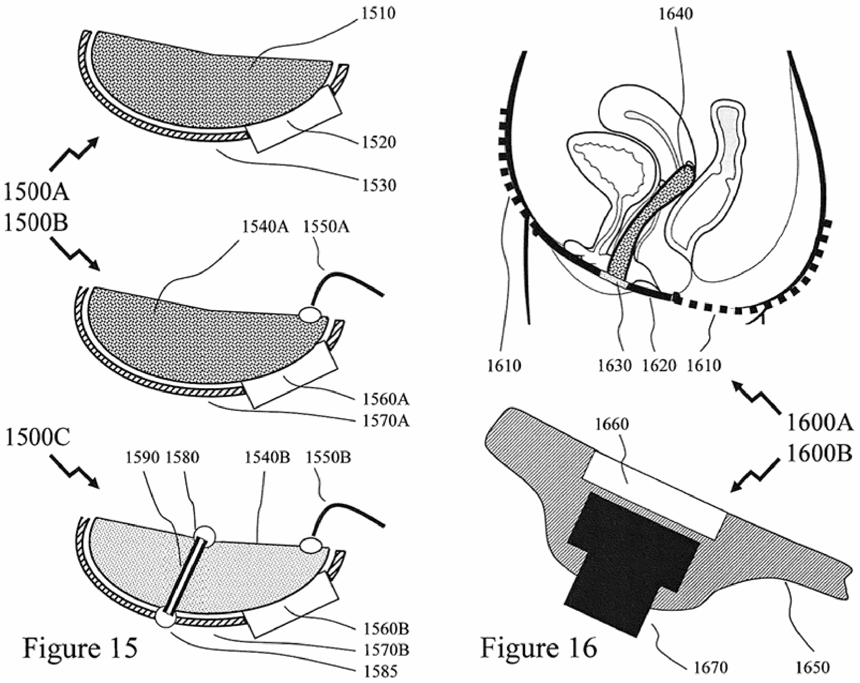

Referring to Figure 15 there are depicted exemplary structural shield / cushion designs, first to third Shielded Cushions 1500A to 1500C, according to embodiments of the invention. As depicted first Shielded Cushion 1500A comprises Cushion 1510 and Structural Shield 1530 with integral Opening 1520 wherein the Cushion 1510 is formed from an elastic (low elasticity modulus) material, such as a silicone, foam, elastomer, gelatin etc. within a high or medium elastic modulus material shell so that the Cushion 1510 whilst conforming the patient's body does not penetrate their vaginal canal, for example.

Second Shielded Cushion 1500B comprises a Cushion 1540A with Filler Tube 1550A and Structural Shield 1560A with integral Opening 1570A. Accordingly, in this variant the Cushion 1540A is essentially another balloon but formed from a medium or high elastic modulus material which is filled with a fluid via the Filler Tube 1550A. Whilst the Filler Tube 1550A is depicted upon the upper surface of the Cushion 1540A which would be disposed towards the patient's body it would be evident that it may be upon another surface of the Cushion 1540A in a region of the Cushion 1540A exposed relative to the Structural Shield 1570A but not passing through the Structural Shield 1570A. Optionally, the Filler Tube 1550A may connect to the Cushion 1540A through the Structural Shield 1570A. Optionally, the Filler Tube 1550A may be a fluidic connector rather than a tube allowing a tube with mating fluidic connector to be connected to it for filling the Cushion 1540A. The fluid for filling the Cushion 1540A may be water, a low viscosity silicone oil, nitrogen, air, or another fluid which may or may not be the same as that employed to inflate the manometry balloon. The Structural Shield 1570A incorporating an Opening 1560A for insertion of a measurement probe through the Structural Shield 1570A into contact with or in proximity to the Cushion 1540A.

Third Shielded Cushion 1500C comprises a Cushion 1540B with Filler Tube 1550B, and a Structural Shield 1570B with Opening 1560B.These being similar to the corresponding elements in second Structural Cushion 1500B denoted by common identifiers with an "A" instead of a "B." Third Structural Cushion 1500C also incorporates a first Fluidic Connector 1580, a second Fluidic Connector 1585 and a Tube 1590 connecting the first Fluidic Connector 1580 to the second Fluidic Connector 1585. Accordingly, a balloon may be connected to the first Fluidic Connector 1580 prior to placement of the Structural Cushion 1500C against the user and the external fluid source and pump / pressure transducer etc. connected to the second Fluidic Connector 1585. For simplicity, a single connector supporting two or more fluidic channels may be employed or alternatively multiple connectors may be employed each for fluid flow into or pressure monitoring etc.

Whilst the Filler Tube 1550A is depicted upon the upper surface of the Cushion 1540B which would be disposed towards the patient's body it would be evident that it may be upon another surface of the Cushion 1540B in a region of the Cushion 1540B exposed relative to the Structural Shield 1570B but not passing through the Structural Shield 1570B. Optionally, the Filler Tube 1550B may connect to the Cushion 1540B through the Structural Shield 1570B. Optionally, the Filler Tube 1550B may be a fluidic connector rather than a tube allowing a tube with mating fluidic connector to be connected to it for filling the Cushion 1540B. The fluid for filling the Cushion 1540B may be water, a low viscosity silicone oil, nitrogen, air, or another fluid which may or may not be the same as that employed to inflate the manometry balloon.

Now referring to Figure 16 there is depicted an exemplary design for "membrane clothing" integrating a structural shield and/or ultrasound probe holder according to embodiments of the invention. Within first image 1600A a Balloon 1640 is deployed and retained in position by a Structural Element 1620 retained in position by a Fitting 1610. The Structural Element 1620 is also depicted as comprising a Region 1630 allowing a measurement probe to be located externally to the Structural Element 1620. The Region 1630 being necessary to provide transparency to the signals of the probe is the material of the Structural Element 1620 absorbs, reflects, or has high attenuation of the signals from the probe. The Structural Element 1620 is formed from a material with medium or high elastic modulus such that the Balloon 1640 is retained in position. The Fitting 1610 may be, for example, straps to fit the Structural Element 1620 and retain such as known in the art with clothing articles such as a jockstrap or protective equipment such as a box. The Fitting 1610 may have the Structural Element 1620 integrated into the Fitting 1610 such that the Fitting 1610 and Structural Element 1620 are similar to an item of clothing in that it is worn by the user rather than fitted. This Fitting 1610 may be elasticated so allow it to be worn but retain the Structural Element 1620 against the user's body. Optionally, the Structural Element 1620 and Region 1630 are the same. Optionally, the Region 1630 may be a thinner region of the same material employed to form the Structural Element 1620.

Referring to second image 1600B there is depicted a variant region of an item such as Fitting 1610 wherein the Region 1660 provides for transparency of the signals from the Probe 1670 when the material forming the majority of the item absorbs, reflects, or highly attenuates the signals from the probe. As such Region 1660 acts as a window within the Structural Element 1650 but now the Structural Element 1620 supports attachment of the Probe 1670 to the Structural Element 1620. For example, the Structural Element 1650 may be formed from an elastic material such that an opening within it supports insertion and removal of the Probe 1670 and maintains the Probe 1670 in position through the Structural Element 1650 fitting around it. Optionally, within other embodiments of the invention the Probe 1670 may be attached to the Structural Element 1650 via other demountable attachments means including, but not limited, hook-and-loop, hooks, snap fasteners etc. Optionally, the Probe 1670 may be attached to the Structural Element 1650 via an intermediate fitting which attaches to standard locations on the Structural Element 1650, but different intermediate fittings allow different Probes 1670 to be employed with a common Structural Element 1650.

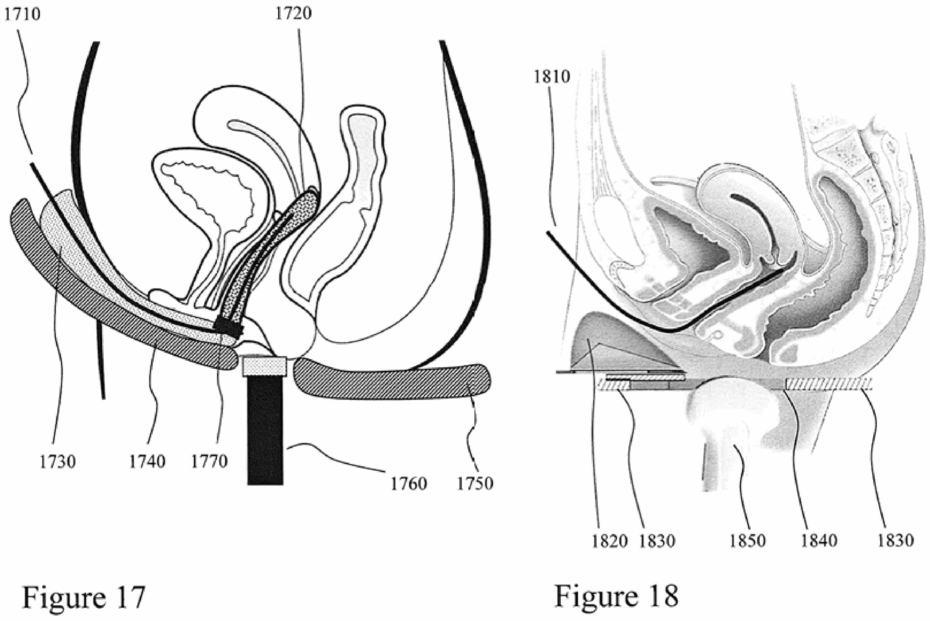

Referring to Figure 17 there is depicted an inflatable support according to an embodiment of the invention supporting use with a VAMA system in conjunction with a seat for supporting the anterior portion of the patient with integral ultrasound probe window or holder according to an embodiment of the invention. As depicted a Balloon 1720 is in use and is retained in position by an inflatable Plug 1770 which forms a ring around the Catheter 1710 as it transitions from the Balloon 1720 to Cushion 1730. The Cushion 1730 is disposed between the patient and the Front Seat Portion 1740 whilst the patient rests directly onto the Rear Seat Portion 1750. Within other embodiments of the invention with rectal rather than vaginal insertion of the Balloon 1720 then the Cushion 1730 may be upon the Rear Seat Portion 1750 and the patient either rests directly on the Front Seat Portion 1740 or upon another cushioning element. Such an additional cushioning element may also be employed upon the Rear Seat Portion 1750 as depicted in the configuration of Figure 17. This additional cushioning may be a thin padded sheet which is disposable. The additional cushioning may be separate to Cushion 1730 or integral with it.

The Front Seat Portion 1740 and Rear Seat Portion 1750 being defined relative to the user's body and forward / back from an opening within the seat of which they form part that allows insertion of the Probe 1760 for measurements. Optionally, the Plug 1770 is integral to the Balloon 1720 so that it remains in position relative to the patient as they move and moves with them. Optionally, the Cushion 1710, Plug 1770 and Balloon 1720 are a single piece part. Optionally, the Plug 1770 may be inflatable independent of the Balloon 1720, where inflating the Plug 1770 would grip the catheter tighter and push against the Cushion 1730 and/or Front Seat Portion 1740 providing support to hold the manometry bag inside but without a rigid element pushing against the user causing discomfort or impacting the measurements. Optionally, the Catheter 1710 may be between the patient and the Cushion 1730 or between the Cushion 1730 and the Front Seat Portion rather than through the Cushion 1730 as depicted.

Now referring to Figure 18 there is depicted an inclined Cushion 1820 for the anterior support of the patient according to an embodiment of the invention use with a VAMA system in conjunction with a Seat 1830 for supporting the anterior portion of the patient with integral Probe Window 1840 for the Probe 1850 or a holder for the Probe 1850 according to embodiments of the invention. The Catheter 1810 running between the patient's body and the Cushion 1820 within the configuration shown although it may within other embodiments of the invention be disposed between the Cushion 1820 and the Seat 1830 or be through another opening within the Seat 1830 or through the Probe Window 1840. The balloon has been omitted for clarity. The inclined Cushion 1820 provides support anterior of the body where internal device exists and reduces likelihood that it falls out, becomes dislodged etc. The Cushion 1840 being designed to temporarily keep internal devices inside during the procedure.

This concept is extended in Figures 19 and 20. Referring to Figure 19 there is depicted a vaginal manometry Plug 1930 according to an embodiment of the invention for retaining the manometry balloon within the vaginal canal during VAMA system characterization of a patient's physiology in conjunction with a Seat 1960 for supporting the anterior portion of the patient with integral Probe Window 1940 or holder allowing the Probe 1950 to take measurements. according to an embodiment of the invention. Accordingly, a Catheter 1910 is depicted as deployed although the balloon is not shown for clarity. The Catheter 1910 as shown runs along the surface of a Cushion 1920 between the patient and the Cushion 1920 before entering the Plug 1930 and therein into the patient. Accordingly, the Plug 1930 is designed to maintain the balloon or another device deployed internally in position during measurement and characterisation procedures.

Referring to Figure 20 there is depicted a plug according to an embodiment of the invention for retaining the manometry balloon within the vaginal canal during VAMA system characterization of a patient's physiology such as Plug 1930 in Figure 19. As depicted the Plug 2020 supports the Catheter 2010 through the middle of the Plug 2020. First to third Plugs 2030 to 2050 depict different cross-sections of plugs such as Plug 1930 and Plug 2020 in Figures 19 and 20 respectively. Accordingly, the plug may be selected according to the user's physiology from a standard set of plugs or within another embodiment of the invention the plug is another USTD fitted specifically to the user based upon dimensions being obtained in an initial measurement stage prior to the manometry and/or probe measurements. In this manner the plug may be fitted specifically to the user's physiology improving comfort and user experience in what may be an extended characterisation and/or measurement process. Optionally, the plug such as depicted in Figures 19 and 20 may be employed in conjunction with an item of clothing such that the plug and catheter (and balloon) are retained in position as the user moves from a supine to seated position of vice-versa.

Optionally, the item of clothing may include or be coupled to all or a subset of a pump, a fluid reservoir, and a pressure transducer to provide a mobile manometry system for obtaining pressure / volume data of the user in a variety of activities either within a specific clinical environment or within everyday life. The plugs depicted in Figures 19 and 20 being formed from a material transparent to the signals from a probe or probes, such as Prob 1950 in Figure 19 so that the plug does not interfere with the measurements performed by the probe. The plug may be formed from a material with low elastic modulus, high elastic modulus, or medium elastic modulus. Optionally the plug may be formed from a material with high or medium elastic modulus for the majority but the upper tip portion may be formed from a soft elastic modulus material to enhance user comfort, patient fit etc.

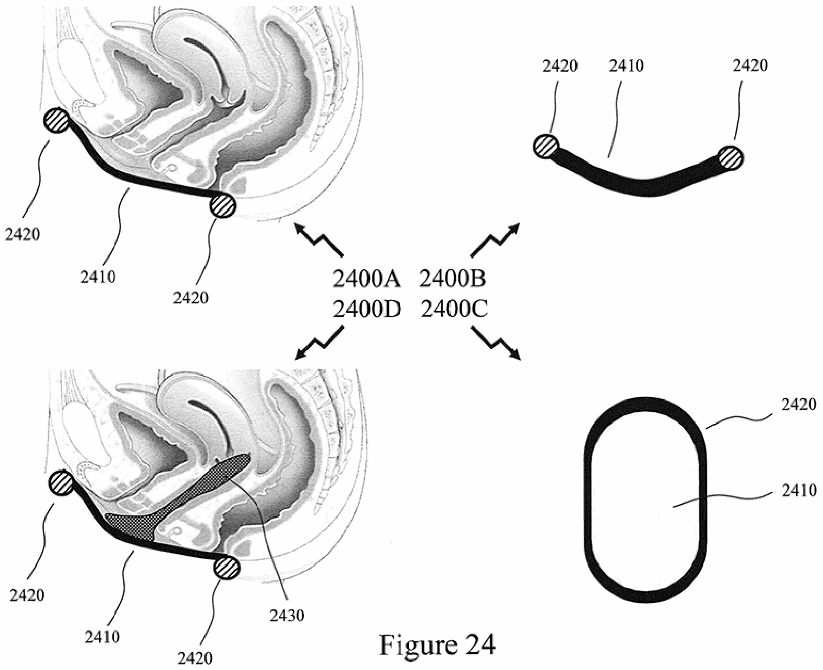

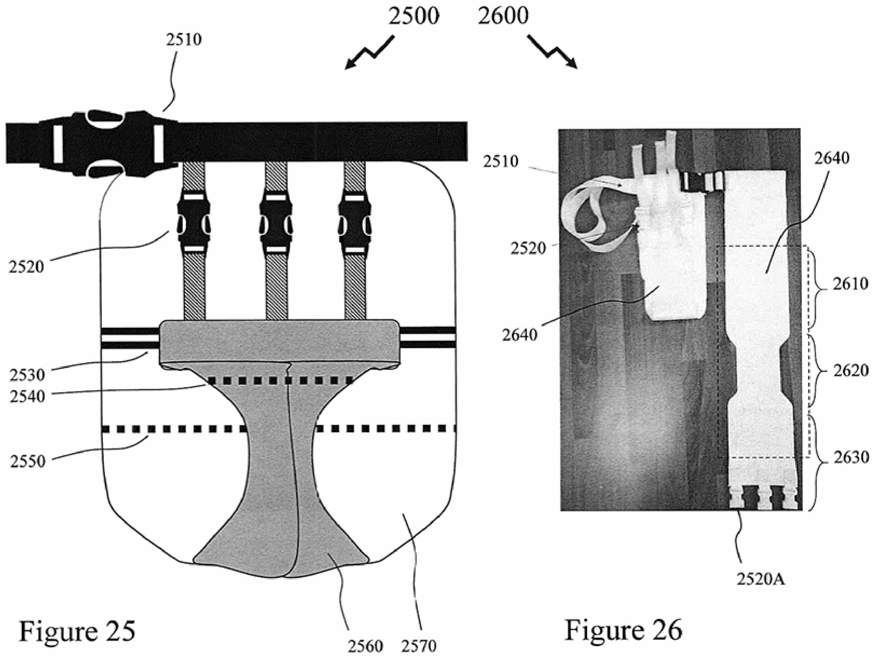

Now referring to Figure 24 there are depicted first to fourth Images 2400A to 2400D respectively for membrane underwear according to an embodiment of the invention allowing coupling of a probe to the user's body for obtaining anatomical images and measurements to support other embodiments of the invention. As depicted in first Image 2400A, which is a cross-sectional image of the abdominal region of user wearing the membrane underwear there is depicted a Membrane 2410 portion and a Frame 2420 portion of the membrane underwear according to an embodiment of the invention. Second Image 2400B depicts a cross-section (orthogonal to the axis of first Image 2400A) of the membrane underwear in isolation whilst third Image 2400C depicts a plan view of the membrane underwear. In each the Membrane 2410 and Frame 2420 are depicted. The Membrane 2410, in combination with ancillary elements such as described and depicted in Figures 25 to 27B provides a user with an item of underwear to cover them whilst providing a region (Membrane 2410) which provides transparency for signals generated and/or detected by a sensor probe disposed externally to the user.

Such an item of membrane underwear may be employed discretely, or it may be employed in conjunction with embodiments of the invention such as described and depicted in Figures 12 to 20 respectively. The composition of the material for the Membrane 2410 being established for transparency of the signals generated and/or detected by the sensor with, optionally, consideration of one or more other factors including, but not limited to, cost, opacity in the visible wavelength range, flexibility, attachment of the Frame 2420, ad cleaning.

Within other embodiments of the invention the Frame 2420 may have limited or no compliance whilst the Membrane 2410 is pulled taught either due to automatic or manual expansion of the Frame 2420 or other elements attached to the Frame 2420. Optionally, the Membrane 2410 may be adjusted through the Frame 2420 and/or other elements in order to provide different levels of tension of the Membrane 2410. The Membrane 2410 is described with respect to Figure 24 as being within an item of underwear although within other embodiments of the invention the Membrane 2410 and Frame 2420 may be deployed without other elements normally associated with an item of underwear.

Optionally, the Membrane 2410 and Frame 2420 may form part of a jockstrap or other element of clothing such as tights, leggings, etc.

For example, the Membrane 2410 may be formed from a silicone. Optionally, referring to fourth Image 2400D the Membrane 2410 may be designed to retain an Internal Device 2430 within a cavity of the user, e.g. vaginal canal. Internal Device 2430 may, for example, be a 2D probe, 3D probe, other measurement / data acquisition device and/or system.

Within embodiments of the invention the membrane underwear may be designed to provide one or more of the following:

- Fully covers vaginal introitus (opening) and/or perineum
- Simple size adjustment, e.g. from tension straps such as described below in respect of Figure 25;
- Simple adjustment of tension of the Membrane 2410 to ensure that the membrane underwear fits tight against the vaginal opening, for example, without discomfort;
- Provide an "Imaging Zone" (e.g. which covers vaginal opening and perineum) through the Membrane 2410 which may be formed from one or more layers of material and is clear of elements within this region that may cause discomfort and/or interfere with measurements etc.;
- The Membrane 2410 may be formed from multiple zones of different materials to support a signal generating probe and signal acquisition probe where the material for signal acquisition probe is selected to enhance imaging quality whilst the material for the signal generating probe is selected for optimum coupling of the signals to the user's body;
- May include structure supports (ribs, elastic, strap, or buckles) to prevent bunching up of material at the perineum
- Can be worn against bare skin in sensitive areas for extended periods of time, e.g. several hours, and be comfortable for the duration whilst the user is laying on their back (e.g. on a medical bed) or sitting upon analysis / measurement equipment;
- Easy to use, e.g. easy to tell front/back, where each component is supposed to attach and adjust etc.;
- Can be put on and removed by the user alone, who may have potentially limited mobility);
- Machine washable and/or autoclavable if reusable rather than disposable.

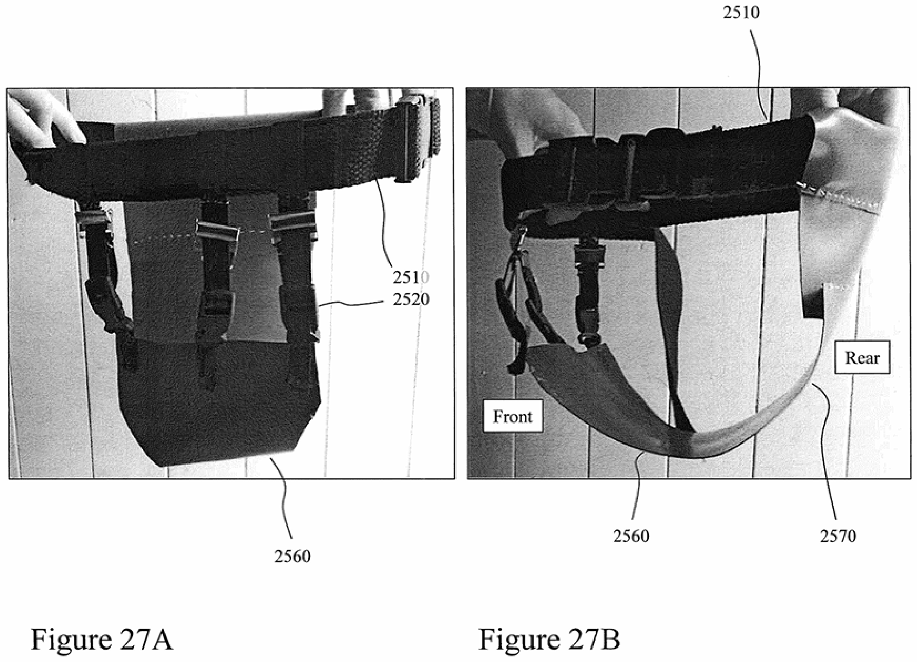

Optionally, the Membrane 2410 and Frame 2420 may be a disposable element of membrane underwear employed with a reusable attachment and adjustment means. Referring to Figures 25 to 27B there are depicted images of exemplary prototype membrane underwear according to embodiments of the invention. Referring initially to Figure 25 there is depicted a front view of a Membrane Underwear 2500 according to an embodiment of the invention whilst front and side views of an initial physical prototype are depicted in Figures 27A and 27B respectively. As depicted the Membrane Underwear 2500 comprises:

- Adjustable Waistband 2510 which whilst depicted as employing an adjustable buckle may be optionally a simple elasticated waistband within other embodiments of the invention;
- Tension Straps 2520 which whilst depicted as employing adjustable buckles may be optionally a simple elasticated waistband within other embodiments of the invention;
- Type A Structural supports 2530 within a Rear Portion 2570 to provide structural shape and/or retention of the Rear Portion 2570 with respect to the user's posterior;
- Type B Structural support 2540 within a Membrane 2560 to provide one or more shape, tension, or retention of the Membrane 2560 with respect to the user's body;
- Type C Structural support 2550 within the Rear Portion 2570 to provide structural shape and/or retention of the Rear Portion 2570 with respect to the user's posterior;
- Membrane 2560 which may be, within embodiments of the invention transparent; and
- Rear Portion 2570.

As evident from Figure 27B the Rear Portion 2570 may be a solid element joined to the Waistband 2510 or it may alternatively with attached via further Tension Straps 2520. Within embodiments of the invention the Membrane 2560 may be formed from two or more materials such that the outer portion of the Membrane 2560 is opaque both visually and to the signals of the measurement / analysis devices (e.g. probes) whilst an inner portion or portions is opaque visually but transparent to the signals of the measurement / analysis devices (e.g. probes).

An alternate design is depicted in Figure 26 with Membrane Underwear 2600. As depicted the Membrane Underwear 2600 comprises on the left hand side the Waistband 2510 and Tension Straps 2520 where the Body 2640 of the Membrane Underwear 2600 is folded over. On the right hand side the Body 2640 is depicted open with Tension Strap Elements 2520A at the bottom (which form part of attaching the front of the Body 2640 to the Waistband 2510 at the front. The Body 2640 comprising first to third Regions 2610 to 2630 which comprise rear body cover, imaging zone and front body cover portions, respectively. The imaging zone (second Region 2620) being Membrane 2410 in Figure 24 for example.

Within embodiments of the invention the Body 2640 may be completely detachable from the Waistband 2510 rather than permanently attached at the rear and demountably attachable at front (or vice-versa). Optionally, the Membrane Underwear 2600 as well as Membrane Underwear 2400 and 2500 in Figures 24 and 25 respectively may be one-size-fits-all through the adjustments provided by Waistband 2510, Tension Straps 2520 etc. and elasticated materials to form the Body 2640. Optionally, several sizes may be employed such as small, medium, and large or juvenile / adult etc. Optionally, the Membrane Underwear 2600 may be configured differently for male and female users.

Optionally, the Membrane Underwear such as membrane underwear depicted in Figures 24 to 26 may comprise of a single piece with regions of varying rigidity and/or structure built into the underwear so that different regions of the Membrane Underwear have different levels of tension. For example, such varying tension within different regions of a single item of clothing such as the Membrane Underwear can be formed through knitting.

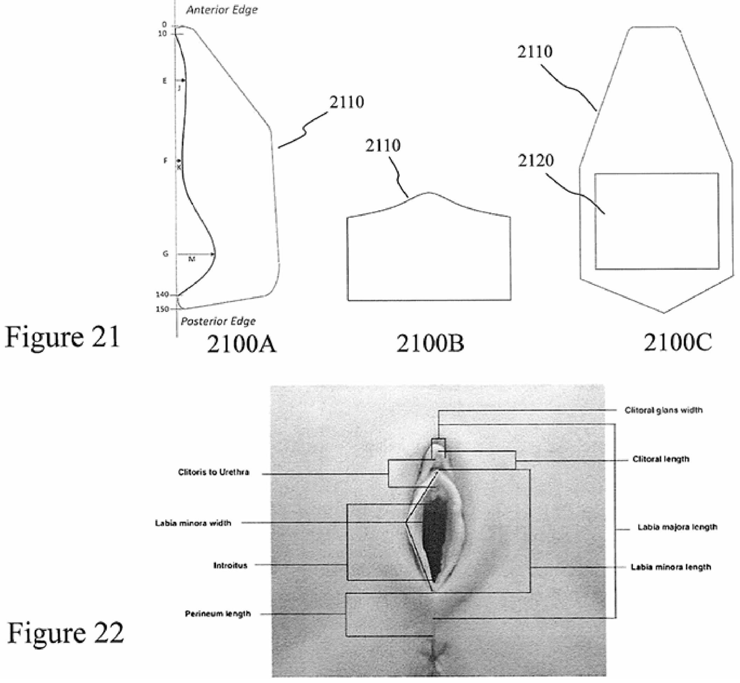

Referring to Figure 21 there is depicted an exemplary geometry of a Cup 2110 according to an embodiment of the invention for retaining the manometry balloon within the vaginal canal during VAMA system characterization of a patient's physiology in conjunction with an access port for an ultrasound probe. Within the preceding description a variety of plugs, cushions, structural supports etc. have been described and depicted with respect to maintaining a balloon in place in combination with measurements performed by one or more probes and/or a manometry system. Within these the cushion has been defined typically as being formed from a material of low or medium elastic modulus in conjunction with a structure shield formed from a material of high elastic modulus. The Cup 2110 accordingly, may be Manometry Cap 380 in Figure 3 or the Structural Shield 1230 in Figures 12 and 13 respectively, Structural Shields 1430A/1430B/1430C and 1470 in Figure 14, Structural Shields 1530/1560/1570A/1570B in Figure 15. Within Figure 21 the Cup 2110 is depicted in side elevation view in first Image 2100A, end elevation view in second Image 2100B and bottom elevation view in third Image 2100C. Evident within third Image 2100C is the Opening 2120 for the probe.

Referring to Table 1 below are surface anatomy measurements for different populations based upon age range where these surface anatomy measurements are depicted in Figure 22. Also presented in Table 1 are the dimensions adopted by the inventors for a pelvic floor model employed to establish the design for an initial cup, such as Cup 2110 in Figure 21. As evident in first Image 2100A in Figure 21 the overall length of the Cup 2110 is 150mm (approximately 6").

Table 1: Surface Anatomy Population Measurements

| Anatomy | Percentile | Age Group (years) | | | | | Pelvic Floor Model* |
| | | 35-44 | 45-54 | 55-64 | 65-74 | 75-84 | |
| --- | --- | --- | --- | --- | --- | --- | --- |
| Clitoral Length (mm) | 5 | 3 | 2.25 | 3 | 3 | 1.6 | 8 |
| | 50 | 6 | 5 | 5 | 5 | 4 | |
| | 95 | 19.85 | 14 | 17.95 | 13.85 | 17 | |
| Labia minora Length (Right) (mm) | 5 | 23 | 20.5 | 20 | 15 | 6 | 64 |
| | 50 | 50 | 44.5 | 31 | 30 | 30 | |
| | 95 | 82.55 | 82.25 | 70 | 55 | 62 | |
| Perineum Length (mm) | 5 | 10 | 10 | 10 | 8.1 | 6.2 | 37 |
| | 50 | 22 | 20 | 20.5 | 20 | 18 | |
| | 95 | 37.55 | 40.5 | 35 | 35 | 32 | |
| Introitus Opening (mm) | 5 | 14 | 14 | 12 | 10 | 11.6 | 40 |
| | 50 | 28.5 | 26 | 30 | 26 | 25 | |
| | 95 | 45 | 46.75 | 45 | 49.9 | 40 | |
| Clitoral - Anus Length (clitoral + labia minora + perineum) (mm) | 5 | 36 | 32.75 | 33 | 26.1 | 13.8 | |
| | 50 | 78 | 69.5 | 56.5 | 55 | 52 | |
| | 95 | 139.95 | 136.75 | 122.95 | 103.85 | 111 | |

Now referring to Figure 23 there are depicted first to fourth computer aided design (CAD) Schematics 2300A to 2300D of manometry cups according to embodiments of the invention. First Schematic 2300A depicts the design of the cup essentially as depicted in first to third Images 2100A to 2100C respectively in Figure 21 except the posterior topography is broader so that the "bump" is less pronounced, and the anterior tip is narrower. Second Schematic 2300B depicts a variant wherein the "bump" within the posterior topography is accentuated further. In third Schematic 2300C the geometry has changed substantially to an elongated pair shape with reduced profile variations, narrower posterior geometry and sharper rounded anterior. In each of first to third Schematics 2300A to 2300C respectively the cup may be employed discretely with the cushion, plug etc. as described and depicted above.

However, in fourth Schematic 2300D the Cup 2310 is employed in conjunction with a Gasket 2320 so that the periphery of the Cup 2310 formed from a high elastic modulus material is softened by the low or medium modulus elastic material employed for the Gasket 2320. The Cup 2310 is also depicted with a Hole 2330 for insertion of a tube, catheter etc. The Gasket 2320 around the entire top surface in addition to enhancing patient comfort also seals where it makes contact with the patient's skin. The Hole 2330 may be one of several holes or other mechanical structures to the Cup 2310 either for tubing interface, fluid connectors etc. or to secure various outer inserts in place such as the cushion, plug etc.

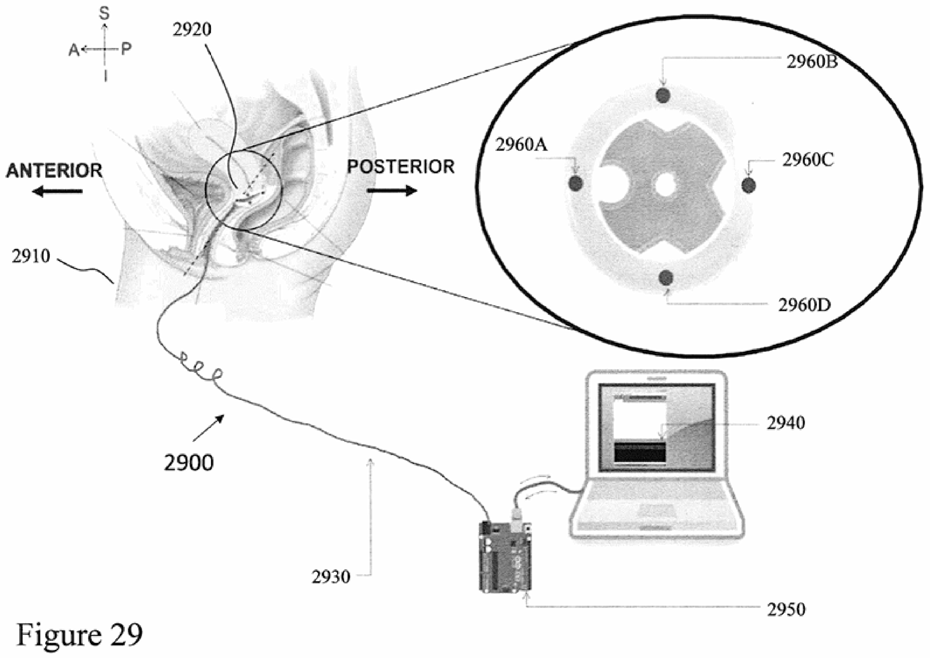

As discussed above embodiments of the invention support the design and implementation of custom USTD devices tailored to a patient's physiology, symptoms, etc. Within embodiments of the invention this includes the use of VAMA systems, 2D ultrasound, 3D ultrasound etc. However, within other embodiments of the invention it may be appropriate to employ a pessary to establish patient related data. Accordingly, referring to Figure 29 there is depicted a Pessary Data Acquisition System (PDAS) 2900 according to an embodiment of the invention for establishing data for fitting USTD devices according to embodiments of the invention. Accordingly, as depicted user 2910 is wearing a Pessary Sensing System (PSS) 2920 is depicted coupled via Cable 2930 to Data Acquisition Module (DAM) 2950. As depicted the DAM 2950 is coupled to a PED with Software 2940 in execution to acquire data from DAM 2950 and therein from the PSS 2920. The PED may push the data to a remote server, see for example Figures 3 and 28 for example. Optionally, the PSS 2920 may comprise a short cable / antenna such that the PSS 2920 is wirelessly coupled to the DAM 2950. The DAM 2950 may be integrated within the PED or be a card inserted into the PED.

The PSS 2920 provides a force/pressure sensing system integrated within a pessary or size-adjustable tool or structure that functions to monitor the pressures and/or forces in distinct regions of the fitted USTD device. As depicted in the insert in Figure 29 the PSS 2920 comprises first to fourth Sensors 2960A to 2960D respectively around the periphery of the PSS 2920. A means to orient the PSS 2920 would be integrated into the design such that the data collected can be correlated with regions of the female urogenital anatomy. For example, this means may be markers visible upon a system such as an ultrasound probe, X-ray, etc. Accordingly, position / orientation of the PSS 2920 within the patient, relative to anatomical directions (anterior, posterior, left, right), may be established and the sensor data from the first to fourth Sensors 2960A to 2960D accordingly orientated with respect to the patient's urogenital anatomy through the use of inertial measurement units (IMUs) or equivalent sensors and devices.

Within other embodiments of the invention the number of sensors may be varied from four. Within other embodiments of the invention the sensors may comprise those providing force / pressure data as well as other sensors providing additional data such as temperature, pH, humidity, etc. The inventors refer to such devices as PSS 2920 as Smart Fitting USTD devices.

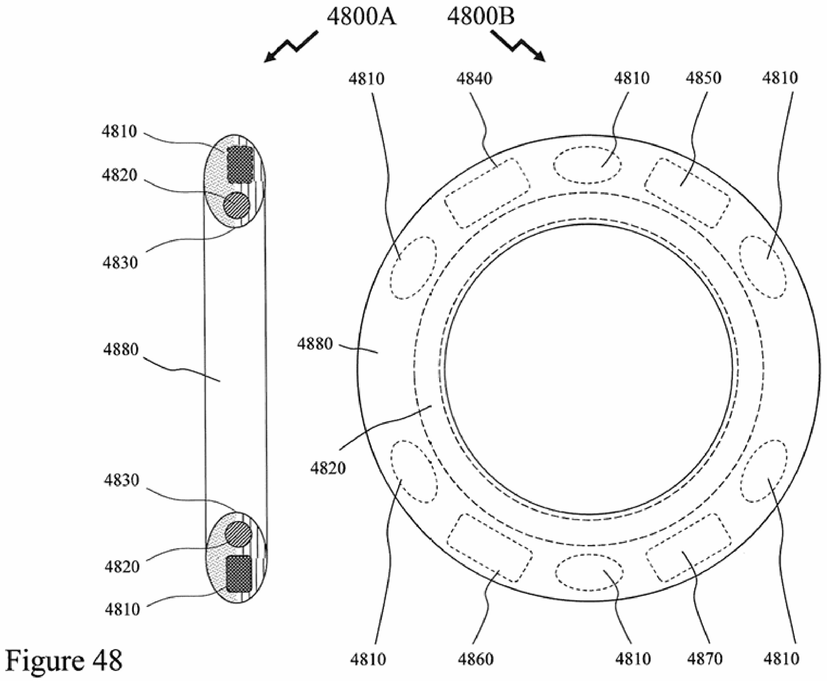

Referring to Figure 48 there is depicted an exemplary device for fitting USTD devices according to embodiments of the invention that includes sensors to measure strain, force, and/or pressure. As depicted in cross-section 4800A and plan view 4800B the USTD comprises a Body 4880 within which are disposed sensors and ancillary electronics. Disposed around the inner periphery of the USTD is a Distributed Element 4820 which may for example be one or more piezoelectric elements disposed to provide local displacement measurements through the voltage generated in the distortions of the one or more piezoelectric elements or provide adjustment in the inner periphery through their electrically induced expansion. Such piezoelectric elements may include, for example, polyvinylidene difluoride (PVDF). Alternatively, the Distributed Element 4880 may comprise one or more micro-motors or other elements for pushing / pulling connecting elements to adjust the opening of the USTD. Optionally, distributed element 4820 may be omitted.

Disposed around the Body 4880 of the USTD are Sensors 4810 such as providing, for example, temperature, pH, humidity, blood oxygenation, pressure, vibration, etc. These are coupled to Microprocessor 4840 which is powered from Battery 4850. The Microprocessor 4840 also connected to a Wireless Transceiver 4860 which transmits data through Antenna 4870. For example, the Wireless Transceiver 4860 may be Bluetooth or LoRa (Long Range, low-power wide-area network protocol) which provide for transmission through short distances of aqueous media. Alternatively, the Antenna 4870 may a connection from the USTD to an antenna element which is within the user's vagina with an end external to their body. Accordingly, the USTD may acquire data and transmit it to an external logging system.

Optionally, a subset of the Sensors 4810 may include or be transducers such as acoustic transducers, heaters, etc. such that the USTD may acquire or execute specific actions and monitor the resulting effects etc. For example, the USTD may adjust the internal periphery until the Sensors 4810 register a certain pressure is being applied. Optionally, the USTD may include another Distributed Element 4820 around the external periphery of the device to adjust its external peripheral dimension or distort the geometry of the USTD from circular. Similarly, the inner periphery may be distorted through the action of the one or more elements within the Distributed Element 4820. It would be evident that piezoelectric elements may be employed to adjust the dimensions / geometry of the USTD and provide sensor feedback to subsequent pressure etc. applied to them through the Body 4480 of the USTD.

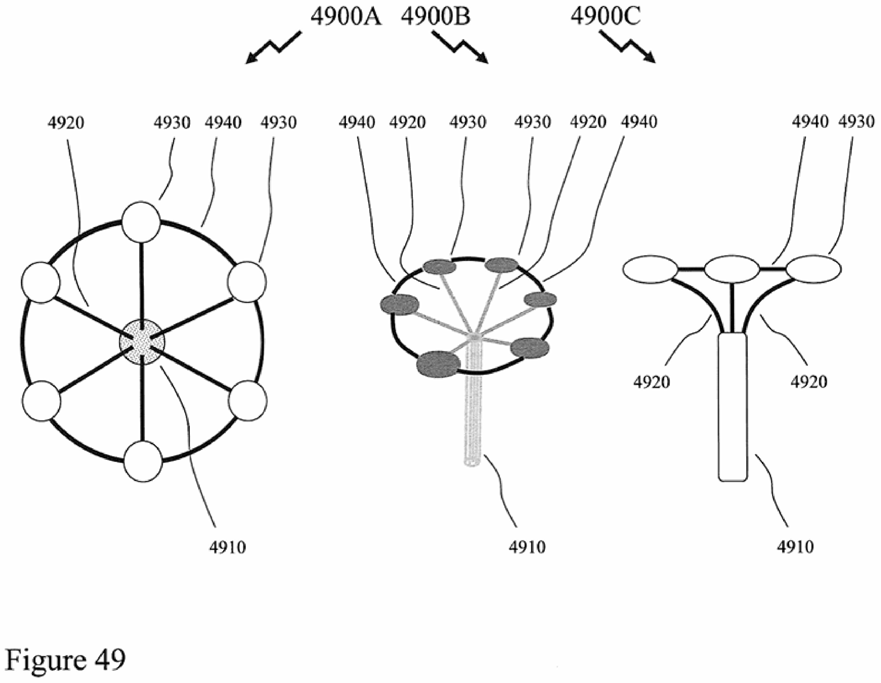

Figure 49 depicts first to third images 4900A to 4900 respectively of an exemplary Sensor Device for fitting USTD devices according to embodiments of the invention that includes sensors to measure strain, force, and/or pressure. First image 4900A being a plan view of the Sensor Device, second image 4900B an upper perspective view of the Sensor Device and third image 4900C a side view of the Sensor Device. As depicted the Sensor Device comprises a central Body 4910 from which a set of Arms 4920 extend each terminating in a Pad 4930. The array of Pads 4940 being connected via Ring Elements 4940. Each Pad 4930 may contain one or more sensors such as temperature, pressure, pH, humidity, etc. and couples these one or more sensors to electronics within the Body 4910 via an Arm 4920. The Body 4910 may be coupled through a wireless or wired connection to data logging system. Where the Body 4910 is wirelessly coupled the antenna may be integrated within the Body 4910 or coupled to the Body 4910. Optionally, the Ring Elements 4940 may be omitted.

Within embodiments of the invention the length of the Arms 4920 may be fixed or within other embodiments of the invention the length of the Arms 4920 may be adjustable wherein these may be extended and/or retracted from the Body 4910 using techniques as known in the art. In retracted and /or extended but undeployed scenarios the Pads 4930 may be towards the axis of the Body 4910. Accordingly, the Arms 4920 may be retracted when the Sensor Device is shipped / stored etc. but extended when measurements are to be taken of a patient. The Arms 4920 may be flexible but semi-rigid such that their relative positions may vary according to the patient's anatomy, but they are retained against the patient's body through their spring like nature seeking to return them to a resting position along the axis of the Body 4910.

Optionally, the Pads 4930 may be coated with a gel to promote adhesion to the user's body when deployed.

Optionally, within embodiments of the invention each Arm 4920 may be extended or retracted discretely from the other Arms 4920 so that they may have different lengths.

Optionally, within embodiments of the invention all Arms 4920 are extended or retracted together so that they have the same length at all times.

Optionally, a subset of the plurality of Pads 4930 comprise sensors, each Pad 4903 of the predetermined subset of the plurality of Pads 4930 comprising a predetermined sensor. Accordingly, each sensor within the plurality of Pads 4930 may be same, different or each Pad 4930 may have differing subsets of multiple sensor types.

Accordingly, as described above embodiments of the invention allow for the collection of patient centric data together with the analysis, design, and manufacture of USTD devices. These embodiments of the invention being directed to predicting and determining USTD device geometries and dimensional parameters in order to optimize the success rate for patients wearing the USTD devices for POP treatment. Accordingly, embodiments of the invention provide for systematically defining each dimensional setting (or variable) through quantifiable input relating to the patient and/or processes relating to analysis etc. Further, another goal for the exemplary processes described and depicted may include minimize the amount of materials employed within the USTD device while providing sufficient supporting forces for each patient's unique individualized POP condition. It a further goal for the exemplary processes described and depicted to facilitate self-management of USTD devices by designing features that facilitate insertion, removal, and cleaning by the patient without dependence on a clinician. Referring to Table 2 exemplary relationships for different components of systems according to embodiments of the invention are presented.

Table 2: Exemplary Component Relationships

| Component | Inputs | Outputs |
|---|---|---|
| POP-Q+ | | |
|     Physical Examination | | Anatomic Measurements: POP-Q+ |
|     Questionnaires | | Patient Demographics <br> QoL |
|     EMR / EHR | | Symptoms <br> Diagno sis <br> Pessary Fitting History |
| Colpodynamic Imaging | | |
| e | 2D Ultrasound Images | Segmented Volume |
| | 3D Volumes with Bag In | Co-ordinate System |
| |     Different maneuvers (Rest, Contract, and Valsalva) ( R/C/V) | |
| |     Different distensions (100%, 80%. 60% and 20%) | |

(continued)

| Colpodynamic Imaging | | | |
|---|---|---|---|
| Ultrasound | 2D Video | | Direction of Movement Location of Forces |
| Manometry | Pressure | | Magnitude of Forces |
| | Volume | | |
| Image Analysis | | | |
| Segmentation and Analysis | Segmented Volume | | Parametric variables |
| | Coordinate System | | |
| Other Devices | | | |
| SMART Fitting USTD | Pressure/Stain/Stress sensors | | Direction and location of POP forces during R/C/V |
| USTD | | | |
| CAD Design | Parametric Variables | | Physical USTD |
| Mechanical Testing | Physical USTD or Pessaries | | Force |
| | Test Setup - e.g. Biomechanics model | | Displacement |

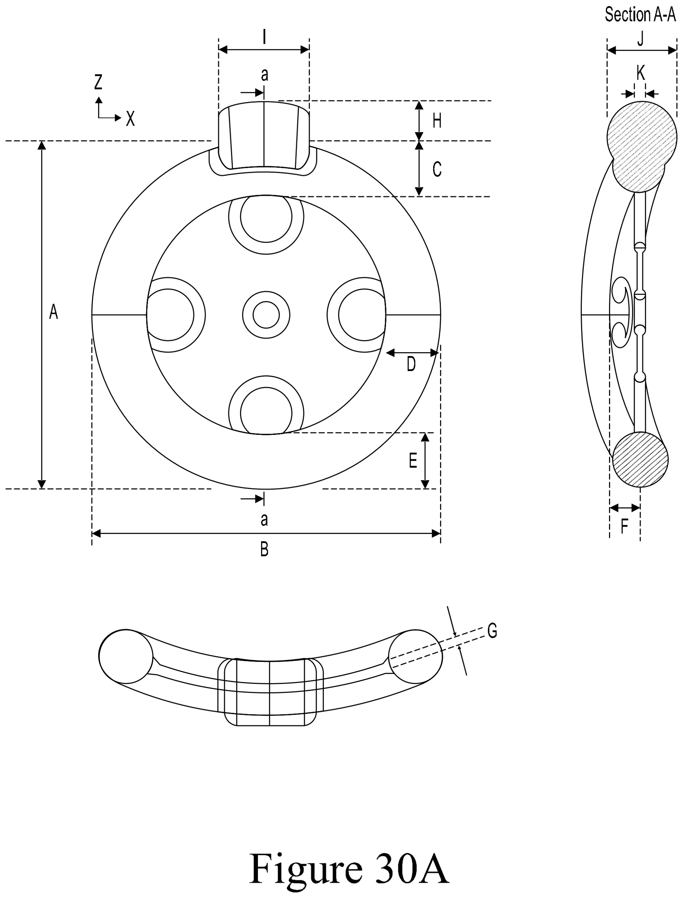

Accordingly, referring to Figure 30A there is depicted a USTD ring pessary device according to embodiments of the invention. The exemplary identified dimensions being presented below in Table 3 with exemplary ranges and the factor(s) leading to the determination of the parametric variable for the patient (user).

Table 3: Exemplary USTD Ring Pessary Device Dimensions

| Dimension | Minimum (mm) | Maximum (mm) | Parametric Variable Determined From |
|---|---|---|---|
| A | 44 | 127 | POP-Q+ (standard method or Measurement Tool) + CDI |
| B | 44 | 127 | |
| C | 5 | 30 | POP force from CDI + mechanical test data + SMART Fitting USTD data |
| D | 5 | 30 | |
| E | 5 | 30 | |
| F | 0 | 70 | Mechanical test data (insertion/removal versus support) + pessary orientation + SMART Fitting USTD |
| G | 1.5 | 30 | Mechanical test data (e.g. 3D force/displacement map) |
| H | 0 | 25 | POP-Q+ (standard method or Measurement Tool) + CDI + 2D Image for urethra location + 2D video and SMART Fitting USTD |
| I | 0 | 45 | POP-Q+ (standard method or Measurement Tool) + CDI |
| J | 0 | 70 | |
| K | -20 | 20 | |

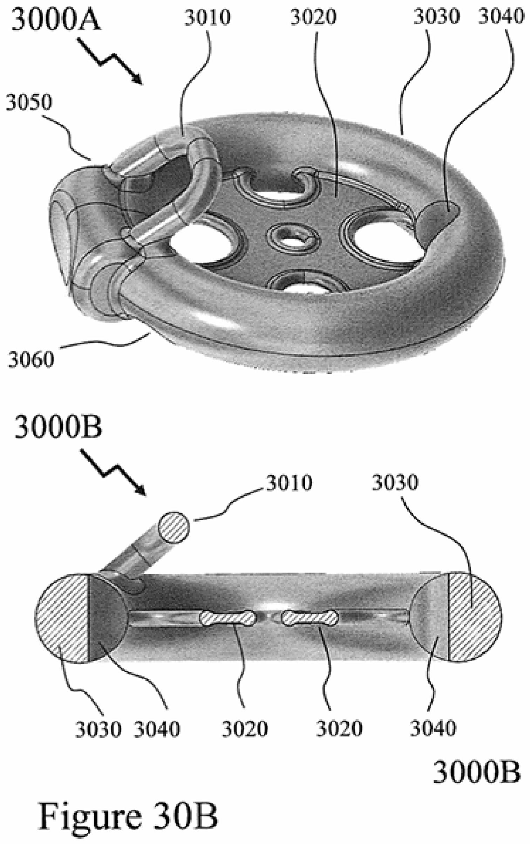

Now referring to Figure 30B there are depicted first and second Ring Pessaries 3000A and 3000B according to embodiments of the invention wherein a "pull-tab" is provided to improve their usability by allowing the pull-tab to be employed in the insertion and/or removal of the first and second Ring Pessaries 3000A and 3000B respectively. First Ring Pessary 3000A is depicted in perspective view and comprises a Pull Tab 3010 which is connected to a Knob 3050 which is disposed at upon the periphery of the Ring 3030. A Support 3020 is disposed on the inside of the Ring 3030. The Ring 3030 having a series of Notches on the inner periphery of the Ring 3030 and lower surface of the Ring 3030, where the upper surface of the Ring 3030 is defined as that above which the Pull Tab 3010 projects, which are depicted as first and second Notches 3040 and 3050 respectively. Second Ring Pessary 3000B depicts a cross-sectional view of a ring pessary without a knob. Accordingly, the second Ring Pessary 3000B comprises a Ring 3030, Pull Tab 3010 and Support 3020 with Notches 3040 on the inner periphery of the Ring 3030. Optionally, the first and second Ring Pessaries 3000A and 3000B may be implemented without the Support 3020.

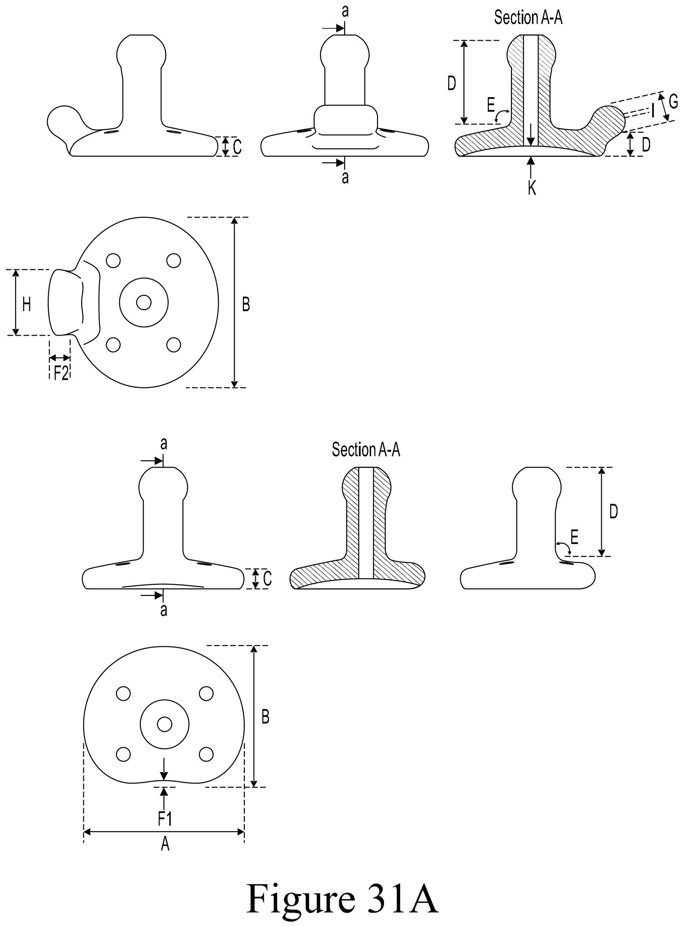

Figure 31A depicts a USTD Gellhorn style pessary device according to embodiments of the invention. The exemplary identified dimensions being presented below in Table 4 with exemplary ranges and the factor(s) leading to the determination of the parametric variable for the patient (user).

Table 4: Exemplary USTD Gellhorn Pessary Device Dimensions

|  | Minimum (mm) | Maximum (mm) | Parametric Variable Determined From |
|---|---|---|---|
| A | 38 | 95 | POP-Q+ (standard method or Measurement Tool) + CDI |
| B | 38 | 95 | |
| C | 3 | 20 | POP force from CDI + mechanical test data + SMART Fitting USTD data |
| D | 25 | 55 | POP-Q+ (standard method or Measurement Tool) + CDI |
| E | 45° | 135° | |
| F1 | 0 | 0.25 * A | POP-Q+ (standard method or Measurement Tool) + CDI + Mechanical test data (insertion/removal versus support) + pessary orientation + SMART Fitting USTD |
| F2 | 0 | 25 | |
| G | 0 | 25 | |
| H | 0 | 45 | |
| I | -65° | 65° | |
| J | 0 | 45 | |
| K | 0 | 20 | 2D video + SMART Fitting USTD |

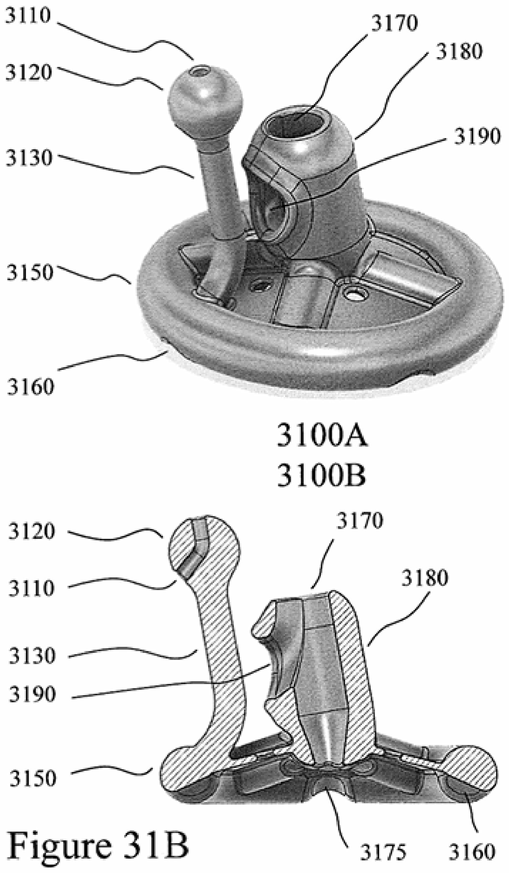

Now referring to Figure 31B there are depicted first and second Images 3100A and 3100B of a Gellhorn-Type Pessary (GT-Pessary) according to an embodiment of the invention wherein a "pull-tab" is provided to improve their usability by allowing the pull-tab to be employed in the insertion and/or removal of the GT-Pessary. Referring to first Image 3100A there is depicted a perspective view of the GT-Pessary comprising a Ring 3150 which has disposed at its centre a Tubular Horn 3180 which is connected to the Ring 3150 by a series of ribs between which are planar supports (these not being indicated by reference numerals for clarity. The Tubular Horn 3180 projects to one side of the Ring 3150 and has an Upper Opening 3170 disposed at a distal end to that connected to the Ring 3150 by the series of ribs. A Side Opening 3190 is also disposed within the sidewall of the Tubular Horn 3180. Also connected to the Ring 3150 is Pull Tab 3130 wherein another distal end of the Pull Tab 3130 to that connected to the Ring 3150 comprises a Grip 3120 within which is a Guide 3110. Disposed upon a lower surface of the Ring 3150 are one or more Notches 3160 which may be omitted within other embodiments of the invention.

Now referring to second Image 3100B in Figure 31B there is depicted a cross-section of the GT-Pessary depicted in first Image 3100A. Accordingly, there is depicted a Lower Opening 3175 disposed towards an end closest to a plane of the Ring 3150 within the Tubular Horn 3180 which is fluidically connected to the Upper Opening 3170 disposed at a second distal end of the Tubular Horn 3180 and the Side Opening 3190. Also evident from the sectioning of the Ring 3150 are the Notches 3160 on the lower surface of the Ring 3150. It is also evident from second Image 3100B that the Guide 3110 runs through the Grip 3120 from a first position to a second position such that the Guide 3110 allows for the insertion of a thread, wire, string or other connection allowing the Grip 3120 to be pulled, and therein the Pull Tab 3130 and Ring 3150, from a location remote from the GT-Pessary. The thread, wire, string or other connection may be attached to the GT-Pessary via the Guide 3110 prior to its insertion however it would be evident that the Grip 3120 and Pull Tab 3130 may be used without any attachment via the Guide 3110.

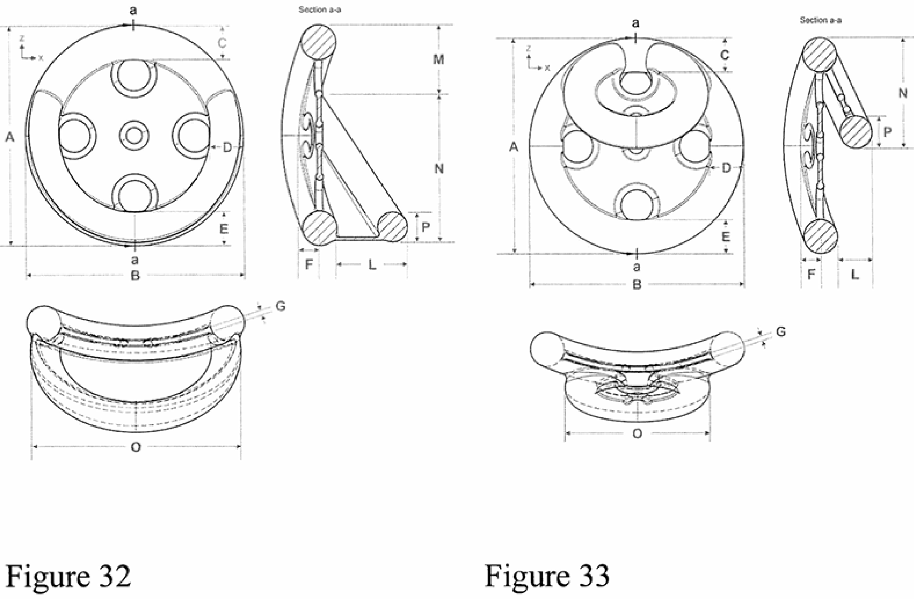

Figures 32 and 33 depict a USTD traditional Marland style ring pessary devices and a USTD double ring Marland style ring pessary device according to embodiments of the invention. The exemplary identified dimensions being presented below in Table 5 with exemplary ranges and the factor(s) leading to the determination of the parametric variable for the patient (user).

Table 5: Exemplary USTD Marland Pessary Device Dimensions

|  | Minimum (mm) | Maximum (mm) | Parametric Variable Determined From |
|---|---|---|---|
| A | 44 | 127 | POP-Q+ (standard method or Measurement Tool) + CDI |
| B | 44 | 127 | |

(continued)

|   | Minimum (mm) | Maximum (mm) | Parametric Variable Determined From |
|---|---|---|---|
| C | 5 | 30 | POP force from CDI + mechanical test data + SMART Fitting USTD |
| D | 5 | 30 | |
| E | 5 | 30 | |
| F | 0 | 70 | POP-Q+ (standard method or Measurement Tool) + Mechanical test data (insertion/removal versus support) + pessary orientation + SMART Fitting USTD |
| G | 1.5 | 30 | Mechanical test data (3D force/displacement map) |
| L | 10 | 85 | CDI + SMART Fitting USTD |
| M | 0 | 127 | CDI |
| N | 0 | 127 | |
| O | 0 | 127 | |
| P | 5 | 30 | POP force from CDI + mechanical test data + SMART Fitting USTD |

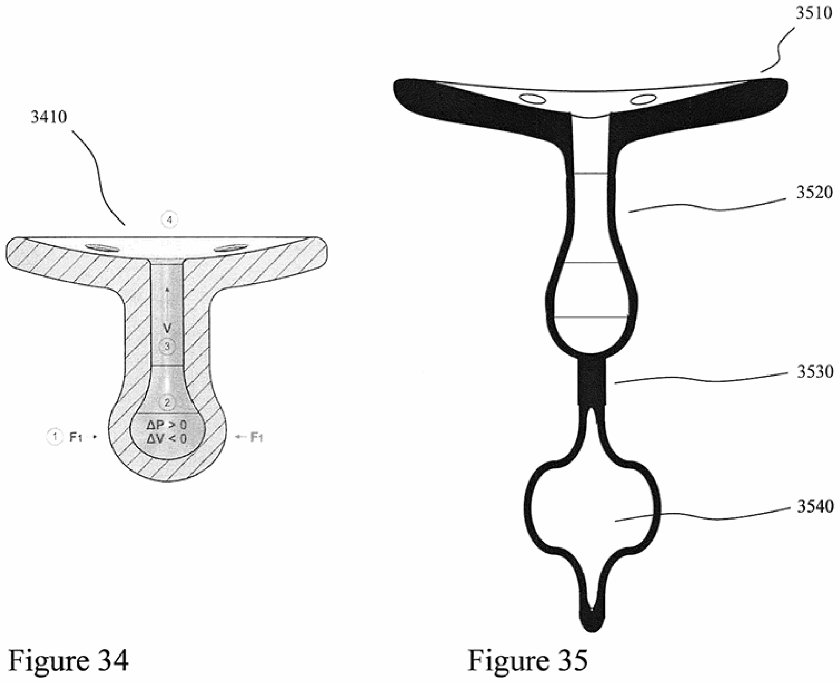

Figure 34 depicts removal of a prior art Gellhorn 3410 pessary device. Accordingly, the patient or clinician applies a force F1 to the air pocket located within the sealed end of the stem of the Gellhorn 3410. This force compresses the air pocket reducing its volume and increasing air pressure within the stem and air pocket. This causes the air to flow out the end of the stem into the cavity between the cap of the Gellhorn 3410 and patient's body. As this pressure increases it breaks the seal of the Gellhorn 3410 cap to the patient's body allowing the Gellhorn 3410 to be removed.

However, to remove the Gellhorn as depicted in Figure 34 the patient's or clinician's fingers must penetrate sufficient deep within the patient's vagina to allow contact with the end of the stem and apply pressure. However, referring to Figure 35 there is depicted a USTD Gellhorn style pessary device according to embodiments of the invention with pull tab and stem for ease of removal which reduces the necessary penetration to facilitate removal. As depicted the Cap 3510 and Stem 3520 may be configured similar to a prior art Gellhorn. However, the Stem 3520 is now connected at its distal end to the Cap 3510 to a Pull Tab 3530 and Finger Pull 3540. Accordingly, the patient or clinician now simply has to reach the Finger Pull 3540 and insert the tip of their finger inside before starting to pull. As they pull the Finger Pull 3540 this will extend to its elastic limit (if elastic) or not deform wherein, at either point, the Finger Pull 3540 pulls on the Stem 3520 via the Pull Tab 3530 wherein the Stem 3520 distends and narrows thereby increasing the pressure within the Stem 3510 and between the Cap 3510 and patient's body. Alternatively, the Finger Pull 3540 now allows sufficient force to be applied to the Cap 3510 such that the suction between it and the patient's body is broken.

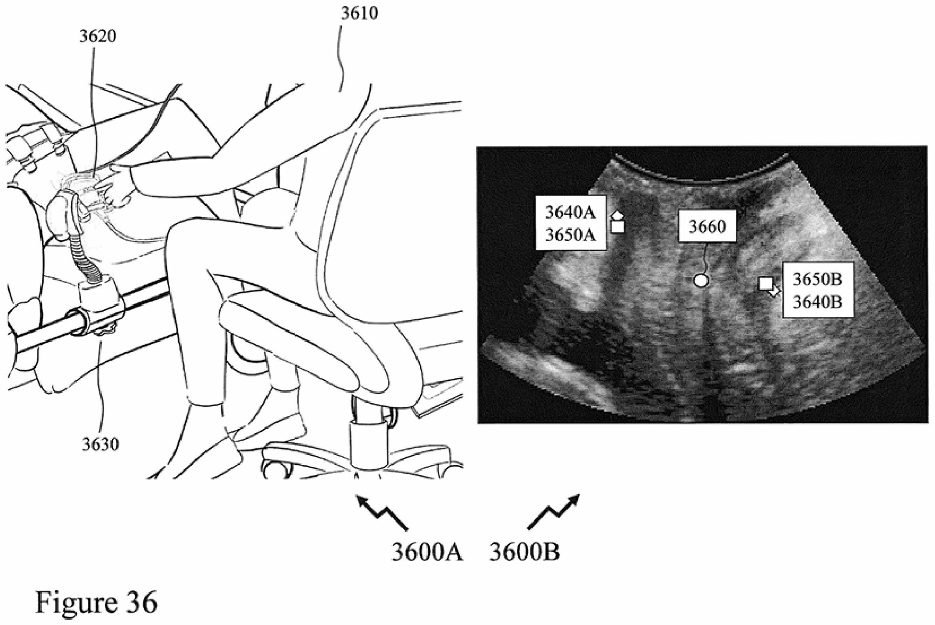

Within the embodiments of the invention described above one potential issue is the difficulty in establishing the appropriate probe placement with respect to location, angle, and pressure in order to establish a good ultrasound image. Accordingly, the inventors have established a system / methodology wherein ultrasound images are processed to detect the patient's pubis symphysis and establish the patient's anorectal angle in order to provide feedback, either to a clinician or a robotic system, as to how to adjust placement of the probe. Accordingly, as depicted in first Image 3600A in Figure 36 a Clinician 3640 positions a Probe 3620 against the patient to establish ultrasound images. With the Probe 3620 supported upon a flexible arm from a Support 3630 images are acquired and processed. A resulting processed image is depicted in second Image 3600B wherein the software analysing the ultrasound image(s) establishes left positions (indicated by first and second Left Markers 3640A and 3650A respectively) and right positions (indicated by first and second Right Markers 3640B and 3650B respectively). Using these in pairs, e.g. first Left Marker 3640A and first Right Marker 3640B or second Left Marker 3650A and second Right marker 3650B, the system can then determine the patient's anorectal angle as well as the locations of the patient's pubis symphysis. Based upon these a target location for the centre of the probe can be established, indicated as Probe Marker 3660

It would be evident that the probe may be mounted to a robotic system such that the position of the probe head is automated wherein pressure sensor(s) allow the contact pressure of the probe head against the patient's body to be monitored / controlled / set. Further, by processing the ultrasound images the robotic system can establish the locations of the patient's pubis symphysis as well as the Probe Marker 3660. Subsequently, the robotic system may adjust the probe position such that the Probe Marker 3660 is central within the acquired images by moving the probe under control of a controller of the robotic system. Once positioned the automated system can proceed to automatically acquire the required ultrasound images, e.g. 2D or 3D, as well as direct the patient to undertake specific movements, e.g. rest, contract, Valsalva.

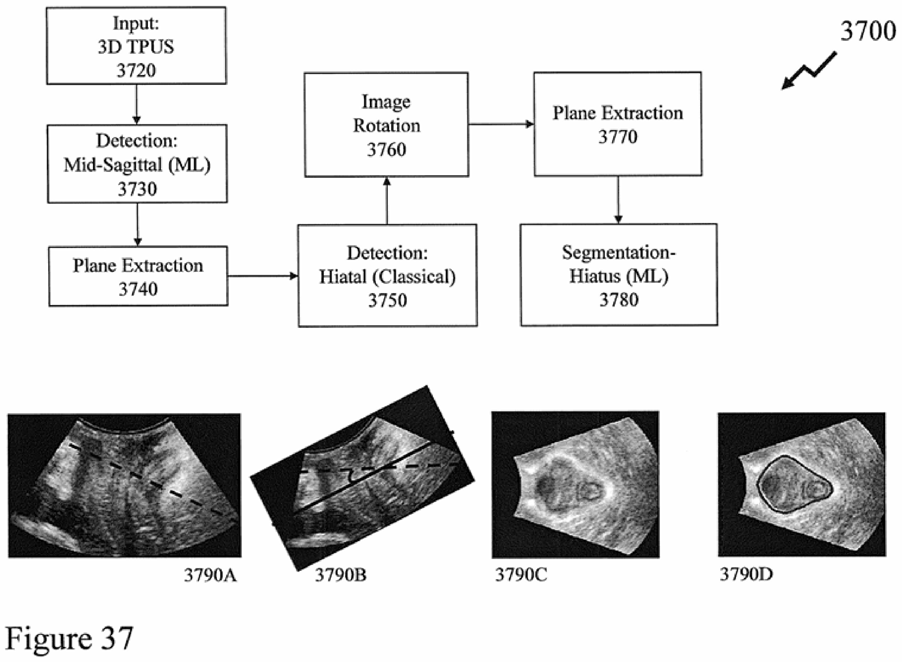

Now referring to Figure 37 these concepts are extended within an exemplary Flow 3700 depicts an exemplary process flow for the automated analysis of hiatus dimensions based upon three-dimensional (3D) transperineal ultrasound (TPUS) measurements. Accordingly, as depicted the Flow 3700 comprises first to seventh steps 3720 to 3780 respectively, comprising:

- First step 3720 wherein one or more 3D TPUS images are acquired;
- Second step 3730 wherein the mid-sagittal line (ML) is detected within the acquired 3D TPUS image(s);
- Third step 3740 wherein a plane along the ML is extracted;
- Fourth step 3750 wherein a Hiatal (classical) is detected;
- Fifth step 3760 wherein the image is rotated;
- Sixth step 3770 wherein a second plane is extracted; and
- Seventh step 3780 the plane is segmented to establish the Hiatus (ML).

Also depicted in Figure 37 are first to fourth Images 3790A to 3790D comprising:

- First Image 3790A depicting identification of the mid-sagittal (ML);
- Second Image 3790B depicts rotation of the image;
- Third Image 3790C wherein the second plane is extracted; and
- Fourth Image 3790D wherein the Hiatus (ML) is segmented and identified.

As outlined above ultrasound imaging of the pelvic floor can be obtained transvaginally or transperineally, with the latter being more widespread at this point, mainly due to its non-invasive nature and the advantage of reducing tissue distortion. 3D and 4D transperineal ultrasound images are often obtained in the supine position, using a curvilinear volumetric probe, for example. To acquire a transperineal 3D image of the pelvic floor, the probe is placed vertically on the central perineum and pubic symphysis, such that in the mid-sagittal view, the following anatomical structures are visualized ventrally to dorsally, namely the symphysis pubis, urethra, bladder neck, vagina, and anorectal junction. The levator ani muscles and genital hiatus can then be visualized on the plane of minimal hiatal dimensions (PMHD), defined as the plane where the distance between the posterior aspect of the pubic symphysis and the anorectal angle (i.e. anterior border of the pubovisceral muscle) is minimal.

However, the PMHD does not fall on any of the orthogonal ultrasound image planes. Therefore, to visualize the PMHD, the transverse plane needs to be rotated so that it goes through the inferior border of the pubic symphysis and the apex of the anorectal angle. This is performed by first, identifying the pubic symphysis and the apex of the anorectal angle landmarks on the midsagittal plane, and then rotating the transverse image plane clockwise until both landmarks lie along a straight line on the oblique image plane.

Multiple biometric indices are described for the PMHD, including levator ani muscle thickness, the anteroposterior and lateral hiatal diameter, and the pelvic floor hiatus inner area. Within the prior art significant correlations between levator hiatus area and pelvic organ descent have been reported. Hiatal enlargement of $\geq 25$ cm$^2$ on Valsalva is defined as an abnormal distensibility (or "ballooning") and is strongly associated with detecting prolapse and symptoms of prolapse. In addition, the shape of the levator ani muscle on the PMHD is associated with increased pelvic floor symptoms.

As the PMHD is an oblique angle, obtaining these measurements is challenging and require manual manipulation of the image to visualize the PMHD. While several techniques have been developed to automate the segmentation of the PMHD to obtain its area, identification of the PMHD is performed manually. Accordingly, the inventors have established a novel algorithm to identify the PMHD automatically wherein the inventive fully automated pipeline also obtains the area of the PMHD. Further, the pipeline(s) according to embodiments of the invention exploit novel methods to robustly estimate the locations of the pubic synthesis (PS) and the anorectal angle (ARA) from the input 3D transperineal ultrasound volume, despite all the challenges mentioned above. The PMHD can then be extracted based on the detected landmarks.

Since clinicians are used to working with pelvic floor ultrasound images in 2D sagittal view to detect PS and ARA, the inventors developed their inventive landmark detection algorithm accordingly, in order to incorporate clinicians' expertise and experience. The pipeline, in summary, exploits a 2D landmark detection algorithm for a given mid-sagittal plane to estimate the locations of PS and ARA. The 2D landmark detection is performed on all the mid-sagittal planes within the volume of interest and the 3D location is then identified via a neighborhood consistency check. The inventors automatic landmark detection algorithm comprises the steps of pre-processing, pubic symphysis detection, anorectal angle detection, and a consistency check.

With respect to pre-processing the generally low quality of pelvic floor ultrasound image, due to image noise, low-contrast, and blurry boundaries, significantly reduces the effectiveness of landmark identification. Therefore, image pre-processing prior to landmark detection is typically required so that the pelvic floor ultrasound image quality can be improved by enhancing the contrast and smoothing the image while preserving the major edges. To achieve this, within an embodiment of the invention, the inventors initially apply an unsharp filter, for contrast enhancement, followed by the fast weighted median filter, for edge preserved smoothing. The reason for using the weighted median filter is due to its power to remove large outliers while incorporating local intensity distribution for edge preservation. From the pre-processed midsagittal image, the structural edge information can then be extracted as an edge map E, using the Canny edge detector.

In order to detect the pubic symphysis (PS) efficiently and reliably, the inventors have focused on two important features. First, assuming the 3D transperineal pelvic ultrasound image is acquired following the standard convention, the PS should always appear roughly on the top left corner of the midsagittal image. Second, since the PS is a cartilaginous joint and the urethra is soft tissue, the PS should always appear to be a higher intensity region on the left side of the urethra. The distinctly different ultrasound properties of the PS and the urethra results in a large intensity change between these two landmarks, which creates an edge, allowing delineation of the boundary of the PS, as shown in Figure 39.

Utilizing the high localization of the PS from conventional transperineal image acquisition, a probability map can be created to provide a strong prior information for PS detection. Within an embodiment of the invention this probability map for PS was created as an average atlas of hand labeled PS from the inventors' data set, convolved with a Gaussian kernel. The edge of urethra, adjacent to the PS, is then detected using a negative gradient flow defined as decreasing intensity from left to right as depicted for example in Figure 40. Mathematically, the negative gradient map is computed as the local pixel differences, following the convolution given in Equation (1) where $I$ denotes the pre-processed mid-sagittal image and $h_{ps}$ is the PS detection kernel as defined by Equation (2).

$$h_{ps} = \begin{bmatrix} 0 & 0 & 0 \\ 0 & 1 & -0.5 \\ 0 & 0 & -0.5 \end{bmatrix} \tag{2}$$

From the previously extracted edge map E, the edges with a negative gradient flow are then identified using Equation (3) where $\cdot$ is an element-wise product operator and $\lfloor \quad \rfloor_0$ is a threshold operator, such that the positive product will be assigned label 1, and the negative product will be assigned label 0. Knowing that the urethra edge is within the negative edge map, the urethra edge can then be detected by selecting a segment within the negative edge map, which maximizes the following discrete cost function given by Equations (4) and (5) where $e_i \in E^-$ is a disjoint edge segment belonging to the negative edge map, and $P(x)$ is the probability map of PS. The edge segment yielding the highest cost will be selected as the urethra edge. Finally, from the urethra edge, we now select the point with the highest curvature as our initial landmark for PS.

$$E^- = \lfloor E \cdot G^- \rfloor_0 \tag{3}$$

$$E_{ps} = \arg\max C_{ps}(e_i) \tag{4}$$

$$C_{ps}(e_i) = \sum_x^{e_i} e_i(x) \cdot P(x) \tag{5}$$

Subsequently, anorectal angle detection is performed. Similar to the PS identification, we mainly focus on two features for the detection of the anorectal angle (ARA). First, as shown in Figure 39, the ARA is located in the lower right position relative to the PS due to anatomy and the image acquisition technique. Second, the anterior border of the pubovisceral muscle, which is used to identify the ARA, is hyperechoic, compared to the rectum. While the rectal ampulla creates a bright echo within the rectum, the rectum lumen is often consistently darker than the anterior border of the pubovisceral muscle, creating a distinct edge between the pubovisceral muscle and the rectum, which forms the ARA. Hence, the inventors detect the edge of the anorectal junction, adjacent to the pubovisceral muscle, using a positive gradient flow defined as increasing intensity from left to right and the positive gradient map can be extracted as given by Equations (6) and (7) where $h_{ara}$ is a local difference kernel to estimate the intensity differences from left to right. From the edge map E, we create the positive edge map $E^+$ as given by Equation (8) such that the edges with positive gradient flow will be assigned as 1, and the rest of the edges as 0.

$$G^+ = I * h_{ara} \tag{6}$$

$$h_{ara} = \begin{bmatrix} 0 & 0 & 0 \\ 0 & 1 & -0.5 \\ 0 & 0 & -0.5 \end{bmatrix} \tag{7}$$

$$E^+ = \lfloor E \cdot G^* \rfloor_0 \tag{8}$$

To further emphasize the intensity changes across the rectum edge, we use another convolutional kernel $h_{normal}$ to estimate the intensity differences along the normal of the ARA edge as given by Equation (9). We then detect the ARA edge $E_{ara}$ by selecting an edge segment within the positive edge map, which maximizes the following discrete cost function, $C_{ara}(e_i)$, as given by Equation (10) where $e_i \in E^+$ is a disjoint edge segment belonging to the positive edge map and $D(x)$ is a Euclidian distance map, measuring the distance between every pixel to the PS. $\alpha$ is a joint parameter to balance the distance term and the intensity term in the cost function.

$$h_{normal} = \begin{bmatrix} -1 & 0 & 0 & 0 & 0 \\ 0 & -1 & 0 & 0 & 0 \\ 0 & 0 & 0 & 0 & 0 \\ 0 & 0 & 0 & 1 & 0 \\ 0 & 0 & 0 & 0 & 1 \end{bmatrix} \tag{9}$$

$$C_{ara}(e_i) = \sum_x e_i(x) \cdot (I(x) * h_{normal}) + \alpha D(x) \tag{10}$$

$$E_{ara} = arg_{ei} max\ C_{ara}(e_i) \tag{11}$$

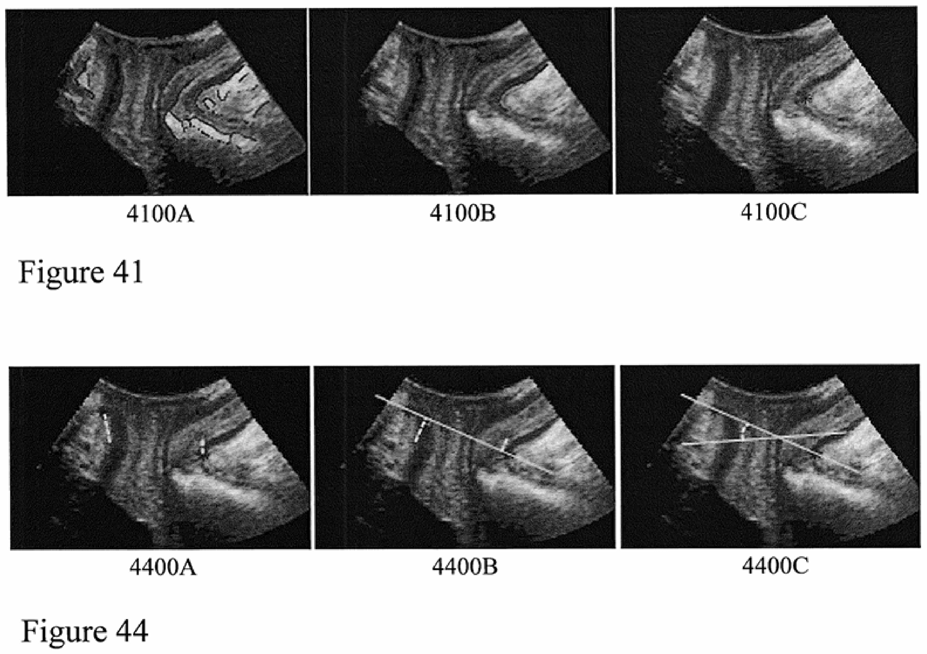

For $I(x) \in [0,1]$ with a resolution of 150 250 then $\alpha$ was chosen as a constant 0.01 for all test cases. From the extracted edge, $E_{ara}$, we now select the point which is closest to the PS as our landmark for the ARA. An exemplary illustration of the aforementioned positive and negative edge maps, detected urethra and rectum edges, as well as the extracted PS and ARA landmarks, are shown in Figure 41 wherein first Image 4100A depicts the edge maps, second Image 4100B the urethra edge, and third Image 4100C the extracted PS and ARA landmarks.

Within an exemplary embodiment of the invention the automated process employed exploits a consistency check. The described landmark detection is based on an input of the optimal mid-sagittal 2D image plane. To improve the automation and avoid the need of manually selecting the optimal image plane, the inventors have established consistency check method to integrate their methodology within a 3D system.

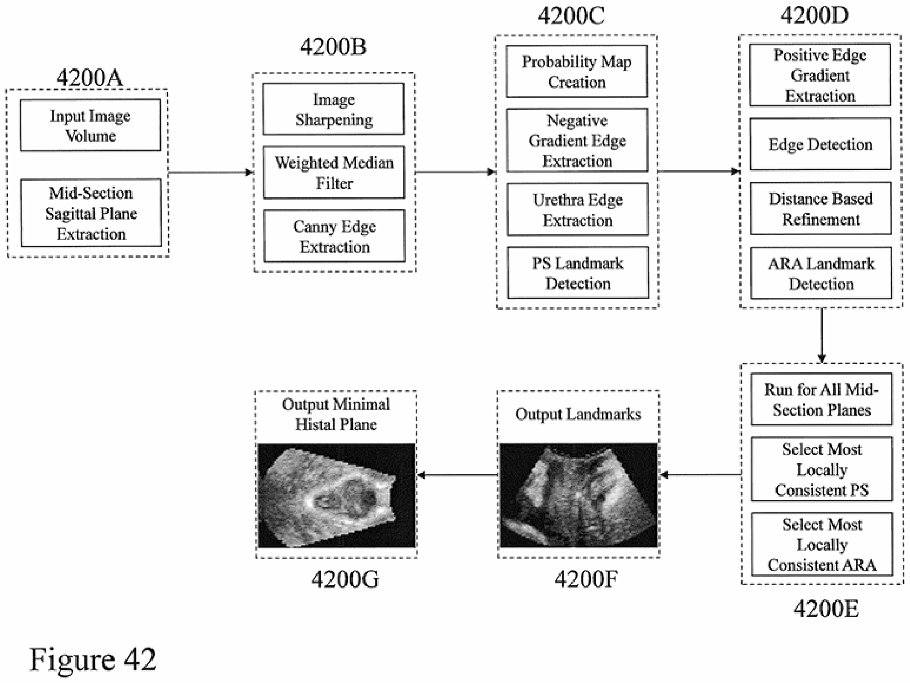

The optimal mid-sagittal plane is defined as a sagittal image plane, which captures all the important anatomies, including the PS, urethra, vagina, rectum, and ARA. Assuming centered image acquisition. We know the optimal mid-sagittal plane is somewhere in the middle of the ultrasound volume. Hence, the proposed 2D landmark detection algorithm can be used for a range of para-sagittal slides in the middle section of the ultrasound volume to extract the most locally consistent landmark positions as the final output. When the final positions of the PS and the ARA landmarks are identified, the rotation angle for the PMHD extraction can be calculated. The PMHD is on an oblique image plane, which contains the detected landmarks. The PMHD extraction procedure is identical to the manual approach explained previously. The overall workflow of our automatic landmark detection algorithm and PMHD extraction method is described in Figure 42.

As depicted in Figure 42 the exemplary process is described and depicted as first to seventh steps 4200A to 4200G respectively. As depicted these comprise:

- First Step 4200A comprising, for example:

    ◦ Input Image Volume; and
    ◦ Mid-Section Sagittal Plane Extraction.

- Second Step 4200B comprising, for example:

    ◦ Image Sharpening;
    ◦ Weighted Median Filter; and
    ◦ Canny Edge Detection.

- Third Step 4200C comprising, for example:

    ◦ Probability map creation;
    ◦ Negative gradient edge extraction;
    ◦ Urethra edge extraction; and

◦ PS landmark detection.

- Fourth Step 4200D comprising, for example:

    ◦ Positive edge gradient extraction;
    ◦ Edge detection;
    ◦ Distance based refinement; and
    ◦ ARA landmark detection.

- Fifth Step 4200E comprising, for example:

    ◦ Processing all mid-section planes;
    ◦ Selecting most locally consistent PS; and
    ◦ Selecting most locally consistent ARA.

- Sixth Step 4200F wherein the landmarks are output; and
- Seventh Step 4200G wherein the minimal histal plane is output.

In order to validate the proposed method the inventors performed two sections of validation. In the first section, the performance of the landmark detection algorithm according to an embodiment of the invention was validated by comparing the predicted landmarks and the groundtruth landmarks, which were manually labeled by experts. In the second section, as a demonstration of the complete clinical workflow, we extracted the PMHD based on the predicted landmarks and performed automatic segmentation of the levator hiatus using U-Net (convolutional neural network for biomedical image segmentation at the University of Freiburg). The resulting automatic segmentations were compared with the manual segmentations of the PMHD, which were also manually extracted by the experts.

The dataset included 108 3D transperineal ultrasound images of the pelvic floor obtained using a GE Voluson-I ultrasound system with RAB4-8 probe. For each image volume, the PMHD was manually extracted by the experts and the levator hiatal area manually segmented to create the groundtruth dataset. Among the total images in the dataset, 35 images were randomly selected as training set and the rest 73 images were used as the test dataset. To train the U-Net for the levator hiatus segmentation, the PMHD of the 35 training images were used as inputs, and the corresponding segmentations were used as labels. Although our landmark detection algorithm doesn't require training sets, all test results were obtained using the testing dataset for consistency purposes.

The landmark and PMHD extraction algorithms were implemented in MATLAB R2018b and the U-Net was implemented with Keras (v2.2.4) using Python (v.3.6.10). An Adam solver was used with a fixed learning rate of 0:001, and a total number of 20 epochs were used with 300 iteration steps per epoch.

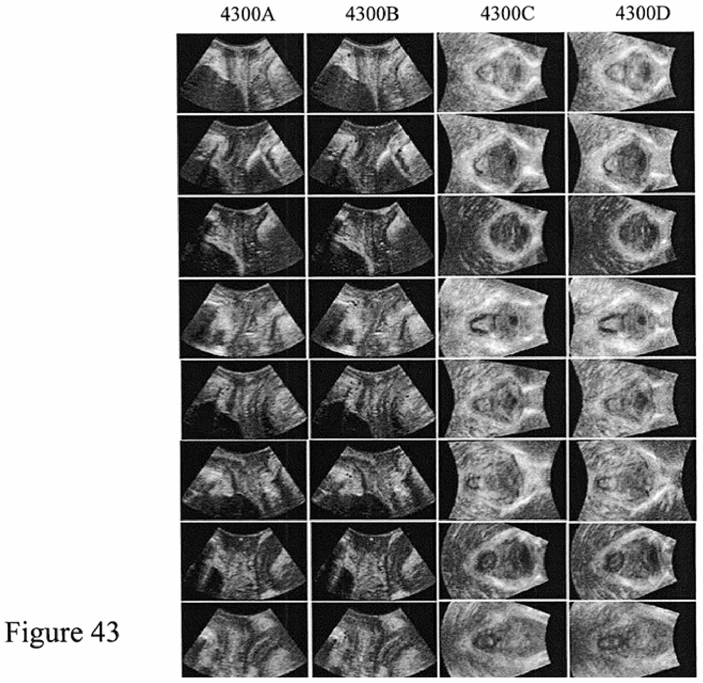

The effectiveness of the presented automatic landmark detection algorithm was evaluated against groundtruth manual segmentation. Compared to the groundtruth for both the landmarks and PMHD , the inventive automatic algorithm according to an embodiment of the invention produced highly accurate results on female pelvic ultrasound images with various pathologies and physiologies. As an example, the qualitative comparisons are presented in Figure 43 for eight patients. The manually selected midsagittal plane is displayed in first Column 4300A, the automatically detected landmarks and the groundtruth landmarks in second Column 4300B, the automatically extracted PMHD in third Column 4300C, and the groundtruth PMHD in fourth Column 4300D.

As shown in the automatically detected and manually identified landmarks in second Column 4300B in Figure 43, the inventive algorithm is able to highly accurately identify the landmarks. In addition, the algorithm performed robustly for patients with different anatomy. Even for images with low contrast, blurry edges, and slightly shifted orientations, the presented algorithm was still able to estimate the locations of the landmarks, which were similar to those identified manually. The accuracy of the algorithm is also reflected by the automatically extracted PMHD, as shown in third Column 4300C which are highly similar to the groundtruth images in fourth Column 4300D based on visual inspection. Due to the low quality of ultrasound image, sometimes it could be unclear, even for the expert clinicians, to accurately identify the exact locations of PS and ARA. Hence, the placements of these landmarks can be somewhat subjective. Nevertheless, since the goal of the landmark identification is to extract the PMHD, as long as the landmarks are placed on or near the PMHD, displacements along the direction of PMHD would not affect the accuracy of PMHD extraction.

Quantitatively, three metrics were used to evaluate the accuracy of the landmarks and their impacts on the PMHD extraction. They include the absolute landmark distance, the effective landmark distance, and the plane angle difference. The absolute landmark distance was calculated as the Euclidean distance between the predicted landmarks and the corresponding groundtruth landmarks, as illustrated in first Image 4400A in Figure 44. The effective landmark distance is calculated as the perpendicular distance between the predicted landmark and the groundtruth PMHD, as shown in second Image 4400B in Figure 44. The plane angle difference is estimated as the angular difference between the predicted PMHD and the groundtruth PMHD, as depicted in third Image 4400C in Figure 44.

Based on the proposed metrics, we evaluated the performance of the landmark detection algorithm using 73 test images. As shown in Table 6, the average effective distance for PS was 5.03 pixels and the average effective distance for ARA was 4.93 pixels. Since the isotropic spatial resolution for pixels on sagittal plane is 0.5 mm by 0.5 mm, the PS effective distance and the ARA effective distance were roughly 2:5mm on average. The average PS absolute distance and the average ARA absolute distance were 3.05mm and 4.85 mm, respectively. Since the presented algorithm is the first automatic landmark detection algorithm for female pelvic floor 3D transperineal ultrasound images, these outcomes could not be compared with any previous study. Nevertheless, in similar studies, such as those on anatomical landmark detection in prostate and abdominal ultrasound images, landmark errors of 5mm or less were considered to be highly accurate. The plane angle difference was less than 5° on average. Such a small rotational difference combined with the small effective landmark distances led to the accurate PMHD extraction in the ultrasound images as reflected in third Image 4400C in Figure 4. The relatively small median and the relatively large standard deviation indicate the presence of the outlier. Since the ultrasound images generally have low qualities and are often corrupted by various image artifacts, our landmark detection algorithm may fail when the boundaries of urethra and rectum lumen are not visible. Improvements in terms of robustness for those rare cases can be anticipated based upon developments to the algorithm and techniques.

Table 6: PS and ARA Distances

| Total (n=73) | PS Effective Distance (mm) | ARA Effective Distance (mm) | PS Absolute Distance (Pixel) | ARA Absolute Distance (Pixel) | Plane Angle Difference (Degree) |
|---|---|---|---|---|---|
| Average | 5.03 | 4.93 | 9.39 | 9.7 | 4.71 |
| Median | 3.63 | 3.71 | 6.09 | 3.62 | 4.11 |
| Standard Deviation | 4.41 | 4.34 | 5.22 | 3.32 | 4.45 |

To quantitatively assess the accuracy of the automatically extracted PMHD and to demonstrate the algorithm's value in clinical applications, the inventors also implemented a deep learning segmentation algorithm, to automatically extract the levator hiatus. Since the dimensions and area of the hiatus are of clinical significance, such an automatic segmentation algorithm would reduce the workload and enable computer assisted analysis.

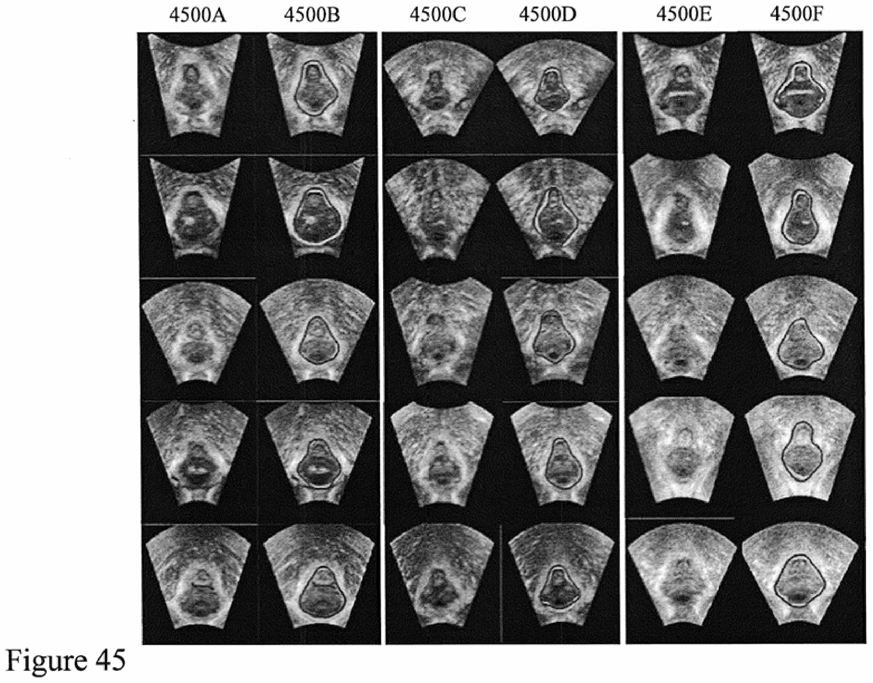

To illustrate the performance of our PMHD extraction algorithm, the extracted PMHD and the segmentation results are shown in Figure 45 for 15 patients where the dark contours represent the automatic segmentation and the light contours represent the groundtruth. First, third and fifth Columns 4500A, 4500C and 4500E depicted the planes whilst second, fourth, and sixth Columns 4500B, 4500D and 4500F depict the contours from which it is evident that the inventive automatic algorithm was able to obtain highly accurate segmentations compared to the groundtruth as the automatic segmentations were able to capture the major shapes of the hiatus regions, which also implies accurate PMHD extraction as it is a necessary step for accurate hiatus segmentation. There are some small deviations near the segmentation boundaries, which could be caused by the boundary ambiguity due to the blurry anatomical structures on ultrasound images and user variability.

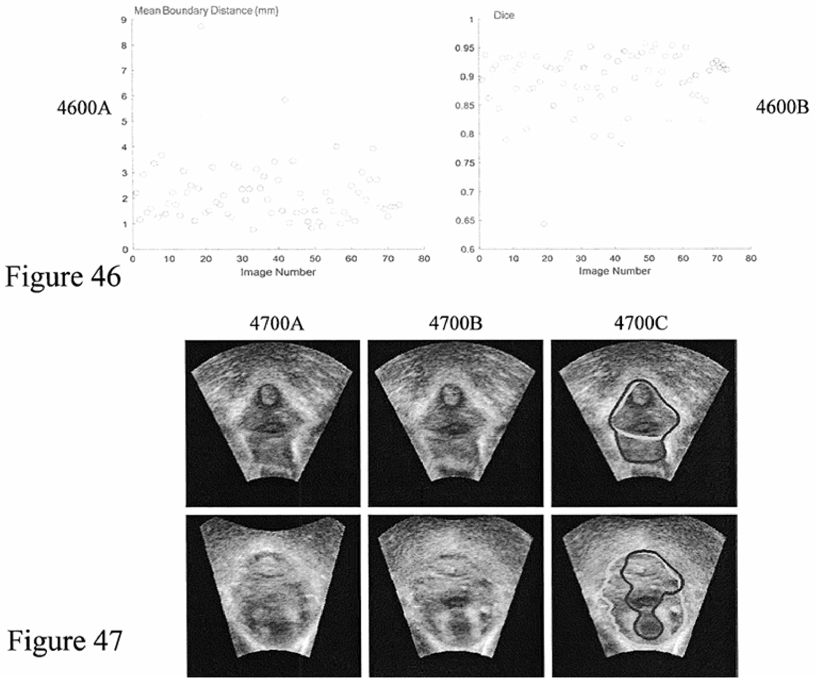

To quantitatively assess the segmentations, we used mean boundary distance (MBD), the Sørensen-Dice Coefficient (DICE) coefficient, and levator hiata area to evaluate the overlap between our segmentations and the groundtruth. As shown in Table 7, the algorithm was able

to perform fairly accurate segmentation with an average DICE score of 0:89, an average MBD of 2:15 mm, and an average area difference of 1:75 cm$^2$. Since the average area of the levator hiatus in the test dataset was 65 cm$^2$, a small deviation of 1:75 cm$^2$ would not have a significant impact clinically, especially due to the potential issues with low image quality. Figure 46 depicts scatter lots of the distributions of the DICE and MBD of our automated segmentations compared to the groundtruth. For majority of the test cases, the DICE and MBD were distributed around the averages of 0:89 and 2:15 mm, respectively with two outliers indicated by the red dash lines.

Figure 47 depicts the extracted PMHD (first Column 4700A), the groundtruth PMHD (second Column 4700B), and the segmentation (third Column 4700C) for the two outlier cases. It was observed that the algorithm was able to produce reasonable PMHD compared to the groundtruth. Nevertheless, the segmentations failed due to unordinary patient anatomy and weakened muscle boundaries from PMHD extraction error. It is anticipated that these issues can be addressed by expanding the training dataset and incorporating subjects with more anatomical variations.

Accordingly, the inventors have presented an automatic approach to identify landmarks from female pelvic floor 3D ultrasound images and also to extract the plane of minimal hiatal dimensions. Given the appropriate parameters, the presented algorithm can systematically estimate the locations of the pubic symphysis and anorectal angle. The inventors have utilized the fact that PS and ARA are relatively close to the urethra and rectum. By identifying the urethra and rectum edges, the locations of the PS and ARA landmarks were estimate robustly, despite the large variations of patient anatomy and ultrasound image qualities. For automation purposes, whilst the exemplary methodology does not explicitly search for the mid-sagittal slice, the landmark detection algorithm was executed for all midsection sagittal slices, where the PS and ARA landmarks were computed for every sagittal slice and the most locally consistent landmark positions within consecutive slices were chosen as the final output. For validation, the automatically extracted landmarks was compared with the manually extracted groundtruth and we focused our evaluation on the landmarks localization impact on identifying the PMHD. Experiments using 73 test images showed that, on average, this algorithm could accurately identify the landmarks with less than 5mm of absolute distance, less than 2:5mm of effective distance, and less than 5° of rotation error. To further evaluate the validity of the extracted PMHD and the usefulness of the proposed automated algorithm the inventors performed an automated segmentation of the levator hiatus and compared the results to the manually segmented levator hiatus from the PMHD groundtruth dataset. The automated segmentations were highly similar to the manual segmentation, with average DICE of 0:89 and average MBD of 2:15 mm. Consultation with clinicians indicates that the accuracy of this level is acceptable and the inventive automated PMHD extraction algorithm can replace the tedious manual work.

Within embodiments of the invention a balloon may be formed from medical grade silicone comprising an initial sticky soft silicone, e.g. 20 durometer, with a micro-layer (e.g. spray coated for example) of high durometer medical grade silicone, for example 70-90 durometer, to create a "slippery" surface. Alternatively, the balloon may be formed from a single later of high durometer medical grade silicone or a medium durometer medical grade silicone.

Within embodiments of the invention discrete pressure monitoring relative to the flow of fluid into a manometry balloon has been described and depicted with respect to Figures 7 to 23 respectively. However, it would be evident that within other embodiments of the invention the pressure may be monitored within the balloon based upon a sequential pump - measure sequence wherein fluid is pumped into the balloon, the pump stops, and the pressure is monitored to remove pressure artifacts of the pumping process. Alternatively, the pressure may be continuously monitored during the pumping process and processed to extract the requisite pressure information.

Within Figures 1, 3 and 5A an AI Engine is referenced. However, in other embodiments of the invention an additional stage may be introduced wherein a clinician, physician, or surgeon (hereinafter referred to as an expert) may review the design and provide feedback prior to production of the USTD. For example, in some embodiments, a design that incorporates patient or expert profile information and, optionally, historical case information, is presented to the expert for evaluation. The design is presented to the expert in a manner that permits the expert to evaluate the position and function of the device using 3D CAD models, as well as permitting the expert to observe and test performance parameters using computer simulation. The expert may access the system remotely via a software application in execution upon their computer system coupled to one or more remote servers and/or computer systems. The expert can select or recommend design changes to the AI Engine directly or through an interface of a USTD software system implementing embodiments of the invention. The software system together with the CBES and/or AI Engine can accordingly incorporate patient- or expert-specific needs based on the wisdom and experience of the expert and the new design can be submitted and tested, and if desired, reevaluated prior to selecting a final design prior to release to production.

Accordingly, upon expert review the AI Engine may perform revisions to the design of the USTD, cup, plug etc. In this case, a new virtual 3D model can be produced, tested, and reevaluated. In some embodiments, only following approval by the expert or through a collaborative approval, such as the expert and/or a designer and/or manufacturing authority can a design be released for manufacture. In some embodiments of the invention the USTD may be designed in conjunction with either an intended surgical procedure or with a recommendation that a surgical procedure be performed. In other embodiments of the invention the design process and/or a clinical evaluation may determine that an area or areas of the user should be surgically manipulated, e.g. a re-alignment, sectioning, re-profiling, or morphological adjustment should be performed. Optionally, the design of the USTD may require that a portion or portions of the USTD are attached to the user's body through a surgical procedure in order to ensure appropriate placement and/or retention of the USTD.

Within embodiments of the invention in a measurement and characterisation stage of determining the characteristics of the user they may be asked to wear a device which provides additional data relating to the user in addition to that identified supra. For example, the user may be asked to wear a device which provides for monitoring vaginal exercises, e.g. a Kegel exercise device, as well as providing for other parameters including, but not limited to, labial blood flow etc. for indications of whether their symptoms change according to initial stages of sexual arousal, during vaginal exercise etc.

Optionally, extended monitoring of the user's vulvar and/or vaginal temperature in conjunction with other biometric data, including vaginal pressure, etc. may allow enhanced determination of the user's exhibition of symptoms alignment with other physical and/or physiological characteristics. Optionally, within embodiments of the invention using photoplethysmography to provide vulvar/vaginal blood flow, but this may be replaced by another element with an electrical characteristic that is temperature dependent such as resistance, inductance, or capacitance for example.

Optionally, optical sensor elements may be employed for determining, for example, labial and vaginal blood flow using photoplethysmography (PPG) and / or laser Doppler imaging (LDI). Within PPG exploiting a reflective mode as depicted the volume of blood is determined in dependence upon the intensity of the reflected whilst each cardiac cycle appears as a peak within the reflected signal. As blood flow to the skin can be modulated by multiple other physiological systems, PPG can also be used to monitor breathing (respiration), medication effects, hypovolemia, and other circulatory conditions, especially where extended monitoring under a variety of conditions including rest and / or sleep provide enhanced baseline and / or early data. For example, the height of AC component of the PPG is proportional to the pulse pressure, the difference between the systolic and diastolic pressure in the arteries. Additionally, the shape of the PPG waveform differs from subject to subject, and varies with the location, providing additional options such as identification of user through PPG data and automatic adjustment of the ADDEV parameters / control program etc. in response therefrom.

Alternatively, Doppler imaging (LDI) wherein the OSAD is typically an infrared laser source in conjunction with a photodetector rather than a visible LED and photodetector in the instance of PPG. Accordingly, the pulsed laser light interacts with moving blood cells such that a small portion of it is reflected with a frequency shift, detected, and converted into an electrical signal. LDI can provide measurements without requiring physical contact and the signals are typically acquired at depth of 2-3mm (approx. 1/8") below the skin surface. Optionally, a device for characterization may employ an array of PPG and/or LDI sensors.

Within other embodiments of the invention a characterization and/or assessment device may exploit multiple electrical contacts (ELCOs) onto its surface. An array of ELCOs may be employed as well as a discrete ELCO and / or spatially separated ELCO pair(s). An ELCO may be employed to measure electrical activity and / or provide electrical stimulation to the user's vagina. Accordingly, the device may provide electrostimulation of the vaginal muscles with part of an exercise / training regime and then determine from user flexing the muscle strength / range of motion etc.

Within an alternate embodiment of the invention one or more of the ELCO elements may be replaced with a microphone such as one based upon capacitive thin film or microelectromechanical systems (MEMS) transducer, a piezoelectric transducer, accelerometer, hydrophone, or another type of microphone in order to measure the acoustic output of a contracting muscle. Accordingly, based upon such microphone placement a characterization device may support phonomyography (PMG) of the pubococcygeus muscle and / or other of the pelvic floor muscles. Typically, PMG has a frequency range of interest that is primarily 5-50Hz.

Within embodiments of the invention in a measurement and characterisation stage of determining the characteristics of the user a device may be employed which can deform to fit into the vagina and recover to fit against the vaginal walls mapping the user's physiology wherein resistance sensors may map the deformation through strain and/or stress. Alternatively, the device may be a balloon of high elasticity material with stress and/or strain sensors which is filled with a fluid expanding the balloon and the deformation mapped from which the user's physiology is derived.

Within the descriptions supra embodiments of the invention have been described with respect to providing simulation and assessment of a user's vagina, vaginal muscles etc. Electrical control and monitoring have been described together with wired and wireless data connectivity of the USTD to the external world. Accordingly, the USTD may be wirelessly connected to a user's PED or FED and access / post content / data to one or more local and / or remote servers associated with different aspects of the user including, but not limited to, their personal USTD profile, personal health records, other PEDs / FEDs / wearables, physician's office, etc.

In some embodiments of the invention, current exercise parameters and the user's performance / progress are sent to a doctor, trainer, or therapist in real-time and / or periodically. In some embodiments of the invention the doctor, trainer or therapist may concurrently within a communication link, such as a phone call, in the reverse direction provide human, personalized instruction, communication, status, or feedback to the user as well as seek additional clarification / information.

The USTD, cup, plug, cushion etc. may be provided in a range of physical sizes such that, for example, the length of an inserted actuated member (e.g. for vaginal insertion) may be 50mm, 65mm, 75mm, 100mm, 125mm, or 150mm for example (2", 2.5", 3", 4", 5", or 6") or other values for this dimension and its lateral dimensions may be, for example, 40mm, 50mm, 65mm, 75mm or 100m (1.6", 2", 2.5", 3", or 4") or other values for this dimension. The construction of a USTD, cup, plug, cushion etc. may employ one or more central scaffolds which provides rigidity or structure to the required portions of the USTD, cup, plug, cushion etc. which may be surrounded by a shell and then a casing. Whilst the casing and shell may be transparent or semi-transparent over portions or all of the USTD, cup, plug, cushion etc. it is common for the USTD, cup, plug, cushion etc.to be opaque. An outer casing may be coloured based upon skin colour tones based upon ethnicity or personal preference, e.g. light, dark, etc. as well as single colour, binary colour, multiple colour etc. According to the complexity acceptable then the outer casing may be formed from a variety of colours and / or be patterned for a specific design. Typically, such colours will be part of a silicone or other elastomer employed in forming the casing although in other embodiments of the invention the casing may be coloured once formed and a protective fluid proof, non-toxic, non-abrasive coating formed atop these applied colours. Such instances of applied colours may include metallic lacquers, particulate lacquers for "sparkle", etc.

Beneficially, medical grade silicone is clear thereby removing the requirement for any additional coating (e.g. food grade urethane) in conjunction with pigmented silicones. Accordingly, an USTD, cup, plug, cushion etc. may with medical grade silicone be clear and formed from an initial sticky soft silicone, e.g. 20 durometers, with a micro-layer (spray coated for example) of high durometer medical grade silicone, for example 70-90 durometer, to create "slippery" surface and avoid silky smooth surface that typically requires use of urethane coating.

Typically, the casing for the USTD, cup, plug, cushion etc. will be formed from a non-toxic, hypoallergenic silicone to provide a safe smooth surface although some regions of the USTD may be coated, textured and / or finished with a variation from that of the remainder of the casing in order to enhance or promote retention of the USTD, cup, plug, cushion etc. against the user's skin or clothing. Typically, the outer surface of the casing will be formed to provide low friction as well as resistance to lubricants, spermicides, and other chemicals that may or may not be employed by the user.

Embodiments of the invention with respect to the USTD, cup, plug, cushion etc. such as described within the embodiments of the invention supra may employ a "sticky" surface for a predetermined portion of the outer surface for engaging a recipient's body (e.g. being formed from a low durometer silicone for example) so that the surface is designed to "stick" to skin, so it stays in place or has higher resistance to motion. This "sticky" surface may be mirror surface, matt or textured for grip. Examples of materials may be those with durometer ideal Shore A10 or lower, Shore A5 or lower, or Shore A1. In some embodiments of the invention a region or regions of the casing may be formed from a gel such as the Ecoflex™ platinum catalyzed silicones for example certified to ISI 10993-10 for skin irritation / sensitization and having, for example, Shore 00-50 hardness (below the Shore A scale), Shore 00-30 hardness, Shore 00-20 hardness, or Shore 00-10 hardness. In embodiments of the invention the casing around the shell may act like a thin sheet (<<1mm thick), like a fabric or material, like a sheet (~1mm), a thick sheet (>1mm). Optionally, the lower surface of the casing designed for placement against a user's groin / stomach may be sticky and when washed recover this stickiness in its entirety or in different regions or areas.

Optionally, the outer surface which contact the user may be smooth with low friction to human skin, smooth with minimal friction to human skin, smooth with moderate friction to human skin, smooth with high friction to human skin in its entirety or in different regions or areas. Alternatively, the surface may be smooth, textured, and / or rough and have low friction, negligible friction, moderate friction, and / or high friction in its entirety or in different regions or areas. Optionally, the surface may be textured with low friction to human skin, textured with minimal friction to human skin, textured with moderate friction to human skin, or textured with high friction to human skin in its entirety or in different regions. Optionally, the surface of the casing in its entirety or in different regions or areas may be used in conjunction with disposable sheets that provide adhesion and / or friction in predetermined levels.

Within embodiments of the invention the casing, for example formed from silicone, is the only material surrounding the casing and the surface profile is derived from applying the casing to the contoured surface of the shell. In other embodiments of the invention the surface profile is derived from multiple applications of a single material forming the casing. In other embodiments of the invention an additional material or materials are disposed between the shell and the casing. This, may for example, be a preform formed from the same material as the casing such that the casing is applied as a single or multiple dip coating for example, a preform formed from another silicone of different characteristics to the casing, a preform formed from a plastic, a preform formed from a low density foam, from a medium density foam, or a high density foam. Alternatively, a combination of materials may be employed such as two or more plastics, two or more foams, a foam and a plastic, a foam and a silicone, a form and metal. The materials may be layered, inserted, embedded, etc. without departing from the scope of the invention. However, a characteristic of these materials is the transmission of vibratory motion arising from the active elements within the USTD according to embodiments of the invention. Within passive embodiments this characteristic of material selection is removed.

Optionally, the USTD, cup, plug, cushion etc. are formed through either one or more additive manufacturing (AM) steps and/or one or more non-additive manufacturing (NAM) steps.

The foregoing disclosure of the exemplary embodiments of the present invention has been presented for purposes of illustration and description. It is not intended to be exhaustive or to limit the invention to the precise forms disclosed. Many variations and modifications of the embodiments described herein will be apparent to one of ordinary skill in the art in light of the above disclosure. The scope of the invention is to be defined only by the claims appended hereto, and by their equivalents.

Further, in describing representative embodiments of the present invention, the specification may have presented the method and/or process of the present invention as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims. In addition, the claims directed to the method and/or process of the present invention should not be limited to the performance of their steps in the order written, and one skilled in the art can readily appreciate that the sequences may be varied and still remain within the spirit and scope of the present invention.

Embodiments of the present disclosure can be described in view of the following clauses.

1. A balloon comprising:

    a first balloon disposed at a first position on a catheter;
    a second balloon disposed adjacent the first balloon on the catheter; wherein

each of the first balloon and second balloon is filled with a predetermined fluid independent from the other of the first balloon and second balloon;

the first balloon is either attached at both ends to the catheter or attached at a first end of the catheter and extends beyond the catheter at a second distal end to the first end; and

the second balloon is attached at both ends to the catheter.

2. A balloon comprising:

an outer body;

an inner wall splitting the interior of the balloon into a first portion and a second portion;

a first end of the balloon is either attached to the catheter or extends beyond the catheter;

the second end of the balloon is attached to the catheter; and

the inner wall is attached to the catheter.

3. A method comprising:

providing an outer cushion formed from a low elastic modulus material for conforming to a predetermined portion of a patient's body;

providing a structural shield formed from a high elastic modulus material disposed with the outer cushion between it and the patient;

providing a manometry balloon for establishing first data relating to the patient; and

providing a probe for establishing second data relating the patient having one or more transducers generating and/or receiving signals of a predetermined type; wherein

the outer cushion and structural shield maintain the manometry balloon in position within a cavity of the patient;

the manometry balloon is filled to a predetermined pressure and/or volume with a predetermined fluid;

the body of the manometry balloon and the predetermined fluid have a high contrast against the patient's body to the signals of the predetermined type; and

the probe can be position against or in close proximity to the outer cushion.

4. The method according to clause 3, wherein

a fluidic connection to the manometry balloon passes through at least one of the outer cushion and structural shield.

5. The method according to clause 3, wherein

the outer cushion is formed from at least one of silicone, silicone with an outer shell of silicone or another material, and gelatin.

6. The method according to clause 3, wherein

the probe is positioned against or in close proximity to the outer cushion through an opening within the structural shield.

7. The method according to clause 3, wherein

the manometry balloon and outer cushion are a single piece-part.

8. The method according to clause 3, wherein

the outer cushion is inflatable and is inflated with a second predetermined fluid.

9. The method according to clause 3, further comprising

a first fluidic connector disposed on the structural shield;

a second fluidic connector disposed on a surface of the outer cushion distal to the structural shield; and

a fluidic connection connecting the first fluidic connector and second fluidic connector through the structural shield and outer cushion;

the first fluidic connector connects to an external manometry system;

the second fluidic connector connects directly or via a tube to the balloon.

10. A method comprising:

providing a structural shield formed from a high elastic modulus material;

providing a manometry balloon for establishing first data relating to the patient; and

providing a probe for establishing second data relating the patient; wherein

the structural shield maintains the manometry balloon in position within a cavity of the patient;

the manometry balloon is filled to a predetermined pressure and/or volume with a predetermined fluid;

the body of the manometry balloon and predetermined fluid present a high contrast against the patient's body to the probe; and
the probe can be positioned against or in close proximity to a window within the structural shield.

11. The method according to clause 10, further comprising:

providing an outer cushion formed from a low elastic modulus material for conforming to a predetermined portion of a patient's body; wherein
the outer cushion is disposed between patient's body and the structural shield.

12. The method according to clause 10, wherein
the structural shield forms part of an item of clothing to be worn by the patient when the first data and second data are obtained.

13. The method according to clause 10, wherein

the structural shield includes a window where the probe is positioned against or in close proximity to the window when the second data is acquired;
the window is transparent to signals generated by the probe; and
the structural shield absorbs, reflects or has high attenuation for the signals generated by the probe.

14. The method according to clause 10, wherein
the structural shield is attached to or forms part of a fitting such that when worn the fitting locates the structural shield in position with respect to a predetermined region of the patient's body.

15. The method according to clause 10, wherein
the structural shield forms part of an item of clothing.

16. The method according to clause 10, wherein

the probe is attached to the structural shield by a fitting which retains the probe against a window within the structural shield;
the window is transparent to signals generated by the probe; and
the structural shield absorbs, reflects or has high attenuation for the signals generated by the probe.

17. The method according to clause 10, wherein

the structural shield includes a fitting accepting the probe and retaining the probe in position relative to a window within the structural shield;
the window is transparent to signals generated by the probe; and
the structural shield absorbs, reflects or has high attenuation for the signals generated by the probe.

18. A method comprising:

providing a cushion formed from a low elastic modulus material for conforming to a predetermined portion of a patient's body; wherein
providing a manometry balloon for establishing first data relating to the patient;
providing a probe for establishing second data relating the patient; and
providing a seat within an opening; wherein
the cushion is disposed between patient's body and the seat;
the cushion maintains the manometry balloon in position within a cavity of the patient;
the manometry balloon is filled to a predetermined pressure and/or volume with a predetermined fluid;
the body of the manometry balloon and predetermined fluid present a high contrast against the patient's body to the probe; and
the probe is positioned in or in close proximity to the opening within the seat to acquire the second data.

19. The method according to clause 18, wherein
the cushion is inflatable and is inflated with a second predetermined fluid.

20. The method according to clause 18, wherein

the manometry balloon comprises a plug disposed at the end towards the exterior of the patient; and

the plug is inflatable and is inflated with a second predetermined fluid independent of the inflation of the manometry balloon with the predetermined fluid.

21. The method according to clause 20, wherein

the manometry balloon comprises a plug disposed at the end towards the exterior of the patient; the plug is inflatable and is inflated with a second predetermined fluid independent of the inflation of the manometry balloon with the predetermined fluid; and
inflation of the plug grips a catheter coupling the predetermined fluid to the manometry balloon retaining it in position.

22. The method according to clause 18, wherein

the manometry balloon comprises a plug disposed at the end towards the exterior of the patient;
the plug is inflatable and is inflated with a second predetermined fluid independent of the inflation of the manometry balloon with the predetermined fluid; and
the plug, manometry balloon and cushion are a single piece-part.

23. The method according to clause 18, wherein
a catheter providing the predetermined fluid to the manometry balloon passes through a predetermined portion of the cushion.
24. A method of performing measurements with respect to a patient comprising:

providing a balloon for insertion within a cavity of the patient;
providing a probe exploiting signals of a predetermined type;
providing a manometry system coupled to the balloon;
providing a support for the patient.

25. The method according to clause 24, wherein
providing the support comprises:

providing a structural shield formed from a material of high elastic modulus; and
providing a cushion formed from a material of low elastic modulus disposed; wherein
the cushion is disposed between the patient and the structural shield;
the cushion and structural shield retain the balloon within the cavity.

26. The method according to clause 25 wherein
the structural shield comprises a gasket disposed around a predetermined portion of the periphery of the structural shield formed from another low elastic modulus material.
27. The method according to clause 25, wherein
the structural shield is at least one of:

part of an item of clothing to be worn by the patient;
worn by the patient via a fitting attached to the structural shield and mounting to the patient's body; and
retained in position by pressure applied by the patient with the support between the patient and a seat upon which the patient sits during subsequent measurements.

28. The method according to clause 25, further comprising

providing a plug formed from a material of predetermined elastic modulus; wherein
the plug is positioned at an opening of the cavity and retained in position during subsequent measurements; and
a fluidic connection to the balloon extends through the plug.

29. The method according to clause 27, wherein
the plug is retained in position by at least one of an item of clothing to be worn by the patient, a structural shield worn by the patient, and a cushion disposed between the patient and a seat upon which the patient sits during subsequent measurements.
30. A device comprising:

an outer cushion formed from a low elastic modulus material for conforming to a predetermined portion of a patient's body;

a structural shield formed from a high elastic modulus material disposed with the outer cushion between it and the patient; wherein

the outer cushion and structural shield maintains a manometry balloon in position within a cavity of the patient when the manometry balloon is inserted into the cavity of the patient.

31. A method comprising:
automatically identifying a pubic symphysis (PS) of a patient and an anorectal angle (ARA) of the patient from a plurality of transperineal ultrasound images.

32. The method according to clause 31, wherein
at least one of:

the automatic identification is established in dependence upon automatic segmentation of the genital hiatal dimension extracted from a plan of minimal hiatal dimension; and

the automatic identification is used to provide feedback to either an automated system or an operator with respect to at least one of placement of an ultrasound probe and captured image quality of images acquired with the ultrasound probe when capturing transperineal ultrasound images.

33. The method according to clause 31, wherein

the automatic identification is established by a microprocessor based system exploiting a process comprising processing the plurality of transperineal ultrasound images to establish PS landmarks and ARA landmarks;

selecting the most locally consistent PS;

selecting the most locally consistent ARA.

34. The method according to clause 33, wherein
processing the plurality of transperineal ultrasound images to establish PS landmarks and ARA landmarks comprises for each transperineal ultrasound image of the plurality of transperineal ultrasound images performing the steps of:

establishing an input image volume from the plurality of transperineal ultrasound images;

extracting a mid-section sagittal plane extraction from the plurality of transperineal ultrasound images;

performing image processing processes comprising image sharpening, applying a weighted median filter and edge detection;

performing first processing comprising creation of a probability map, extraction of a negative gradient edge, extraction of an edge of a urethra of the patient and detecting the PS landmarks;

performing second processing comprising extraction of a positive gradient edge, edge detection, refinement of distance measurements, and detecting the ARA landmarks.

35. A device comprising:

a body;

a plurality of pads; and

a plurality of arms each connected to the body at a first end and to a pad of the plurality of pads at a second distal end; wherein

each arm of the plurality of arms can be extended from or retracted into the body; and

a subset of the plurality of pads comprise sensors, each pad of the predetermined subset of the plurality pads comprising a predetermined sensor.

36. A device comprising:

a body;

a plurality of sensors;

a distributed element around a periphery of the device comprising a plurality of elements; and

an electronic circuit comprising a microprocessor coupled to each sensor ofthe plurality of sensors and each element of the plurality of elements of the distributed element.

37. The device according to clause 36, wherein

the body comprises an annulus having an internal opening;

the periphery of the device around which the distributed element is disposed is the internal opening;

the distributed element under action of the microprocessor adjusts the plurality of elements to at least one of vary an overall dimension of the internal opening and adjust the geometry of the internal opening.

38. The device according to clause 36, wherein

the body comprises an annulus having an internal opening;

the periphery of the device around which the distributed element is disposed is an outer edge of the device distal to the internal opening; and

the distributed element under action of the microprocessor adjusts the plurality of elements to at least one of vary an overall dimension of the device and adjust the geometry of the device.

39. A method of establishing a user specific therapeutic device (USTD) comprising: establishing user data by performing measurement and characterisation with respect to the user where:

a first portion of the user data comprises structural measurements of a region of an anatomy of the user to which the USTD relates;

a second portion of the user data comprises at least one of physiological measurements and biomechanical measurements of the region of the anatomy of the user to which the USTD relates;

a third portion of the user data comprises establishing at least one of currently quality of life (QoL) data for the user, one or more QoL goals for the user, one or more symptoms experienced by the user, and user lifestyle data;

performing an automatic analysis and modelling of the USTD upon a computer system in dependence upon at least the user data;

establishing a design of the USTD in dependence upon the automatic analysis and modelling of the USTD;

establishing clinician approval of the design of the USTD via a clinical decision confirmation system (CDCS); and

manufacturing the USTD to the design of the USTD approved by the clinician.

40. The method according to clause 39, wherein the CDCS provides for:

rendering of the recommended design of the USTD to the clinician;

rendering of a three-dimensional visualization of the user's physiology and the USTD device;

editing and updating of at least one of one or more key features and one or more dimensions of the USTD by the clinician; and

clinician approval of the design for manufacturing.

41. The method according to clause 39, wherein the automatic analysis and modelling of the USTD in dependence upon at least the user data comprises:

establishing assessment data in dependence upon the user data;

establishing performance goal data in dependence upon the user data; and

applying at least one of one or more machine learning processes and one or more artificial intelligence processes to the user data to generate the design of the USTD.

42. The method according to clause 41, wherein the assessment data comprises at least one of:

bone structure;

soft tissue structure;

soft tissue strains;

anatomical geometry; and

static and dynamic measurements.

43. The method according to clause 41, wherein the performance goal data comprises at least one of:

one or more static performance goals;

one or more dynamic performance goals;
antimicrobial requirements of the USTD;
contraceptive requirements of the USTD;
drug delivery requirements of the USTD; and
requirements with respect to at least one of removal, cleaning and insertion of the UTSD.

## Claims

1. A method comprising:

   detecting, via at least one computational algorithm, a pubic symphysis, PS, landmark of a user within one or more transperineal ultrasound, TPUS, images of a patient;
   detecting, via the at least one computational algorithm, an anorectal angle, ARA, landmark from within the one or more TPUS images;
   extracting data characterizing a plane of minimal hiatal dimensions, PMHD, from the one or more TPUS images based on the detected PS landmark and the detected ARA landmark; and
   designing, based on the extracted data, a user-specific therapeutic device, USTD, to treat at least one ailment of the patient.

2. The method of claim 1, further comprising: providing feedback, based on the automatic identification of the PMHD, to either an automated system or an operator, with respect to at least one of: a placement of an ultrasound probe and captured image quality of images acquired with the ultrasound probe.

3. The method according to claim 1, further comprising: establishing clinician approval of the design of the USTD via a clinical decision confirmation system, CDCS; and manufacturing the USTD to the design of the USTD approved by the clinician.

4. The method of claim 1, further comprising, prior to detecting PS landmark and the ARA landmark, for each TPUS image of the one or more TPUS images, processing the TPUS image to improve an image quality of the TPUS image.

5. The method of claim 4, wherein processing each TPUS image of the one or more TPUS images comprises applying an unsharp filter to the TPUS image to enhance a contrast of the TPUS image.

6. The method of claim 5, wherein processing each TPUS image of the one or more TPUS images further comprises applying a fast-weighted median filter to the TPUS image to smooth the TPUS image.

7. The method of claim 1, wherein detecting the PS landmark comprises:

   detecting an edge of a urethra of the patient within the one or more TPUS images;
   selecting a point of highest curvature of the edge of the urethra as the PS landmark.

8. The method of claim 7, wherein the edge of the urethra is detected using a negative gradient flow process.

9. The method of claim 1, wherein detecting the ARA landmark comprises:

   detecting an edge of a rectum of the patient within the one or more TPUS images;
   selecting a point of the edge of the rectum that is closest to the PS landmark as the ARA landmark.

10. The method of claim 1, further comprising determining a rotation angle for extracting the PMHD based on the PS landmark and the ARA landmark.

11. The method of claim 1, further comprising extracting a mid-sagittal plane from the one or more TPUS images, wherein the PS landmark and the ARA landmark are detected in the extracted mid-sagittal plane.

12. The method of claim 1, further comprising detecting a symptom of a pelvic floor disorder, PFD, using the data characterizing the PMHD.

**13.** The method of claim 1, wherein the UTSD is a pessary device.

**14.** The method of claim 1, wherein designing the UTSD comprises determining a material or a geometry for the UTSD.

**15.** The method of claim 1, wherein the one or more TPUS images are three-dimensional TPUS images.

Figure 1

100

**Measurement & Characterisation 110**

*Structural* 112
- Manual
- Imaging
- Mechanical

*Force, Strain & Distension* 114
- Urodynamics
- Biomechanical

*Quality of Life* 116
- Current QoL Data
- QoL Goals
- Symptoms
- User lifestyle

**Analysis & Modelling 120**

*Assessment* 122
- Bone Structure
- Soft Tissue Structure
- Soft Tissue Strains
- Geometry
- Static and Dynamic Measurements

*Performance Goals* 124
- Static
- Dynamic
- Antimicrobial
- Contraceptive
- Drug delivery
- Removal/Cleaning/Insertion

160

**Custom Device Manufacturing & Fitting 130**

132 *Manufacture*
- Scaffold Structure
- Shell Structure
- Casing Structure
- Passive-Active Integration
- Lock-Release Structure
- Special Coatings

134 *Fitting*
- Initial Fitting
- Assessment
- Device Monitoring
- User Monitoring

OK

Monitor for Quality of Life / Performance

140

150 — Objectives Met — NO / YES

210

220

230

240

250

260

Figure 2

EP 4 725 409 A2

Figure 3

EP 4 725 409 A2

Figure 4

**Figure 5A**

Page content labels: 500A; Input 510 (Parameter Set A, Parameter Set B, Parameter Set C, Parameter Set D, Parameter Set E, Pressure/Volume Measurements, Pre-Processed Clinical Data); Prediction Model 520 (Statistical Analysis 520A, Deep Learning Algorithms 520B); Prediction and Design Output 530 (Pessary 530A: Success, Type, Size; Gynethotics™ 530B: CAD Model Parameters); Pessary Registry 540; Data Updates and New Data 550

500B

560

Method 1 → Physical Examination
Anatomic Measurements
POP-Q
Pessary Fitting History

Method 2 → Physical Examination
Anatomic Measurements
POP-Q+

Method 3 → Volumetric Measurements
Pressure Measurements

**PERSONALIZED**

DIAGNOSTICS                                    THERAPY

Clinician Design ──────▶

POP-Q+ ──────▶        Gynethotics
                      (Personalized)

CDI ──────▶

Ring with Support (Size 2,3,4) Diameter (cm)

Vaginal Introitus
$y=1.0219x+2.146$
$R_2=0.9688$

Genital Hiatus
$y=1.4103x+0.5577$
$R_2=0.8978$

Measurement (cm)

Gellhorn (Sizes 2.25, 2.5, 2.75, 3) Diameter (cm)

Vaginal Introitus
$y=0.9484x+1.5119$
$R_2=0.9822$

Genital Hiatus
$y=0.8033x+1.6779$
$R_2=0.7455$

Measurement (cm)

570                                              580

Figure 5B

EP 4 725 409 A2

600A

Length A

Width B

Thickness C

Thickness D

Thickness E

600B

Band Depth F

600C

Support Thickness G

600D

Knob Width I

Knob Length H

600E

Knob Height J

Knob Offset Height in Y-axis K

**FIG. 6**

700

710

720

730

740

750

760

**FIG. 7**

Figure 9

Figure 8

Figure 10

10-15 cm

Tied to catheter

Closed end or sealed to catheter

30-85°

Ø5-15cm when inflated

Thickness 0.015-0.085 mm

Nominal (Working) Pressure:8.0 kPa
Burst Pressure: >20 kPa

1100A
1100B

1160

1110

1130

1140    1120

1150

1100C    1100D

1170    1180    1190

Figure 11A

EP 4 725 409 A2

Figure 11B

EP 4 725 409 A2

Figure 13

Figure 12

Figure 14

Figure 16

Figure 15

Figure 18

Figure 17

Figure 20

Figure 19

Figure 21

2100A    2100B    2100C

Figure 22

Figure 23

Figure 24

Figure 25

Figure 26

EP 4 725 409 A2

Figure 27A

Figure 27B

Figure 28

EP 4 725 409 A2

Figure 29

EP 4 725 409 A2

Figure 30A

Figure 30B

Figure 31A

3110

3120

3130

3150

3160

3170

3180

3190

3100A
3100B

3120

3110

3130

3190

3150

3170

3180

3175    3160

# Figure 31B

EP 4 725 409 A2

Figure 33

Figure 32

76

Figure 35

Figure 34

3610

3620

3630

3600A   3600B

3640A
3650A

3660

3650B
3640B

Figure 36

EP 4 725 409 A2

Figure 37

EP 4 725 409 A2

Figure 39

Figure 40

Figure 38

4100A    4100B    4100C

Figure 41

4400A    4400B    4400C

Figure 44

Figure 42

4200A
- Input Image Volume
- Mid-Section Sagital Plane Extraction

4200B
- Image Sharpening
- Weighted Median Filter
- Canny Edge Extraction

4200C
- Probability Map Creation
- Negative Gradient Edge Extraction
- Urethra Edge Extraction
- PS Landmark Detection

4200D
- Positive Edge Gradient Extraction
- Edge Detection
- Distance Based Refinement
- ARA Landmark Detection

4200E
- Run for All Mid-Section Planes
- Select Most Locally Consistent PS
- Select Most Locally Consistent ARA

4200F
- Output Landmarks

4200G
- Output Minimal Histal Plane

Figure 43

Figure 45

Figure 46

Figure 47

EP 4 725 409 A2

Figure 48

Figure 49

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63133913

- WO 2019051579 A